# EUROPEAN PATENT SPECIFICATION

(11) **EP 2 633 321 B1**
(45) Date of publication and mention of the grant of the patent: **06.12.2017**
(21) Application number: 11837226.7
(22) Date of filing: 28.10.2011
(51) Int. Cl.: G01N 33/551, G01N 33/569, G01N 33/541, G01N 33/68

(54) **ANTISERA ASSAYS FOR MLV RELATED VIRUSES IN HUMANS AND OTHER MAMMALS**
ANTISERENASSAYS FÜR MLV-VERMITTELTE VIREN IN MENSCHEN UND SÄUGETIEREN
DOSAGES D'ANTISÉRUMS POUR VIRUS APPARENTÉS AU MLV CHEZ DES ÊTRES HUMAINS ET D'AUTRES MAMMIFÈRES

(30) Priority: 31.10.2010 US 408630 P
(43) Date of publication of application: 04.09.2013
(73) Proprietor: Tocagen Inc., San Diego, CA 92109 (US)
(72) Inventor: JOLLY, Douglas, J., Encinitas, CA 92024 (US); IBANEZ, Carlos, San Diego, CA 92129 (US); PEREZ, Omar, D., San Diego, CA 92103 (US); GRUBER, Harry, E., Rancho Santa Fe, CA 92067 (US)
(74) Representative: Daniels, Jeffrey Nicholas
(86) International application number: PCT/US2011/058457
(87) International publication number: WO 2012/058637

(56) References cited:
- US-A- 4 855 242
- US-A- 6 093 548
- US-A1- 2010 167 268
- US-A1- 2011 135 674
- N. MAKAROVA ET AL: "Antibody Responses against Xenotropic Murine Leukemia Virus-Related Virus Envelope in a Murine Model", PLOS ONE, vol. 6, no. 4, 6 April 2011 (2011-04-06), page e18272, XP055106073, PLOS ONE Inc, San Francisco, CA USA DOI: 10.1371/journal.pone.0018272
- R. O'KENNEDY ET AL: "Experimental section - A Review of Enzyme-Immunoassay and a Description of a Competitive Enzyme-Linked Immunosorbent Assay for the Detection of Immunoglobulin Concentrations", BIOCHEMICAL EDUCATION, PERGAMON, vol. 18, no. 3, 1 July 1990 (1990-07-01), pages 136-140, XP023538198, ISSN: 0307-4412, DOI: 10.1016/0307-4412(90)90219-E [retrieved on 1990-07-01]
- HOHN, 0. ET AL.: 'Lack of evidence for xenotropic murine leukemia virus-related virus(XMRV) in German prostate cancer patients.' RETROVIROLOGY. vol. 6, 16 October 2009, pages 92 - 102, XP021063734
- BRIGGS JOHN A G ET AL: "Do lipid rafts mediate virus assembly and pseudotyping?", JOURNAL OF GENERAL VIROLOGY, SOCIETY FOR GENERAL MICROBIOLOGY, SPENCERS WOOD, GB, vol. 84, no. PART 4, 1 April 2003 (2003-04-01), pages 757-768, ISSN: 0022-1317, DOI: 10.1099/VIR.0.18779-0 [retrieved on 2003-01-15]
- RAWAT SATINDER S ET AL: "Modulation of entry of enveloped viruses by cholesterol and sphingolipids (Review)", MOLECULAR MEMBRANE BIOLOGY, TAYLOR AND FRANCIS, GB, vol. 20, no. 3, 1 July 2003 (2003-07-01), pages 243-254, ISSN: 0968-7688, DOI: 10.1080/0968768031000104944
- GESELOWITZ D A ET AL: "Bovine serum albumin is a major oligonucleotide-binding protein found on the surface of cultured cells", ANTISENSE RESEARCH AND DEVELOPMENT 1995 US, vol. 5, no. 3, 1995, pages 213-217, ISSN: 1050-5261

### SEQUENCE LISTING

<110> Tocagen Inc.
   Jolly, Douglas J.
<120> ANTISERA ASSAYS FOR MLV RELATED VIRUSES IN HUMANS AND OTHER MAMMALS
<130> 00014-013WO1
<140> Not yet assigned
   <141> 2011-10-28
<150> US 61/408,630
   <151> 2010-10-31
<160> 11
<170> PatentIn version 3.5
<210> 1
   <211> 20
   <212> DNA
   <213> Artificial Sequence
<220>
   <223> Oligonucleotide Primer
<400> 1
   tttgattcct cagtgggctc 20
<210> 2
   <211> 22
   <212> DNA
   <213> Artificial Sequence
<220>
   <223> Oligonucleotide Primer
<400> 2
   cgatacagtc ttagtcccca tg 22
<210> 3
   <211> 26
   <212> DNA
   <213> Artificial Sequence
<220>
   <223> Oligonucleotide Primer
<400> 3
   cccttttacc cgcgtcagtg aattct 26
<210> 4
   <211> 21
   <212> DNA
   <213> Artificial Sequence
<220>
   <223> Oligonucleotide Primer
<400> 4
   aacaagcggg tggaagacat c 21
<210> 5
   <211> 21
   <212> DNA
   <213> Artificial Sequence
<220>
   <223> Oligonucleotide Primer
<400> 5
   caaaggcgaa gagaggctga c 21
<210> 6
   <211> 25
   <212> DNA
   <213> Artificial Sequence
<220>
   <223> Oligonucleotide Primer
<400> 6
   cccaccgtgc ccaaccctta caacc 25
<210> 7
   <211> 11892
   <212> DNA
   <213> Artificial Sequence
<220>
   <223> pAC3-yCD2 recombinant vector
<400> 7
   tagttattaa tagtaatcaa ttacggggtc attagttcat agcccatata tggagttccg      60
   cgttacataa cttacggtaa atggcccgcc tggctgaccg cccaacgacc cccgcccatt     120
   gacgtcaata atgacgtatg ttcccatagt aacgccaata gggactttcc attgacgtca     180
   atgggtggag tatttacggt aaactgccca cttggcagta catcaagtgt atcatatgcc     240
   aagtacgccc cctattgacg tcaatgacgg taaatggccc gcctggcatt atgcccagta     300
   catgacctta tgggactttc ctacttggca gtacatctac gtattagtca tcgctattac     360
   catggtgatg cggttttggc agtacatcaa tgggcgtgga tagcggtttg actcacgggg     420
   atttccaagt ctccacccca ttgacgtcaa tgggagtttg ttttggcacc aaaatcaacg     480
   ggactttcca aaatgtcgta acaactccgc cccattgacg caaatgggcg gtaggcgtgt     540
   acggtgggag gtctatataa gcagagctgg tttagtgaac cggcgccagt cctccgattg     600
   actgagtcgc ccgggtaccc gtgtatccaa taaaccctct tgcagttgca tccgacttgt     660
   ggtctcgctg ttccttggga gggtctcctc tgagtgattg actacccgtc agcgggggtc     720
   tttcatttgg gggctcgtcc gggatcggga gacccctgcc cagggaccac cgacccacca     780
   ccgggaggta agctggccag caacttatct gtgtctgtcc gattgtctag tgtctatgac      840
   tgattttatg cgcctgcgtc ggtactagtt agctaactag ctctgtatct ggcggacccg      900
   tggtggaact gacgagttcg gaacacccgg ccgcaaccct gggagacgtc ccagggactt      960
   cgggggccgt ttttgtggcc cgacctgagt ccaaaaatcc cgatcgtttt ggactctttg     1020
   gtgcaccccc cttagaggag ggatatgtgg ttctggtagg agacgagaac ctaaaacagt     1080
   tcccgcctcc gtctgaattt ttgctttcgg tttgggaccg aagccgcgcc gcgcgtcttg     1140
   tctgctgcag catcgttctg tgttgtctct gtctgactgt gtttctgtat ttgtctgaga     1200
   atatgggcca gactgttacc actcccttaa gtttgacctt aggtcactgg aaagatgtcg     1260
   agcggatcgc tcacaaccag tcggtagatg tcaagaagag acgttgggtt accttctgct     1320
   ctgcagaatg gccaaccttt aacgtcggat ggccgcgaga cggcaccttt aaccgagacc     1380
   tcatcaccca ggttaagatc aaggtctttt cacctggccc gcatggacac ccagaccagg     1440
   tcccctacat cgtgacctgg gaagccttgg cttttgaccc ccctccctgg gtcaagccct     1500
   ttgtacaccc taagcctccg cctcctcttc ctccatccgc cccgtctctc ccccttgaac     1560
   ctcctcgttc gaccccgcct cgatcctccc tttatccagc cctcactcct tctctaggcg     1620
   ccaaacctaa acctcaagtt ctttctgaca gtggggggcc gctcatcgac ctacttacag     1680
   aagacccccc gccttatagg gacccaagac cacccccttc cgacagggac ggaaatggtg     1740
   gagaagcgac ccctgcggga gaggcaccgg acccctcccc aatggcatct cgcctacgtg     1800
   ggagacggga gccccctgtg gccgactcca ctacctcgca ggcattcccc ctccgcgcag     1860
   gaggaaacgg acagcttcaa tactggccgt tctcctcttc tgacctttac aactggaaaa     1920
   ataataaccc ttctttttct gaagatccag gtaaactgac agctctgatc gagtctgttc     1980
   tcatcaccca tcagcccacc tgggacgact gtcagcagct gttggggact ctgctgaccg     2040
   gagaagaaaa acaacgggtg ctcttagagg ctagaaaggc ggtgcggggc gatgatgggc     2100
   gccccactca actgcccaat gaagtcgatg ccgcttttcc cctcgagcgc ccagactggg     2160
   attacaccac ccaggcaggt aggaaccacc tagtccacta tcgccagttg ctcctagcgg     2220
   gtctccaaaa cgcgggcaga agccccacca atttggccaa ggtaaaagga ataacacaag     2280
   ggcccaatga gtctccctcg gccttcctag agagacttaa ggaagcctat cgcaggtaca     2340
   ctccttatga ccctgaggac ccagggcaag aaactaatgt gtctatgtct ttcatttggc     2400
   agtctgcccc agacattggg agaaagttag agaggttaga agatttaaaa aacaagacgc     2460
   ttggagattt ggttagagag gcagaaaaga tctttaataa acgagaaacc ccggaagaaa     2520
   gagaggaacg tatcaggaga gaaacagagg aaaaagaaga acgccgtagg acagaggatg     2580
   agcagaaaga gaaagaaaga gatcgtagga gacatagaga gatgagcaag ctattggcca     2640
   ctgtcgttag tggacagaaa caggatagac agggaggaga acgaaggagg tcccaactcg 2700
   atcgcgacca gtgtgcctac tgcaaagaaa aggggcactg ggctaaagat tgtcccaaga 2760
   aaccacgagg acctcgggga ccaagacccc agacctccct cctgacccta gatgactagg 2820
   gaggtcaggg tcaggagccc ccccctgaac ccaggataac cctcaaagtc ggggggcaac 2880
   ccgtcacctt cctggtagat actggggccc aacactccgt gctgacccaa aatcctggac 2940
   ccctaagtga taagtctgcc tgggtccaag gggctactgg aggaaagcgg tatcgctgga 3000
   ccacggatcg caaagtacat ctagctaccg gtaaggtcac ccactctttc ctccatgtac 3060
   cagactgtcc ctatcctctg ttaggaagag atttgctgac taaactaaaa gcccaaatcc 3120
   actttgaggg atcaggagcc caggttatgg gaccaatggg gcagcccctg caagtgttga 3180
   ccctaaatat agaagatgag catcggctac atgagacctc aaaagagcca gatgtttctc 3240
   tagggtccac atggctgtct gattttcctc aggcctgggc ggaaaccggg ggcatgggac 3300
   tggcagttcg ccaagctcct ctgatcatac ctctgaaagc aacctctacc cccgtgtcca 3360
   taaaacaata ccccatgtca caagaagcca gactggggat caagccccac atacagagac 3420
   tgttggacca gggaatactg gtaccctgcc agtccccctg gaacacgccc ctgctacccg 3480
   ttaagaaacc agggactaat gattataggc ctgtccagga tctgagagaa gtcaacaagc 3540
   gggtggaaga catccacccc accgtgccca acccttacaa cctcttgagc gggctcccac 3600
   cgtcccacca gtggtacact gtgcttgatt taaaggatgc ctttttctgc ctgagactcc 3660
   accccaccag tcagcctctc ttcgcctttg agtggagaga tccagagatg ggaatctcag 3720
   gacaattgac ctggaccaga ctcccacagg gtttcaaaaa cagtcccacc ctgtttgatg 3780
   aggcactgca cagagaccta gcagacttcc ggatccagca cccagacttg atcctgctac 3840
   agtacgtgga tgacttactg ctggccgcca cttctgagct agactgccaa caaggtactc 3900
   gggccctgtt acaaacccta gggaacctcg ggtatcgggc ctcggccaag aaagcccaaa 3960
   tttgccagaa acaggtcaag tatctggggt atcttctaaa agagggtcag agatggctga 4020
   ctgaggccag aaaagagact gtgatggggc agcctactcc gaagacccct cgacaactaa 4080
   gggagttcct agggacggca ggcttctgtc gcctctggat ccctgggttt gcagaaatgg 4140
   cagccccctt gtaccctctc accaaaacgg ggactctgtt taattggggc ccagaccaac 4200
   aaaaggccta tcaagaaatc aagcaagctc ttctaactgc cccagccctg gggttgccag 4260
   atttgactaa gccctttgaa ctctttgtcg acgagaagca gggctacgcc aaaggtgtcc 4320
   taacgcaaaa actgggacct tggcgtcggc cggtggccta cctgtccaaa aagctagacc 4380
   cagtagcagc tgggtggccc ccttgcctac ggatggtagc agccattgcc gtactgacaa 4440
   aggatgcagg caagctaacc atgggacagc cactagtcat tctggccccc catgcagtag 4500
   aggcactagt caaacaaccc cccgaccgct ggctttccaa cgcccggatg actcactatc 4560
   aggccttgct tttggacacg gaccgggtcc agttcggacc ggtggtagcc ctgaacccgg 4620
   ctacgctgct cccactgcct gaggaagggc tgcaacacaa ctgccttgat atcctggccg 4680
   aagcccacgg aacccgaccc gacctaacgg accagccgct cccagacgcc gaccacacct 4740
   ggtacacgga tggaagcagt ctcttacaag agggacagcg taaggcggga gctgcggtga 4800
   ccaccgagac cgaggtaatc tgggctaaag ccctgccagc cgggacatcc gctcagcggg 4860
   ctgaactgat agcactcacc caggccctaa agatggcaga aggtaagaag ctaaatgttt 4920
   atactgatag ccgttatgct tttgctactg cccatatcca tggagaaata tacagaaggc 4980
   gtgggttgct cacatcagaa ggcaaagaga tcaaaaataa agacgagatc ttggccctac 5040
   taaaagccct ctttctgccc aaaagactta gcataatcca ttgtccagga catcaaaagg 5100
   gacacagcgc cgaggctaga ggcaaccgga tggctgacca agcggcccga aaggcagcca 5160
   tcacagagac tccagacacc tctaccctcc tcatagaaaa ttcatcaccc tacacctcag 5220
   aacattttca ttacacagtg actgatataa aggacctaac caagttgggg gccatttatg 5280
   ataaaacaaa gaagtattgg gtctaccaag gaaaacctgt gatgcctgac cagtttactt 5340
   ttgaattatt agactttctt catcagctga ctcacctcag cttctcaaaa atgaaggctc 5400
   tcctagagag aagccacagt ccctactaca tgctgaaccg ggatcgaaca ctcaaaaata 5460
   tcactgagac ctgcaaagct tgtgcacaag tcaacgccag caagtctgcc gttaaacagg 5520
   gaactagggt ccgcgggcat cggcccggca ctcattggga gatcgatttc accgagataa 5580
   agcccggatt gtatggctat aaatatcttc tagtttttat agataccttt tctggctgga 5640
   tagaagcctt cccaaccaag aaagaaaccg ccaaggtcgt aaccaagaag ctactagagg 5700
   agatcttccc caggttcggc atgcctcagg tattgggaac tgacaatggg cctgccttcg 5760
   tctccaaggt gagtcagaca gtggccgatc tgttggggat tgattggaaa ttacattgtg 5820
   catacagacc ccaaagctca ggccaggtag aaagaatgaa tagaaccatc aaggagactt 5880
   taactaaatt aacgcttgca actggctcta gagactgggt gctcctactc cccttagccc 5940
   tgtaccgagc ccgcaacacg ccgggccccc atggcctcac cccatatgag atcttatatg 6000
   gggcaccccc gccccttgta aacttccctg accctgacat gacaagagtt actaacagcc 6060
   cctctctcca agctcactta caggctctct acttagtcca gcacgaagtc tggagacctc 6120
   tggcggcagc ctaccaagaa caactggacc gaccggtggt acctcaccct taccgagtcg 6180
   gcgacacagt gtgggtccgc cgacaccaga ctaagaacct agaacctcgc tggaaaggac 6240
   cttacacagt cctgctgacc acccccaccg ccctcaaagt agacggcatc gcagcttgga 6300
   tacacgccgc ccacgtgaag gctgccgacc ccgggggtgg accatcctct agactgacat 6360
   ggcgcgttca acgctctcaa aaccccctca agataagatt aacccgtgga agcccttaat 6420
   agtcatggga gtcctgttag gagtagggat ggcagagagc ccccatcagg tctttaatgt 6480
   aacctggaga gtcaccaacc tgatgactgg gcgtaccgcc aatgccacct ccctcctggg 6540
   aactgtacaa gatgccttcc caaaattata ttttgatcta tgtgatctgg tcggagagga 6600
   gtgggaccct tcagaccagg aaccgtatgt cgggtatggc tgcaagtacc ccgcagggag 6660
   acagcggacc cggacttttg acttttacgt gtgccctggg cataccgtaa agtcggggtg 6720
   tgggggacca ggagagggct actgtggtaa atgggggtgt gaaaccaccg gacaggctta 6780
   ctggaagccc acatcatcgt gggacctaat ctcccttaag cgcggtaaca ccccctggga 6840
   cacgggatgc tctaaagttg cctgtggccc ctgctacgac ctctccaaag tatccaattc 6900
   cttccaaggg gctactcgag ggggcagatg caaccctcta gtcctagaat tcactgatgc 6960
   aggaaaaaag gctaactggg acgggcccaa atcgtgggga ctgagactgt accggacagg 7020
   aacagatcct attaccatgt tctccctgac ccggcaggtc cttaatgtgg gaccccgagt 7080
   ccccataggg cccaacccag tattacccga ccaaagactc ccttcctcac caatagagat 7140
   tgtaccggct ccacagccac ctagccccct caataccagt tacccccctt ccactaccag 7200
   tacaccctca acctccccta caagtccaag tgtcccacag ccacccccag gaactggaga 7260
   tagactacta gctctagtca aaggagccta tcaggcgctt aacctcacca atcccgacaa 7320
   gacccaagaa tgttggctgt gcttagtgtc gggacctcct tattacgaag gagtagcggt 7380
   cgtgggcact tataccaatc attccaccgc tccggccaac tgtacggcca cttcccaaca 7440
   taagcttacc ctatctgaag tgacaggaca gggcctatgc atgggggcag tacctaaaac 7500
   tcaccaggcc ttatgtaaca ccacccaaag cgccggctca ggatcctact accttgcagc 7560
   acccgccgga acaatgtggg cttgcagcac tggattgact ccctgcttgt ccaccacggt 7620
   gctcaatcta accacagatt attgtgtatt agttgaactc tggcccagag taatttacca 7680
   ctcccccgat tatatgtatg gtcagcttga acagcgtacc aaatataaaa gagagccagt 7740
   atcattgacc ctggcccttc tactaggagg attaaccatg ggagggattg cagctggaat 7800
   agggacgggg accactgcct taattaaaac ccagcagttt gagcagcttc atgccgctat 7860
   ccagacagac ctcaacgaag tcgaaaagtc aattaccaac ctagaaaagt cactgacctc 7920
   gttgtctgaa gtagtcctac agaaccgcag aggcctagat ttgctattcc taaaggaggg 7980
   aggtctctgc gcagccctaa aagaagaatg ttgtttttat gcagaccaca cggggctagt 8040
   gagagacagc atggccaaat taagagaaag gcttaatcag agacaaaaac tatttgagac 8100
   aggccaagga tggttcgaag ggctgtttaa tagatccccc tggtttacca ccttaatctc 8160
   caccatcatg ggacctctaa tagtactctt actgatctta ctctttggac cttgcattct 8220
   caatcgattg gtccaatttg ttaaagacag gatctcagtg gtccaggctc tggttttgac 8280
   tcagcaatat caccagctaa aacccataga gtacgagcca tgaacgcgtt actggccgaa 8340
   gccgcttgga ataaggccgg tgtgcgtttg tctatatgtt attttccacc atattgccgt 8400
   cttttggcaa tgtgagggcc cggaaacctg gccctgtctt cttgacgagc attcctaggg 8460
   gtctttcccc tctcgccaaa ggaatgcaag gtctgttgaa tgtcgtgaag gaagcagttc 8520
   ctctggaagc ttcttgaaga caaacaacgt ctgtagcgac cctttgcagg cagcggaacc 8580
   ccccacctgg cgacaggtgc ctctgcggcc aaaagccacg tgtataagat acacctgcaa 8640
   aggcggcaca accccagtgc cacgttgtga gttggatagt tgtggaaaga gtcaaatggc 8700
   tctcctcaag cgtattcaac aaggggctga aggatgccca gaaggtaccc cattgtatgg 8760
   gatctgatct ggggcctcgg tgcacatgct ttacatgtgt ttagtcgagg ttaaaaaaac 8820
   gtctaggccc cccgaaccac ggggacgtgg ttttcctttg aaaaacacga ttataaatgg 8880
   tgaccggcgg catggcctcc aagtgggatc aaaagggcat ggatatcgct tacgaggagg 8940
   ccctgctggg ctacaaggag ggcggcgtgc ctatcggcgg ctgtctgatc aacaacaagg 9000
   acggcagtgt gctgggcagg ggccacaaca tgaggttcca gaagggctcc gccaccctgc 9060
   acggcgagat ctccaccctg gagaactgtg gcaggctgga gggcaaggtg tacaaggaca 9120
   ccaccctgta caccaccctg tccccttgtg acatgtgtac cggcgctatc atcatgtacg 9180
   gcatccctag gtgtgtgatc ggcgagaacg tgaacttcaa gtccaagggc gagaagtacc 9240
   tgcaaaccag gggccacgag gtggtggttg ttgacgatga gaggtgtaag aagctgatga 9300
   agcagttcat cgacgagagg cctcaggact ggttcgagga tatcggcgag taagcggccg 9360
   cagataaaat aaaagatttt atttagtctc cagaaaaagg ggggaatgaa agaccccacc 9420
   tgtaggtttg gcaagctagc ttaagtaacg ccattttgca aggcatggaa aaatacataa 9480
   ctgagaatag agaagttcag atcaaggtca ggaacagatg gaacagctga atatgggcca 9540
   aacaggatat ctgtggtaag cagttcctgc cccggctcag ggccaagaac agatggaaca 9600
   gctgaatatg ggccaaacag gatatctgtg gtaagcagtt cctgccccgg ctcagggcca 9660
   agaacagatg gtccccagat gcggtccagc cctcagcagt ttctagagaa ccatcagatg 9720
   tttccagggt gccccaagga cctgaaatga ccctgtgcct tatttgaact aaccaatcag 9780
   ttcgcttctc gcttctgttc gcgcgcttct gctccccgag ctcaataaaa gagcccacaa 9840
   cccctcactc ggggcgccag tcctccgatt gactgagtcg cccgggtacc cgtgtatcca 9900
   ataaaccctc ttgcagttgc atccgacttg tggtctcgct gttccttggg agggtctcct 9960
   ctgagtgatt gactacccgt cagcgggggt ctttcattac atgtgagcaa aaggccagca 10020
   aaaggccagg aaccgtaaaa aggccgcgtt gctggcgttt ttccataggc tccgcccccc 10080
   tgacgagcat cacaaaaatc gacgctcaag tcagaggtgg cgaaacccga caggactata 10140
   aagataccag gcgtttcccc ctggaagctc cctcgtgcgc tctcctgttc cgaccctgcc 10200
   gcttaccgga tacctgtccg cctttctccc ttcgggaagc gtggcgcttt ctcaatgctc 10260
   acgctgtagg tatctcagtt cggtgtaggt cgttcgctcc aagctgggct gtgtgcacga 10320
   accccccgtt cagcccgacc gctgcgcctt atccggtaac tatcgtcttg agtccaaccc 10380
   ggtaagacac gacttatcgc cactggcagc agccactggt aacaggatta gcagagcgag 10440
   gtatgtaggc ggtgctacag agttcttgaa gtggtggcct aactacggct acactagaag 10500
   gacagtattt ggtatctgcg ctctgctgaa gccagttacc ttcggaaaaa gagttggtag 10560
   ctcttgatcc ggcaaacaaa ccaccgctgg tagcggtggt ttttttgttt gcaagcagca 10620
   gattacgcgc agaaaaaaag gatctcaaga agatcctttg atcttttcta cggggtctga 10680
   cgctcagtgg aacgaaaact cacgttaagg gattttggtc atgagattat caaaaaggat 10740
   cttcacctag atccttttaa attaaaaatg aagttttaaa tcaatctaaa gtatatatga 10800
   gtaaacttgg tctgacagtt accaatgctt aatcagtgag gcacctatct cagcgatctg 10860
   tctatttcgt tcatccatag ttgcctgact ccccgtcgtg tagataacta cgatacggga 10920
   gggcttacca tctggcccca gtgctgcaat gataccgcga gacccacgct caccggctcc 10980
   agatttatca gcaataaacc agccagccgg aagggccgag cgcagaagtg gtcctgcaac 11040
   tttatccgcc tccatccagt ctattaattg ttgccgggaa gctagagtaa gtagttcgcc 11100
   agttaatagt ttgcgcaacg ttgttgccat tgctgcaggc atcgtggtgt cacgctcgtc 11160
   gtttggtatg gcttcattca gctccggttc ccaacgatca aggcgagtta catgatcccc 11220
   catgttgtgc aaaaaagcgg ttagctcctt cggtcctccg atcgttgtca gaagtaagtt 11280
   ggccgcagtg ttatcactca tggttatggc agcactgcat aattctctta ctgtcatgcc 11340
   atccgtaaga tgcttttctg tgactggtga gtactcaacc aagtcattct gagaatagtg 11400
   tatgcggcga ccgagttgct cttgcccggc gtcaacacgg gataataccg cgccacatag 11460
   cagaacttta aaagtgctca tcattggaaa acgttcttcg gggcgaaaac tctcaaggat 11520
   cttaccgctg ttgagatcca gttcgatgta acccactcgt gcacccaact gatcttcagc 11580
   atcttttact ttcaccagcg tttctgggtg agcaaaaaca ggaaggcaaa atgccgcaaa 11640
   aaagggaata agggcgacac ggaaatgttg aatactcata ctcttccttt ttcaatatta 11700
   ttgaagcatt tatcagggtt attgtctcat gagcggatac atatttgaat gtatttagaa 11760
   aaataaacaa ataggggttc cgcgcacatt tccccgaaaa gtgccacctg acgtctaaga 11820
   aaccattatt atcatgacat taacctataa aaataggcgt atcacgaggc cctttcgtct 11880
   tcaagaattc at 11892
<210> 8
   <211> 11892
   <212> DNA
   <213> Artificial Sequence
<220>
   <223> pAC3-yCD recombinant vector
<400> 8
<210> 9
   <211> 11893
   <212> DNA
   <213> Artificial Sequence
<220>
   <223> pAC3-yCD2 recombinant vector
<400> 9
   tagttattaa tagtaatcaa ttacggggtc attagttcat agcccatata tggagttccg        60
   cgttacataa cttacggtaa atggcccgcc tggctgaccg cccaacgacc cccgcccatt       120
   gacgtcaata atgacgtatg ttcccatagt aacgccaata gggactttcc attgacgtca       180
   atgggtggag tatttacggt aaactgccca cttggcagta catcaagtgt atcatatgcc       240
   aagtacgccc cctattgacg tcaatgacgg taaatggccc gcctggcatt atgcccagta       300
   catgacctta tgggactttc ctacttggca gtacatctac gtattagtca tcgctattac       360
   catggtgatg cggttttggc agtacatcaa tgggcgtgga tagcggtttg actcacgggg       420
   atttccaagt ctccacccca ttgacgtcaa tgggagtttg ttttggcacc aaaatcaacg       480
   ggactttcca aaatgtcgta acaactccgc cccattgacg caaatgggcg gtaggcgtgt       540
   acggtgggag gtctatataa gcagagctgg tttagtgaac cggcgccagt cctccgattg       600
   actgagtcgc ccgggtaccc gtgtatccaa taaaccctct tgcagttgca tccgacttgt       660
   ggtctcgctg ttccttggga gggtctcctc tgagtgattg actacccgtc agcgggggtc       720
   tttcatttgg gggctcgtcc gggatcggga gacccctgcc cagggaccac cgacccacca       780
   ccgggaggta agctggccag caacttatct gtgtctgtcc gattgtctag tgtctatgac       840
   tgattttatg cgcctgcgtc ggtactagtt agctaactag ctctgtatct ggcggacccg 900
   tggtggaact gacgagttcg gaacacccgg ccgcaaccct gggagacgtc ccagggactt 960
   cgggggccgt ttttgtggcc cgacctgagt ccaaaaatcc cgatcgtttt ggactctttg 1020
   gtgcaccccc cttagaggag ggatatgtgg ttctggtagg agacgagaac ctaaaacagt 1080
   tcccgcctcc gtctgaattt ttgctttcgg tttgggaccg aagccgcgcc gcgcgtcttg 1140
   tctgctgcag catcgttctg tgttgtctct gtctgactgt gtttctgtat ttgtctgaaa 1200
   atatgggcca gactgttacc actcccttaa gtttgacctt aggtcactgg aaagatgtcg 1260
   agcggatcgc tcacaaccag tcggtagatg tcaagaagag acgttgggtt accttctgct 1320
   ctgcagaatg gccaaccttt aacgtcggat ggccgcgaga cggcaccttt aaccgagacc 1380
   tcatcaccca ggttaagatc aaggtctttt cacctggccc gcatggacac ccagaccagg 1440
   tcccctacat cgtgacctgg gaagccttgg cttttgaccc ccctccctgg gtcaagccct 1500
   ttgtacaccc taagcctccg cctcctcttc ctccatccgc cccgtctctc ccccttgaac 1560
   ctcctcgttc gaccccgcct cgatcctccc tttatccagc cctcactcct tctctaggcg 1620
   ccaaacctaa acctcaagtt ctttctgaca gtggggggcc gctcatcgac ctacttacag 1680
   aagacccccc gccttatagg gacccaagac cacccccttc cgacagggac ggaaatggtg 1740
   gagaagcgac ccctgcggga gaggcaccgg acccctcccc aatggcatct cgcctacgtg 1800
   ggagacggga gccccctgtg gccgactcca ctacctcgca ggcattcccc ctccgcgcag 1860
   gaggaaacgg acagcttcaa tactggccgt tctcctcttc tgacctttac aactggaaaa 1920
   ataataaccc ttctttttct gaagatccag gtaaactgac agctctgatc gagtctgtcc 1980
   tcatcaccca tcagcccacc tgggacgact gtcagcagct gttggggact ctgctgaccg 2040
   gagaagaaaa acaacgggtg ctcttagagg ctagaaaggc ggtgcggggc gatgatgggc 2100
   gccccactca actgcccaat gaagtcgatg ccgcttttcc cctcgagcgc ccagactggg 2160
   attacaccac ccaggcaggt aggaaccacc tagtccacta tcgccagttg ctcctagcgg 2220
   gtctccaaaa cgcgggcaga agccccacca atttggccaa ggtaaaagga ataacacaag 2280
   ggcccaatga gtctccctcg gccttcctag agagacttaa ggaagcctat cgcaggtaca 2340
   ctccttatga ccctgaggac ccagggcaag aaactaatgt gtctatgtct ttcatttggc 2400
   agtctgcccc agacattggg agaaagttag agaggttaga agatttaaaa aacaagacgc 2460
   ttggagattt ggttagagag gcagaaaaga tctttaataa acgagaaacc ccggaagaaa 2520
   gagaggaacg tatcaggaga gaaacagagg aaaaagaaga acgccgtagg acagaggatg 2580
   agcagaaaga gaaagaaaga gatcgtagga gacatagaga gatgagcaag ctattggcca 2640
   ctgtcgttag tggacagaaa caggatagac agggaggaga acgaaggagg tcccaactcg 2700
   atcgcgacca gtgtgcctac tgcaaagaaa aggggcactg ggctaaagat tgtcccaaga 2760
   aaccacgagg acctcgggga ccaagacccc agacctccct cctgacccta gatgactagg 2820
   gaggtcaggg tcaggagccc ccccctgaac ccaggataac cctcaaagtc ggggggcaac 2880
   ccgtcacctt cctggtagat actggggccc aacactccgt gctgacccaa aatcctggac 2940
   ccctaagtga taagtctgcc tgggtccaag gggctactgg aggaaagcgg tatcgctgga 3000
   ccacggatcg caaagtacat ctagctaccg gtaaggtcac ccactctttc ctccatgtac 3060
   cagactgtcc ctatcctctg ttaggaagag atttgctgac taaactaaaa gcccaaatcc 3120
   actttgaggg atcaggagcc caggttatgg gaccaatggg gcagcccctg caagtgttga 3180
   ccctaaatat agaagatgag tatcggctac atgagacctc aaaagagcca gatgtttctc 3240
   tagggtccac atggctgtct gattttcctc aggcctgggc ggaaaccggg ggcatgggac 3300
   tggcagttcg ccaagctcct ctgatcatac ctctgaaagc aacctctacc cccgtgtcca 3360
   taaaacaata ccccatgtca caagaagcca gactggggat caagccccac atacagagac 3420
   tgttggacca gggaatactg gtaccctgcc agtccccctg gaacacgccc ctgctacccg 3480
   ttaagaaacc agggactaat gattataggc ctgtccagga tctgagagaa gtcaacaagc 3540
   gggtggaaga catccacccc accgtgccca acccttacaa cctcttgagc gggctcccac 3600
   cgtcccacca gtggtacact gtgcttgatt taaaggatgc ctttttctgc ctgagactcc 3660
   accccaccag tcagcctctc ttcgcctttg agtggagaga tccagagatg ggaatctcag 3720
   gacaattgac ctggaccaga ctcccacagg gtttcaaaaa cagtcccacc ctgtttgatg 3780
   aggcactgca cagagaccta gcagacttcc ggatccagca cccagacttg atcctgctac 3840
   agtacgtgga tgacttactg ctggccgcca cttctgagct agactgccaa caaggtactc 3900
   gggccctgtt acaaacccta gggaacctcg ggtatcgggc ctcggccaag aaagcccaaa 3960
   tttgccagaa acaggtcaag tatctggggt atcttctaaa agagggtcag agatggctga 4020
   ctgaggccag aaaagagact gtgatggggc agcctactcc gaagacccct cgacaactaa 4080
   gggagttcct agggacggca ggcttctgtc gcctctggat ccctgggttt gcagaaatgg 4140
   cagccccctt gtaccctctc accaaaacgg ggactctgtt taattggggc ccagaccaac 4200
   aaaaggccta tcaagaaatc aagcaagctc ttctaactgc cccagccctg gggttgccag 4260
   atttgactaa gccctttgaa ctctttgtcg acgagaagca gggctacgcc aaaggtgtcc 4320
   taacgcaaaa actgggacct tggcgtcggc cggtggccta cctgtccaaa aagctagacc 4380
   cagtagcagc tgggtggccc ccttgcctac ggatggtagc agccattgcc gtactgacaa 4440
   aggatgcagg caagctaacc atgggacagc cactagtcat tctggccccc catgcagtag 4500
   aggcactagt caaacaaccc cccgaccgct ggctttccaa cgcccggatg actcactatc 4560
   aggccttgct tttggacacg gaccgggtcc agttcggacc ggtggtagcc ctgaacccgg 4620
   ctacgctgct cccactgcct gaggaagggc tgcaacacaa ctgccttgat atcctggccg 4680
   aagcccacgg aacccgaccc gacctaacgg accagccgct cccagacgcc gaccacacct 4740
   ggtacacgga tggaagcagt ctcttacaag agggacagcg taaggcggga gctgcggtga 4800
   ccaccgagac cgaggtaatc tgggctaaag ccctgccagc cgggacatcc gctcagcggg 4860
   ctgaactgat agcactcacc caggccctaa agatggcaga aggtaagaag ctaaatgttt 4920
   atactgatag ccgttatgct tttgctactg cccatatcca tggagaaata tacagaaggc 4980
   gtgggttgct cacatcagaa ggcaaagaga tcaaaaataa agacgagatc ttggccctac 5040
   taaaagccct ctttctgccc aaaagactta gcataatcca ttgtccagga catcaaaagg 5100
   gacacagcgc cgaggctaga ggcaaccgga tggctgacca agcggcccga aaggcagcca 5160
   tcacagagac tccagacacc tctaccctcc tcatagaaaa ttcatcaccc tacacctcag 5220
   aacattttca ttacacagtg actgatataa aggacctaac caagttgggg gccatttatg 5280
   ataaaacaaa gaagtattgg gtctaccaag gaaaacctgt gatgcctgac cagtttactt 5340
   ttgaattatt agactttctt catcagctga ctcacctcag cttctcaaaa atgaaggctc 5400
   tcctagagag aagccacagt ccctactaca tgctgaaccg ggatcgaaca ctcaaaaata 5460
   tcactgagac ctgcaaagct tgtgcacaag tcaacgccag caagtctgcc gttaaacagg 5520
   gaactagggt ccgcgggcat cggcccggca ctcattggga gatcgatttc accgagataa 5580
   agcccggatt gtatggctat aaatatcttc tagtttttat agataccttt tctggctgga 5640
   tagaagcctt cccaaccaag aaagaaaccg ccaaggtcgt aaccaagaag ctactagagg 5700
   agatcttccc caggttcggc atgcctcagg tattgggaac tgacaatggg cctgccttcg 5760
   tctccaaggt gagtcagaca gtggccgatc tgttggggat tgattggaaa ttacattgtg 5820
   catacagacc ccaaagctca ggccaggtag aaagaatgaa tagaaccatc aaggagactt 5880
   taactaaatt aacgcttgca actggctcta gagactgggt gctcctactc cccttagccc 5940
   tgtaccgagc ccgcaacacg ccgggccccc atggcctcac cccatatgag atcttatatg 6000
   gggcaccccc gccccttgta aacttccctg accctgacat gacaagagtt actaacagcc 6060
   cctctctcca agctcactta caggctctct acttagtcca gcacgaagtc tggagacctc 6120
   tggcggcagc ctaccaagaa caactggacc gaccggtggt acctcaccct taccgagtcg 6180
   gcgacacagt gtgggtccgc cgacaccaga ctaagaacct agaacctcgc tggaaaggac 6240
   cttacacagt cctgctgacc acccccaccg ccctcaaagt agacggcatc gcagcttgga 6300
   tacacgccgc ccacgtgaag gctgccgacc ccgggggtgg accatcctct agactgacat 6360
   ggcgcgttca acgctctcaa aaccccctca agataagatt aacccgtgga agcccttaat 6420
   agtcatggga gtcctgttag gagtagggat ggcagagagc ccccatcagg tctttaatgt 6480
   aacctggaga gtcaccaacc tgatgactgg gcgtaccgcc aatgccacct ccctcctggg 6540
   aactgtacaa gatgccttcc caaaattata ttttgatcta tgtgatctgg tcggagagga 6600
   gtgggaccct tcagaccagg aaccgtatgt cgggtatggc tgcaagtacc ccgcagggag 6660
   acagcggacc cggacttttg acttttacgt gtgccctggg cataccgtaa agtcggggtg 6720
   tgggggacca ggagagggct actgtggtaa atgggggtgt gaaaccaccg gacaggctta 6780
   ctggaagccc acatcatcgt gggacctaat ctcccttaag cgcggtaaca ccccctggga 6840
   cacgggatgc tctaaagttg cctgtggccc ctgctacgac ctctccaaag tatccaattc 6900
   cttccaaggg gctactcgag ggggcagatg caaccctcta gtcctagaat tcactgatgc 6960
   aggaaaaaag gctaactggg acgggcccaa atcgtgggga ctgagactgt accggacagg 7020
   aacagatcct attaccatgt tctccctgac ccggcaggtc cttaatgtgg gaccccgagt 7080
   ccccataggg cccaacccag tattacccga ccaaagactc ccttcctcac caatagagat 7140
   tgtaccggct ccacagccac ctagccccct caataccagt tacccccctt ccactaccag 7200
   tacaccctca acctccccta caagtccaag tgtcccacag ccacccccag gaactggaga 7260
   tagactacta gctctagtca aaggagccta tcaggcgctt aacctcacca atcccgacaa 7320
   gacccaagaa tgttggctgt gcttagtgtc gggacctcct tattacgaag gagtagcggt 7380
   cgtgggcact tataccaatc attccaccgc tccggccaac tgtacggcca cttcccaaca 7440
   taagcttacc ctatctgaag tgacaggaca gggcctatgc atgggggcag tacctaaaac 7500
   tcaccaggcc ttatgtaaca ccacccaaag cgccggctca ggatcctact accttgcagc 7560
   acccgccgga acaatgtggg cttgcagcac tggattgact ccctgcttgt ccaccacggt 7620
   gctcaatcta accacagatt attgtgtatt agttgaactc tggcccagag taatttacca 7680
   ctcccccgat tatatgtatg gtcagcttga acagcgtacc aaatataaaa gagagccagt 7740
   atcattgacc ctggcccttc tactaggagg attaaccatg ggagggattg cagctggaat 7800
   agggacgggg accactgcct taattaaaac ccagcagttt gagcagcttc atgccgctat 7860
   ccagacagac ctcaacgaag tcgaaaagtc aattaccaac ctagaaaagt cactgacctc 7920
   gttgtctgaa gtagtcctac agaaccgcag aggcctagat ttgctattcc taaaggaggg 7980
   aggtctctgc gcagccctaa aagaagaatg ttgtttttat gcagaccaca cggggctagt 8040
   gagagacagc atggccaaat taagagaaag gcttaatcag agacaaaaac tatttgagac 8100
   aggccaagga tggttcgaag ggctgtttaa tagatccccc tggtttacca ccttaatctc 8160
   caccatcatg ggacctctaa tagtactctt actgatctta ctctttggac cttgcattct 8220
   caatcgattg gtccaatttg ttaaagacag gatctcagtg gtccaggctc tggttttgac 8280
   tcagcaatat caccagctaa aacccataga gtacgagcca tgaacgcgtt actggccgaa 8340
   gccgcttgga ataaggccgg tgtgcgtttg tctatatgtt attttccacc atattgccgt 8400
   cttttggcaa tgtgagggcc cggaaacctg gccctgtctt cttgacgagc attcctaggg 8460
   gtctttcccc tctcgccaaa ggaatgcaag gtctgttgaa tgtcgtgaag gaagcagttc 8520
   ctctggaagc ttcttgaaga caaacaacgt ctgtagcgac cctttgcagg cagcggaacc 8580
   ccccacctgg cgacaggtgc ctctgcggcc aaaagccacg tgtataagat acacctgcaa 8640
   aggcggcaca accccagtgc cacgttgtga gttggatagt tgtggaaaga gtcaaatggc 8700
   tctcctcaag cgtattcaac aaggggctga aggatgccca gaaggtaccc cattgtatgg 8760
   gatctgatct ggggcctcgg tgcacatgct ttacatgtgt ttagtcgagg ttaaaaaaac 8820
   gtctaggccc cccgaaccac ggggacgtgg ttttcctttg aaaaacacga ttataaatgg 8880
   tgaccggcgg catggcctcc aagtgggatc aaaagggcat ggatatcgct tacgaggagg 8940
   ccctgctggg ctacaaggag ggcggcgtgc ctatcggcgg ctgtctgatc aacaacaagg 9000
   acggcagtgt gctgggcagg ggccacaaca tgaggttcca gaagggctcc gccaccctgc 9060
   acggcgagat ctccaccctg gagaactgtg gcaggctgga gggcaaggtg tacaaggaca 9120
   ccaccctgta caccaccctg tccccttgtg acatgtgtac cggcgctatc atcatgtacg 9180
   gcatccctag gtgtgtgatc ggcgagaacg tgaacttcaa gtccaagggc gagaagtacc 9240
   tgcaaaccag gggccacgag gtggtggttg ttgacgatga gaggtgtaag aagctgatga 9300
   agcagttcat cgacgagagg cctcaggact ggttcgagga tatcggcgag taagcggccg 9360
   cagataaaat aaaagatttt atttagtctc cagaaaaagg ggggaatgaa agaccccacc 9420
   tgtaggtttg gcaagctagc ttaagtaacg ccattttgca aggcatggaa aaatacataa 9480
   ctgagaatag agaagttcag atcaaggtca ggaacagatg gaacagctga atatgggcca 9540
   aacaggatat ctgtggtaag cagttcctgc cccggctcag ggccaagaac agatggaaca 9600
   gctgaatatg ggccaaacag gatatctgtg gtaagcagtt cctgccccgg ctcagggcca 9660
   agaacagatg gtccccagat gcggtccagc cctcagcagt ttctagagaa ccatcagatg 9720
   tttccagggt gccccaagga cctgaaatga ccctgtgcct tatttgaact aaccaatcag 9780
   ttcgcttctc gcttctgttc gcgcgcttct gctccccgag ctcaataaaa gagcccacaa 9840
   cccctcactc ggggcgccag tcctccgatt gactgagtcg cccgggtacc cgtgtatcca 9900
   ataaaccctc ttgcagttgc atccgacttg tggtctcgct gttccttggg agggtctcct 9960
   ctgagtgatt gactacccgt cagcgggggt ctttcattac atgtgagcaa aaggccagca 10020
   aaaggccagg aaccgtaaaa aggccgcgtt gctggcgttt ttccataggc tccgcccccc 10080
   tgacgagcat cacaaaaatc gacgctcaag tcagaggtgg cgaaacccga caggactata 10140
   aagataccag gcgtttcccc ctggaagctc cctcgtgcgc tctcctgttc cgaccctgcc 10200
   gcttaccgga tacctgtccg cctttctccc ttcgggaagc gtggcgcttt ctcatagctc 10260
   acgctgtagg tatctcagtt cggtgtaggt cgttcgctcc aagctgggct gtgtgcacga 10320
   accccccgtt cagcccgacc gctgcgcctt atccggtaac tatcgtcttg agtccaaccc 10380
   ggtaagacac gacttatcgc cactggcagc agccactggt aacaggatta gcagagcgag 10440
   gtatgtaggc ggtgctacag agttcttgaa gtggtggcct aactacggct acactagaag 10500
   gacagtattt ggtatctgcg ctctgctgaa gccagttacc ttcggaaaaa gagttggtag 10560
   ctcttgatcc ggcaaacaaa ccaccgctgg tagcggtggt ttttttgttt gcaagcagca 10620
   gattacgcgc agaaaaaaag gatctcaaga agatcctttg atcttttcta cggggtctga 10680
   cgctcagtgg aacgaaaact cacgttaagg gattttggtc atgagattat caaaaaggat 10740
   cttcacctag atccttttaa attaaaaatg aagttttaaa tcaatctaaa gtatatatga 10800
   gtaaacttgg tctgacagtt accaatgctt aatcagtgag gcacctatct cagcgatctg 10860
   tctatttcgt tcatccatag ttgcctgact ccccgtcgtg tagataacta cgatacggga 10920
   gggcttacca tctggcccca gtgctgcaat gataccgcga gacccacgct caccggctcc 10980
   agatttatca gcaataaacc agccagccgg aagggccgag cgcagaagtg gtcctgcaac 11040
   tttatccgcc tccatccagt ctattaattg ttgccgggaa gctagagtaa gtagttcgcc 11100
   agttaatagt ttgcgcaacg ttgttgccat tgctgcaggc atcgtggtgt cacgctcgtc 11160
   gtttggtatg gcttcattca gctccggttc ccaacgatca aggcgagtta catgatcccc 11220
   catgttgtgc aaaaaagcgg ttagctcctt cggtcctccg atcgttgtca gaagtaagtt 11280
   ggccgcagtg ttatcactca tggttatggc agcactgcat aattctctta ctgtcatgcc 11340
   atccgtaaga tgcttttctg tgactggtga gtactcaacc aagtcattct gagaatagtg 11400
   tatgcggcga ccgagttgct cttgcccggc gtcaacacgg gataataccg cgccacatag 11460
   cagaacttta aaagtgctca tcattggaaa acgttcttcg gggcgaaaac tctcaaggat 11520
   cttaccgctg ttgagatcca gttcgatgta acccactcgt gcacccaact gatcttcagc 11580
   atcttttact ttcaccagcg tttctgggtg agcaaaaaca ggaaggcaaa atgccgcaaa 11640
   aaagggaata agggcgacac ggaaatgttg aatactcata ctcttccttt ttcaatatta 11700
   ttgaagcatt tatcagggtt attgtctcat gagcggatac atatttgaat gtatttagaa 11760
   aaataaacaa ataggggttc cgcgcacatt tccccgaaaa gtgccacctg acgtctaaga 11820
   aaccattatt atcatgacat taacctataa aaataggcgt atcacgaggc cctttcgtct 11880
   tcaagaattc cat 11893
<210> 10
   <211> 8332
   <212> DNA
   <213> Moloney murine Leukemia virus
<400> 10
<210> 11
   <211> 8185
   <212> DNA
   <213> Xenotropic murine leukemia virus
<400> 11
   gcgccagtca tccgatagac tgagtcgccc gggtacccgt gttcccaata aagccttttg 60
   ctgtttgcat ccgaagcgtg gcctcgctgt tccttgggag ggtctcctca gagtgattga 120
   ctacccagct cgggggtctt tcatttgggg gctcgtccgg gattcggaga cccccgccca 180
   gggaccaccg acccaccgtc gggaggtaag ccggccggcg atcgttttgt ctttgtctct 240
   gtctttgtgc gtgtgtgtgt gtgccggcat ctaatcctcg cgcctgcgtc tgaatctgta 300
   ctagttagct aactagatct gtatctggcg gttccgcgga agaactgacg agttcgtatt 360
   cccggccgca gccctgggag acgtcccagc ggcctcgggg gcccgttttg tggcccattc 420
   tgtatcagtt aacctacccg agtcggactt tttggagtgg ctttgttggg ggacgagaga 480
   cagagacact tcccgccccc gtctgaattt ttgctttcgg ttttacgccg aaaccgcgcc 540
   gcgcgtctga tttgttttgt tgttcttctg ttcttcgtta gttttcttct gtctttaagt 600
   gttctcgaga tcatgggaca gaccgtaact acccctctga gtctaacctt gcagcactgg 660
   ggagatgtcc agcgcattgc atccaaccag tctgtggatg tcaagaagag gcgctgggtt 720
   accttctgtt ccgccgaatg gccaactttc aatgtaggat ggcctcagga tggtactttt 780
   aatttaggtg ttatctctca ggtcaagtct agagtgtttt gtcctggtcc ccacggacac 840
   ccggatcagg tcccatatat cgtcacctgg gaggcacttg cctatgaccc ccctccgtgg 900
   gtcaaaccgt ttgtctctcc taaaccccct cctttaccga cagctcccgt cctcccgccc 960
   ggtccttctg cgcaacctcc gtcccgatct gccctttacc ctgcccttac cccctctata 1020
   aagtccaaac ctcctaagcc ccaggttctc cctgatagcg gcggacctct cattgacctt 1080
   ctcacagagg atcccccgcc gtacggagca caaccttcct cctctgccag ggagaacaat 1140
   gaagaagagg cggccaccac ctccgaggtt tccccccctt ctcccatggt gtctcgactg 1200
   cggggaagga gagaccctcc cgcagcggac tccaccacct cccaggcatt cccactccgc 1260
   atggggggag atggccagct tcagtactgg ccgttttcct cctctgattt atataattgg 1320
   aaaaataata acccttcctt ttctgaagat ccaggtaaat tgacggcctt gattgagtcc 1380
   gtcctcatca cccaccagcc cacctgggac gactgtcagc agttgttggg gaccctgctg 1440
   accggagaag aaaagcagcg ggtgctccta gaggctagaa aggcagtccg gggcaatgat 1500
   ggacgcccca ctcagttgcc taatgaagtc aatgctgctt ttccccttga gcgccccgat 1560
   tgggattaca ccactacaga aggtaggaac cacctagtcc tctaccgcca gttgctctta 1620
   gcgggtctcc aaaacgcggg caggagcccc accaatttgg ccaaggtaaa agggataacc 1680
   cagggaccta atgagtctcc ctcagccttt ttagagagac tcaaggaggc ctatcgcagg 1740
   tacactcctt atgaccctga ggacccaggg caagaaacca atgtgtccat gtcattcatc 1800
   tggcagtctg ccccggatat cggacgaaag ttagagcggt tagaagattt aaagagcaag 1860
   accttaggag acttagtgag ggaagctgaa aagatcttta ataagcgaga aaccccggaa 1920
   gaaagagagg aacgtatcag gagagaaata gaggaaaaag aagaacgccg tagggcagag 1980
   gatgagcaga gagagagaga aagggaccgc agaagacata gagagatgag caagctcttg 2040
   gccactgtag ttattggtca gagacaggat agacaggggg gagagcggag gaggccccaa 2100
   cttgataagg accaatgcgc ctactgcaaa gaaaagggac actgggctaa ggactgccca 2160
   aagaagccac gagggccccg aggaccgagg ccccagacct ccctcctgac cttaggtgac 2220
   tagggaggtc agggtcagga gcccccccct gaacccagga taaccctcaa agtcgggggg 2280
   caacccgtca ccttcctggt agatactggg gcccaacact ccgtgctgac ccaaaatcct 2340
   ggacccctaa gtgacaagtc tgcctgggtc caaggggcta ctggaggaaa gcggtatcgc 2400
   tggaccacgg atcgcaaagt acatctggct accggtaagg tcacccactc tttcctccat 2460
   gtaccagact gcccctatcc tctgctagga agagacttgc tgactaaact aaaagcccaa 2520
   atccactttg agggatcagg agctcaggtt gtgggaccga tgggacagcc cctgcaagtg 2580
   ctgacagtaa acatagaaga tgagtattgg ctacatgata ccaggaaaga gccagatgtt 2640
   cctctagggt ccacatggct ttctgatttc cttcaggcct gggcggaaac cgggggcatg 2700
   ggactggcag ttcgccaagc tcctctgatc atacctctga aggcaacctc tacccccgtg 2760
   tccataaaac aataccccat gtcacaagaa gccagactgg ggatcaagcc ccacatacag 2820
   aggctgttgg accagggaat actggtaccc tgccagtccc cctggaacac gcccctgcta     2880
   cccgttaaga aaccagggac taatgattat aggcctgtcc aggatctgag agaagtcaac     2940
   aagcgggtgg aagacatcca ccccaccgtg cccaaccctt acaacctctt gagcgggctc     3000
   ccaccgtccc accagtggta cactgtgctt gatttaaagg atgccttttt ctgcctgaga     3060
   ctccacccca ccagtcagcc tctcttcgcc tttgagtgga gagatccaga gatgggaatc     3120
   tcaggacaac tgacctggac cagactccca cagggtttca aaaacagtcc caccctgttt     3180
   gatgaggcac tgcacagaga cctagcagat ttccggatcc agcacccaga cttgatcctg     3240
   ctacagtacg tggatgactt actgctggcc gccacttctg agcaagactg ccaacgaggt     3300
   actcgggccc tattacaaac cctagggaac ctcgggtatc gggcctcggc caagaaagcc     3360
   caaatttgcc agaaacaggt caagtatctg gggtatctcc taaaagaggg acagagatgg     3420
   ctgactgagg ccagaaaaga gactgtgatg gggcagccca ctccgaagac ccctcgacaa     3480
   ctaagggagt tcctagggac ggcaggcttc tgtcgcctct ggatccctgg gtttgcagaa     3540
   atggcagccc ccttgtaccc tcttaccaaa acggggactc tgtttaattg gggcccagac     3600
   cagcaaaagg cctatcaaga aatcaaacag gctcttctaa ctgcccccgc cctgggattg     3660
   ccagatttga ctaagccctt tgaactcttt gtcgacgaga agcagggcta cgccaaaggc     3720
   gtcctaacgc aaaaactggg accttggcgt cggcctgtgg cctacctgtc caaaaagcta     3780
   gacccagtgg cagctgggtg gcccccttgc ctacggatgg tagcagccat tgccgttctg     3840
   acaaaaaatg caggcaagct aactatggga cagccgctag tcattctggc cccccatgcg     3900
   gtagaagcac tggtcaaaca accccctgac cgttggctat ccaatgcccg catgacccac     3960
   tatcaggcaa tgctcctgga tacagaccgg gttcagttcg gaccggtggt ggccctcaac     4020
   ccggccaccc tgctccccct accggaaaag gaagcccccc atgactgcct cgagatcttg     4080
   gctgagacgc acggaaccag accggacctc acggaccagc ccatcccaga cgctgattac     4140
   acttggtaca cagatggaag cagcttccta caagaaggac aacggagagc tggagcagcg     4200
   gtgactactg agaccgaggt aatctgggcg agggctctgc cggctggaac atccgcccaa     4260
   cgagccgaac tgatagcact cacccaagcc ttaaagatgg cagaaggtaa gaagctaaat     4320
   gtttacactg atagccgcta tgccttcgcc acggcccatg tccatggaga aatatatagg     4380
   aggcgagggt tgctgacctc agaaggcaga gaaattaaaa acaagaacga gatcttggcc     4440
   ttgctaaaag ctctctttct gcccaaacga cttagtataa ttcactgtcc aggacatcaa     4500
   aaaggaaaca gtgctgaggc cagaggcaac cgtatggcag atcaagcagc ccgagaggca     4560
   gccatgaagg cagttctaga aacctctaca ctcctcatag aggactcaac cccgtatacg     4620
   cctccccatt tccattacac cgaaacagat ctcaaaagac tacgggaact gggagccacc     4680
   tacaatcaga caaaaggata ttgggtccta caaggcaaac ctgtgatgcc cgatcagtcc     4740
   gtgtttgaac tgttagactc cctacacaga ctcacccatc tgagccctca aaagatgaag 4800
   gcactcctcg acagagaaga aagcccctac tacatgttaa accgggacag aactatccag 4860
   tatgtgactg agacctgcac cgcctgtgcc caagtaaatg ccagcaaagc caaaattggg 4920
   gcaggggtgc gagtacgcgg acatcggcca ggcacccatt gggaagttga tttcacggaa 4980
   gtaaagccag gactgtatgg gtacaagtac ctcctagtgt ttgtagacac cttctctggc 5040
   tgggtagagg cattcccgac caagcgggaa actgccaagg tcgtgtccaa aaagctgtta 5100
   gaagacattt ttccgagatt tggaatgccg caggtattgg gatctgataa cgggcctgcc 5160
   ttcgcctccc aggtaagtca gtcagtggcc gatttactgg ggatcgattg gaagttacat 5220
   tgtgcttata gaccccagag ttcaggacag gtagaaagaa tgaatagaac aattaaggag 5280
   actttgacca aattaacgct tgcatctggc actagagact gggtactcct actcccctta 5340
   gccctctacc gagcccggaa tactccgggc ccccacggac tgactccgta tgaaattctg 5400
   tatggggcac ccccgcccct tgtcaatttt catgatcctg aaatgtcaaa gttaactaat 5460
   agtccctctc tccaagctca cttacaggcc ctccaagcag tacaacaaga ggtctggaag 5520
   ccgctggccg ctgcttatca ggaccagcta gatcagccag tgataccaca ccccttccgt 5580
   gtcggtgacg ccgtgtgggt acgccggcac cagactaaga acttagaacc tcgctggaaa 5640
   ggaccctaca ccgtcctgct gacaaccccc accgctctca aagtagacgg catctctgcg 5700
   tggatacacg ccgctcacgt aaaggcggcg acaactcctc cggccggaac agcatggaaa 5760
   gtccagcgtt ctcaaaaccc cttaaagata agattaaccc gtggggcccc ctgataatta 5820
   tggggatctt ggtgagggca ggagcctcag tacaacgtga cagccctcac caggtcttta 5880
   atgtcacttg gaaaattacc aacctaatga caggacaaac agctaatgct acctccctcc 5940
   tggggacgat gacagacact ttccctaaac tatattttga cttgtgtgat ttagttggag 6000
   acaactggga tgacccggaa cccgatattg gagatggttg ccgctctccc gggggaagaa 6060
   aaaggacaag actatatgat ttctatgttt gccccggtca tactgtatta acagggtgtg 6120
   gagggccgag agagggctac tgtggcaaat ggggatgtga gaccactgga caggcatact 6180
   ggaagccatc atcatcatgg gacctaattt cccttaagcg aggaaacact cctaagggtc 6240
   agggcccctg ttttgattcc tcagtgggct ccggtagcat ccagggtgcc acaccggggg 6300
   gtcgatgcaa ccccctagtc ctagaattca ctgacgcggg taaaagggcc agctgggatg 6360
   cccccaaaac atggggacta agactgtatc gatccactgg ggccgacccg gtgaccctgt 6420
   tctctctgac ccgccaggtc ctcaatgtag ggccccgcgt ccccattggg cctaatcccg 6480
   tgatcactga acagctaccc ccctcccaac ccgtgcagat catgctcccc aggactcctc 6540
   gtcctcctcc ttcaggcgcg gcctctatgg tgcctggggc tcccccgcct tctcaacaac 6600
   ctgggacggg agacaggctg ctaaacctgg tagaaggagc ctacctagcc ctcaacctca 6660
   ccagtcccga caaaacccaa gagtgctggc tgtgtctagt atcgggaccc ccctactacg 6720
   aaggggtggc cgtcctaggt acttactcca accatacctc tgccccggct aactgctccg 6780
   tgacctccca acacaagctg accctgtccg aagtgaccgg gcagggactc tgcataggag 6840
   cagttcccaa aacccatcag gccctgtgta ataccaccca gaagacgagc gacgggtcct 6900
   actatttggc ctctcccgcc gggaccattt gggcttgcag caccgggctc actccctgtc 6960
   tatctactac tgtgcttaac ttaaccactg attactgtgt cctggttgaa ctctggccaa 7020
   aggtaaccta ccactcccct aattatgttt atggccagtt tgaaaagaaa actaaatata 7080
   aaagagagcc ggtgtcatta actctggccc tgctgttggg aggacttact atgggcggca 7140
   tagctgcagg agttggaaca gggactacag ccctagtggc caccaaacaa ttcgagcagc 7200
   tccaggcagc catacataca gaccttgggg ccttagaaaa atcagtcagt gccctagaaa 7260
   agtctctgac ctcgttgtct gaggtggtcc tacagaaccg gaggggatta gatctactgt 7320
   tcctaaaaga aggaggatta tgtgctgccc taaaagaaga atgctgtttt tacgcggacc 7380
   acactggcgt agtaagagat agcatggcaa agctaagaga aaggttaaac cagagacaaa 7440
   aattgttcga atcaggacaa gggtggtttg agggactgtt taacaggtcc ccatggttca 7500
   cgaccctgat atccaccatt atgggccctc tgatagtact tttattaatc ctactcttcg 7560
   gaccctgtat tctcaaccgc ttggtccagt ttgtaaaaga cagaatttcg gtagtgcagg 7620
   ccctggttct gacccaacag tatcaccaac tcaaatcaat agatccagaa gaagtggaat 7680
   cacgtgaata aaagatttta ttcagtttcc agaaagaggg gggaatgaaa gaccccacca 7740
   taaggcttag cacgctagct acagtaacgc cattttgcaa ggcatggaaa agtaccagag 7800
   ctgagttctc aaaagttaca aggaagttta attaaagaat aaggctgaat aacactggga 7860
   caggggccaa acaggatatc tgtagtcagg cacctgggcc ccggctcagg gccaagaaca 7920
   gatggtcctc agataaagcg aaactaacaa cagtttctgg aaagtcccac ctcagtttca 7980
   agttccccaa aagaccggga aataccccaa gccttattta aactaaccaa tcagctcgct 8040
   tctcgcttct gtacccgcgc tttttgctcc ccagtcctag ccctataaaa aaggggtaag 8100
   aactccacac tcggcgcgcc agtcatccga tagactgagt cgcccgggta cccgtgttcc 8160
   caataaagcc ttttgctgtt tgcaa 8185

## Description

### TECHNICAL FIELD

The present invention relates to a method for detecting anti-viral antibodies for amphophotropic murine leukemia virus or a murine leukemia virus-related virus in a biological sample. More generally, this disclosure relates to methods of detecting MLV related viruses, including retroviral replicating vectors, and detecting immune responses to such viruses using purified whole viral particles as the target antigen by ELISA, competitive binding, Western blot or other serological detection methods. The purified particles can also be used for stimulation of T cells to detect cellular immune response.

### BACKGROUND

There are several needs and advantages that come from the ability to detect the presence of MLV or MLV related viruses in human or mammalian subjects. Firstly therapies are currently being developed that employ retroviral replicating vectors (RRV) in human therapies, mainly for cancer (see WO2010036986 and (www).clinicaltrials.gov NCT01156584) and there is a need to follow the RRV replication in the blood stream in case this reaches levels considered potentially pathogenic and warrant pharmacological intervention (e.g., around several hundred thousand copies/ml for HIV). Quantitative or semi-quantitative seropositivity has been a useful way to follow ongoing viral infections in general, and retroviral infection in particular, and provides information on infection and active replication of such agents. However the specifics of individual RRV's may vary and it is possible that changes in the virus may occur after prolonged replication in humans or animals. Therefore it is important that an assay for monitoring such events be as broad-based as possible and not focus on one or a few antigenic epitopes. Secondly, it seems very likely that some individuals may be or have been infected with MLV related viruses. This is based on the observation that high level seropositivity to MLV related viruse has been clearly shown to exist in a small percentage of individuals (around 1%). Recently various publications suggest that several exogenous gammaretroviruses have been isolated from humans, and these are known as XMRV or MLV-related viruses (MLVRV, Urisman A, Molinaro RJ, et al. (2006) PLoS Pathog 2(3): e25., Lombardi V.c. et al. Science 2009, Lo et al PNAS 2010 (www).pnas.org/cgi/doi/10.1073/pnas.1006901107).

US2010167268 discloses methods of detecting, diagnosing, monitoring or managing an XMRV-related disease such as an XMRV-related neuroimmune disease such as chronic fatigue syndrome or an XMRV-related lymphoma such as mantle cell lymphoma in a subject.

### SUMMARY

The present invention provides a method for detecting anti-viral antibodies for amphotropic murine leukemia virus (MLV) or a murine leukemia virus-related virus (MLVRV) in a biological sample comprising: a) incubating a biological fluid sample with a capture reagent immobilized on a solid support to bind multiple anti-viral antibodies to amphotropic MLV or MLVRV to the capture reagent, wherein the capture reagent comprises a preparation of serum-free whole viral particles of MLV or MLVRV; b)detecting bound anti-viral antibodies bound to immobilized capture reagent by contacting the bound anti-viral antibodies with a labeled detecting secondary agent. In one embodiment, the biological sample is isolated from a mammal. In further embodiment, the mammal is an autoimmune, virally infected, immune suppressed or cancer patient. In yet another embodiment, the biological sample is isolated from a human. In a further embodiment, the human is an autoimmune, virally infected immune suppression or cancer patient. In one embodiment, the method further comprises measuring the amount of anti-viral antibody, wherein the amount of antibody is titred using a dilution series; wherein the amount of antibody determined is IgG or IgM by use of specific detecting secondary reagents. In one embodiment, the biological sample is plasma, serum, urine, saliva, bucchal swab, nasal swab, fine needle aspirate, semen, vaginal secretion or milk. In yet another embodiment, the capture reagent is immobilized at about 1.5 µg/mL. In one embodiment, the solid support is a microtiter plate, a glass slide, a nylon slide, a nitrocellulose membrane or a PVDF membrane. In another embodiment, the solid support is a latex bead, a glass bead or a microsphere. In a further embodiment, the solid support is a pipette tip, microfluidic card or semiconductor chip. In yet another embodiment, the secondary agent is a secondary antibody that is detectably labeled. In certain embodiment, the method further comprises a control comprising the monoclonal 83A25 mAb. In a further embodiment, the method further comprises a control comprising human serum or plasma from an individual who is strongly seropositive for MLV, identified by screening multiple human sera.

### FURTHER ASPECTS OF THE DISCLOSURE

The disclosure also provides an immunoassay kit for detecting anti-viral antibodies comprising: (a) a monoclonal antibody that specifically binds to amphotropic envelop as a control; and (b) a capture reagent comprising a purified viral particle. In one aspect of the disclosure, the solid support is a microtiter plate. In a further aspect of the disclosure, the solid support is a microsphere. In yet another aspect of the disclosure, the capture reagent is an MLV-like viral particle. In yet another aspect of the disclosure, the capture reagent is a purified protein of MLV origin. In yet another aspect of the disclosure, the capture reagent is a purified recombinant protein of MLV origin or MLV-like sequence. In another aspect of the disclosure, the positive control is human serum or plasma from an individual who is strongly seropositive to the capture antigen, and such serum has been identified by screening multiple human sera. In one aspect of the disclosure, the detectable antibody is a monoclonal antibody. In yet a further aspect of the disclosure, the detectable monoclonal antibody is a murine monoclonal antibody. In yet another aspect of the disclosure, the detectable antibody is a polyclonal antibody. In a further aspect of the disclosure, the detectable polyclonal antibody is a rabbit or goat or sheep or chicken polyclonal antibody. In any of the foregoing aspects of the disclosure, the the kit is used in a clinical or diagnostic setting. In one aspect of the disclosure, the kit is used to monitor the immune responses of MLV-infected or MLV-like infected or treated individuals.

In any of the foregoing methods or kits the purified viral particle may be produced by a method comprising transforming a 293 cell line with a plasmid encoding a retroviral MLV or MVL-like virus; culturing the 293 cell to produce viral particles; isolating the viral particles; infecting an HT1080 cell line with the viral particles thereby producing the viral particle producing cell line, producing the viral particles and purifying the viral particles.

The disclosure provides retrovirus producing cell lines for the production of a replication competent retrovirus particles, the cell line comprising a mammalian cell line such as a prostate cancer cell, a T cell line, a neural tumor line, a fibrosarcoma, an osteosarcoma a thymoma cell line, a fibroblast line or endothelial cell line, said cell line stably expressing a retroviral genome comprising a gag gene, *pol* gene, env gene, and retroviral *psi* (Ψ) factor for the assembly of the recombinant retroviral genome. In one aspect of the disclosure, the replication competent retrovirus particle is stably expressed. In another aspect of the disclosure the immortalized cell line is a human cell line. In another aspect of the disclosure the cell line is selected from one of:the prostate tumor cell lines LNCAP (ATCC#CRL-1740, DU145 (ATCC#HTB-81), or PC-3 (ATCC#CRL-1435); the human fibrosarcoma cell line HT1080 (ATCC#CCL-121); the Human glioma cell line U87-MG (ATCC#HTB-14); the human T cell line SupT1 cell line (ATCC#CRL-1942); the human cervical carcinoma line Hela, the african green monkey cell line CV1 or derivatives such as COS cells; HEK 293 and 293 derived cells; or CHO and CHO derived cells. In another aspect of the disclosure the cell line was isolated and already expressed the retrovirus. In a related aspect of the disclosure the cell line is 222Rv1 (ATCC CRL-2505, Knouf et al. J.Virol 83: 7353-7356, 2009)which makes the artifactual recombinant XMRV (T.Paprotka, et al. 2011, op.cit.). In another aspect of the disclosure, the half life is greater than 7 days at 2-8°C. In one aspect of the disclosure, the retroviral polynucleotide sequence is derived from one of murine leukemia virus (MLV), Moloney murine leukemia virus (MoMLV), amphotropic MoMLV, Xenotropic MLV related retrovirus (XMRV), MLV related virus (MLVRV) found in humans, xenotropic MLV, Polytropic MLV, amphotropic MLV, Gibbon ape leukemia virus (GALV), murine mammary tumor virus (MuMTV), Rous Sarcoma Virus (RSV), Gibbon ape leukemia virus (GALV), baboon endogenous virus (BEV), feline virus RD114, T5.0002 (WO2010045002A2) or other gamma retrovirus, or hybrids of the above. The genome of the retrovirus produced by the producer cell line or introduced into a cell line to make a producer cell line comprises various domains. For example, the promoter may comprise a CMV promoter having a sequence as set forth in SEQ ID NO:7, 8 or 9 from nucleotide 1 to about nucleotide 582 and may include modification to one or more nucleic acid bases and which is capable of directing and initiating transcription; a CMV-R-U5 domain polynucleotide comprises a sequence as set forth in SEQ ID NO: 7, 8 or 9 from about nucleotide 1 to about nucleotide 1202 or sequences that are at least 95% identical to a sequence as set forth in SEQ ID NO: 7, 8 or 9, wherein the polynucleotide promotes transcription of a nucleic acid molecule operably linked thereto; the gag nucleic acid domain comprises a sequence from about nucleotide number 1203 to about nucleotide 2819 of SEQ ID NO: 7 or 9 or a sequence having at least 60%, 70% 80%, 90%, 95%, 98%, 99% or 99.8% identity thereto; the pol domain comprises a sequence from about nucleotide number 2820 to about nucleotide 6358 of SEQ ID NO:7 or 9 or a sequence having at least 60%, 70% 80%, 90%, 95%, 98%, 99% or 99.8% identity thereto; the env domain comprises a sequence from about nucleotide number 6359 to about nucleotide 8323 of SEQ ID NO:7 or 9 or a sequence having at least 60%, 70% 80%, 90%, 95%, 98%, 99% or 99.8% identity thereto; the IRES and the heterologous nucleic acid are optional extra sequences that are used when the virus is being used as a vector.

The disclosure also provides a cell free preparation comprising purified viral particles obtained from the retrovirus producing cell line described herein. In some easpects of the disclosure, a preparation the isolated viral particles is used to make an ELISA or other antibody binding assay for testing human or animal blood, plasma, serum, urine, saliva, bucchal swab, nasal swab, fine needle aspirate, semen, vaginal secretion, milk or other bodily fluid or extract. Antibodies detected can be IgM, IgG, IgA, IgE or IgD, depending on the type of secondary antibody or other secondary binding reagent used in the assay.

The disclosure also provides a method of constructing a vector producing cell line described herein comprising transforming a 293 cell line with a plasmid encoding a retroviral vector comprising from 5' to 3': a CMV-R-U5 fusion of the immediate early promoter from human cytomegalovirus to an MLV R-U5 region; a PBS, primer binding site for reverse transcriptase; a 5' splice site; ψ packaging signal; a gag coding sequence for MLV group specific antigen; a pol coding sequence for MLV polymerase polyprotein; a 3' splice site; an env coding sequence for envelope protein; culturing the 293 cell to produce viral particles; isolating the viral particles; infecting a cell line with the viral particles thereby producing the viral particle producing cell line. The disclosure also provides plasmids where the promoter is not a CMV-R-U5 fusion of the immediate early promoter from human cytomegalovirus to an MLV R-U5 region but is a fusion of the MLV r-U5 region with and alternate promoter such as the RSV promoter or the actin promoter, or the intact MLV LTR with U3, R and U5 regions. The disclosure also provides cell lines generated by the foregoing methods. The disclosure also provides virus producing lines, made by these methods with HT1080 cells, PC3 cells, LNCaPcells, SupT1 cells, U87 cells, D17 cells, CF2 cells, 293 cells, Hela cells, CV1 cells, CHO cells or cell lines derived from any of these.

The disclosure also provides a method for producing a composition for testing for anti-retrovirus antibodies comprising culturing the cell lines described herein to produce viral particles and substantially purifying the viral particles.

The disclosure also provides cell banks comprising the cell lines of the disclosure. In some aspects of the disclosure, the cell lines of the disclosure are grown in suspension. In some aspects of the disclosure, the cell lines of the disclosure are grown in serum free medium. In some aspects of the disclosure, the cell line is grown in suspension in serum free medium.

### DESCRIPTION OF DRAWINGS

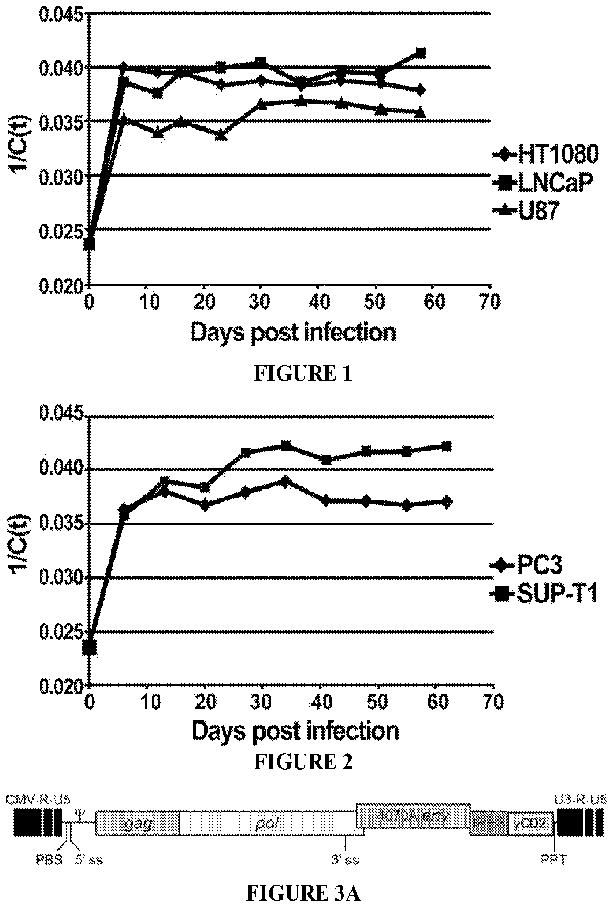

**Figure 1** shows the viral replication kinetics of 22Rv1 in HT1080, U87 and LNCaP cell lines.
**Figure 2** shows the viral replication kinetics of 22Rv1 in PC3 and Sup-T1 cell lines.
**Figure 3A-F** shows (a) a schematic of a recombinant retroviral vector of the disclosure; (b and c) a plasmid map of a polynucleotide of the disclosure (CMV Promotor:1 - 582; R:583 - 650; U5:651 - 1202; Primer binding site (PBS):728 - 776; 5' slicing site:788 - 789; gag:1203 - 2819; pol:2820 - 6358; 3'splicing site:3314 - 3315; 4070A env: 6359 - 8323; EMCV IRES:8327 - 8876; yCD2:8877 - 9353; Poly purine tract (PPT):9386 - 9404; U3:9405 - 9854; R:9855 - 9921; U5:9922 - 9998; (d and e) a sequence of a polynucleotide of the disclosure (SEQ ID NO:7); (f) a schematic of a first and second generation RCR of the disclosure.

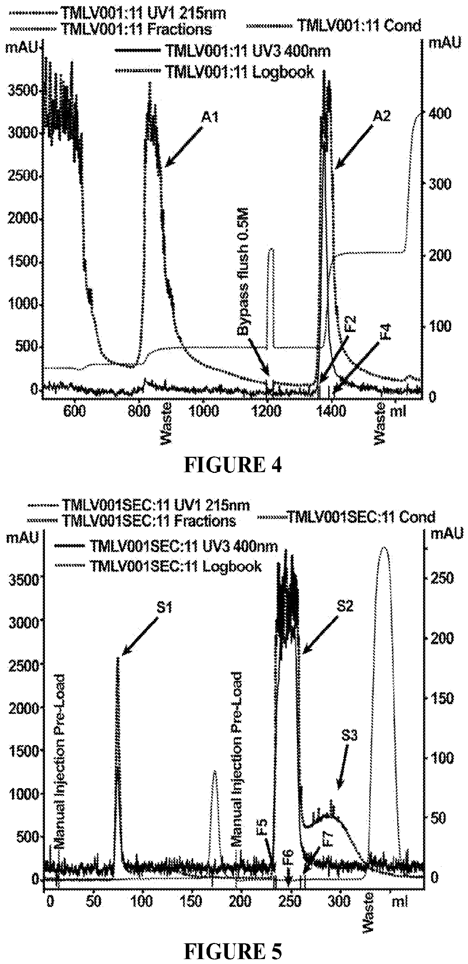

**Figure 4** is a chromatogram showing Anion Exchange (AEX) of amphotropic murine leukemic virus (MLV), Tocagen Lot #: TMLV001. The left part of chromatogram shows loading of harvested culture supernatant (x-scale is non-quatnitative). The middle part, starting about 800mL, shows a wash peak (A1) as a result of an intermediate increase in NaCl concentration. The right part of chromatogram shows elution with main target peak (A2) eluting from the AEX column just before 1400mL.
**Figure 5** shows a chromatogram showing Size Exclusion (SEC) of amphotropic MLV. Left part of chromatogram shows a pre-load of SEC column aimed at priming chromatography resin so as to minimize loss due to unwanted interactions between target and chromatography resin (Pre-load Peak: S1). Right part of chromatogram shows actual load and isocratic elution of target MLV. Main target peak (S2) is around 250mL and specifically excludes waste peak starting around 260-270mL (S3).

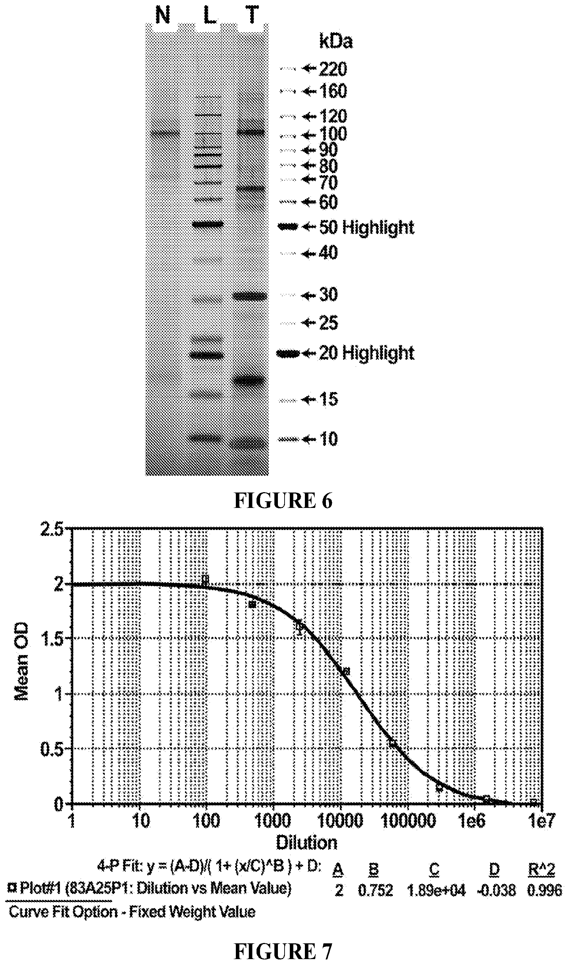

**Figure 6** shows a silver stained SDS-PAGE showing (T) purified wild-type amphotropic murine leukemia virus (MLV), Tocagen Lot #: TMLV001 and, (N) identically processed naive HT1080. Tocagen Lot #: TMLV001 and (N) processed naive HT1080 (N) treated using the identical process. SDS-PAGE run under reducing conditions and subsequently silver stained. Samples are from left to right: (N) Naive HT1080 cultured and processed analogously to TMLV001 (right lane), (L) Invitrogen Benchmark Protein Ladder (see right panel for protein sizes), (T) Purified wild-type amphotropic MLV (Tocagen TMLV001; from HT1080) having a TU titer of 5.4x10⁸/mL.
**Figure 7** shows a dose response curve of Assay Positive Control. Reactivity of assay positive control was determine using a cocktail of anti-rat Ig and anti-human IgG HRP conjugated antibody.

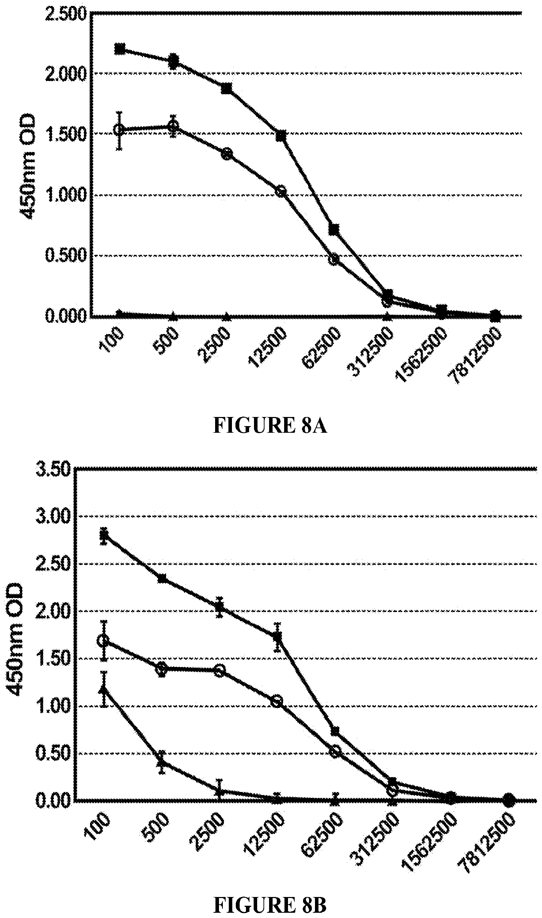

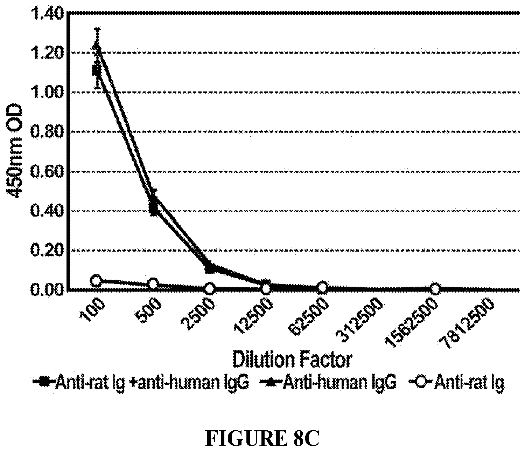

**Figure 8A-C** shows dose response curves of 83A25 and human serum (HS) alone or in combination with different secondary antibodies. Reactivity of 83A25 mAb and HS were assayed in the human anti-MLV ELISA assay and developed using anti-rat Ig, or anti-Human IgG HRP conjugated antibodies alone and the cocktail conjugate. A) 83A25 antibody alone: open circle anti-rat, black square anti- rat + anti- human, black triangle anti-human, B) 83A25 antibody spiked in HS: open circle anti-rat, black square anti- rat + anti- human, black triangle anti-human and C) HS alone: open circle anti-rat, black square anti- rat + anti- human, black triangle anti-human. 1:5 dilution factor was tested starting at 1:100 dilution.

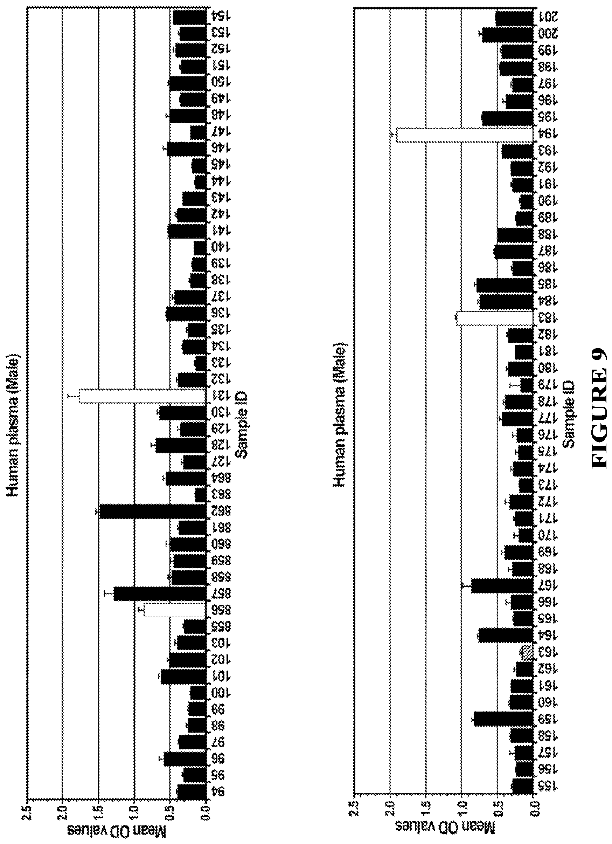

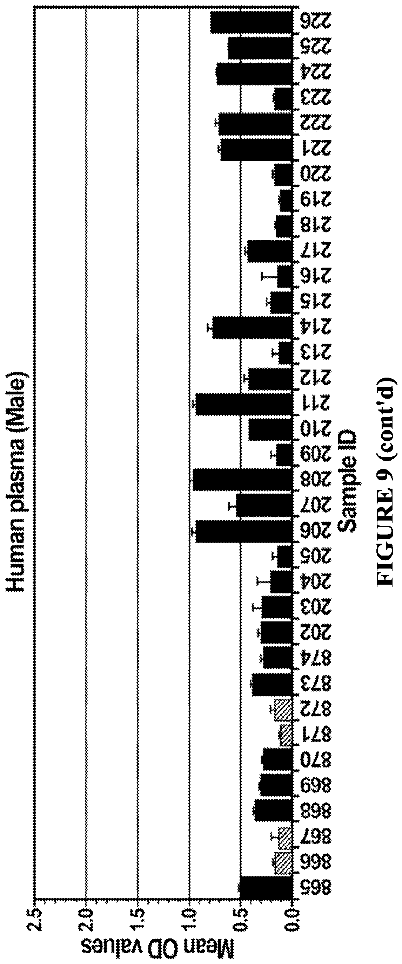

**Figure 9** shows plasmas selected from screened samples for generating the ELISA Negative Control and ELISA positive control and for testing by Western blot analysis. Human plasmas with intermediate and high OD₄₅₀ nm values were selected for analysis for anti-MLV antigens using Western blotting (thatched bars). Plasmas selected to generate the Negative Control plasma are also shown (black bars). Samples are displayed in relation to all plasmas screened using anti-MLV ELISA.

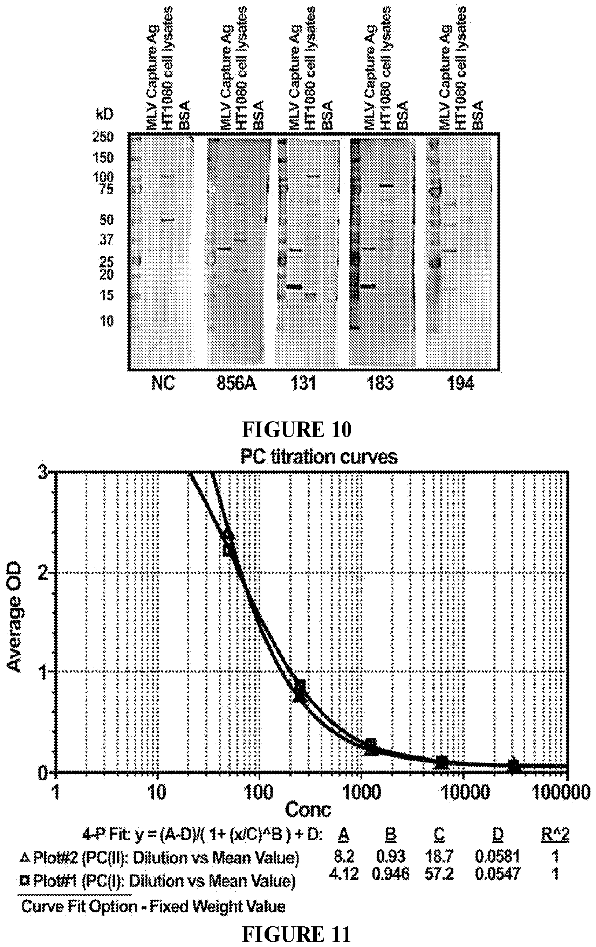

**Figure 10** shows confirmation of the presence of anti-MLV antibodies in human plasma samples by Western blot analysis. Plasmas with high (131, 194), intermediate (856A, 183) or low (NC) mean OD450nm values as tested by ELISA were tested for the presence of anti-MLV antibodies. Cell lysates from HT1080 cells and purified BSA were included in the assay as lane controls.
**Figure 11** shows titration curves of a human positive control plasma (PC) that was generated from donor 131 in the anti-MLV ELISA. Dilutions of PC plasma (Lot# OT11DV-68-30) were tested and are plotted against calculated mean OD450nm values for two separate replicates. PC titier was calculated at 1:250 for two PC replicates. A human matched species control is used as the positive control in the anti-MLV ELISA for human samples.

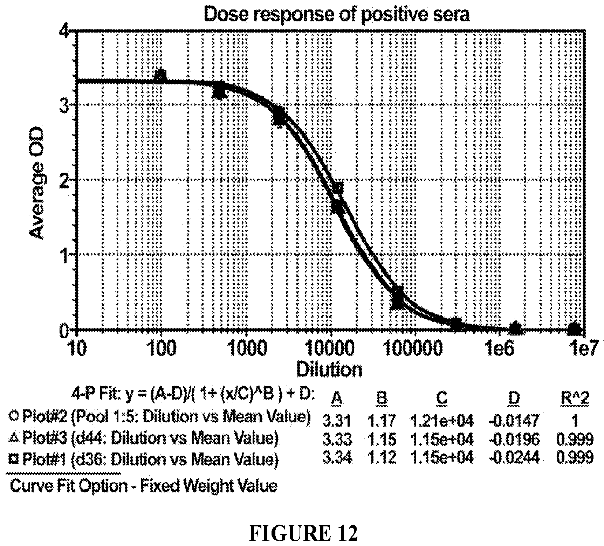

**Figure 12** shows a comparison of antibody detection by sera collected on day 36, day 44 and pooled d36+d44 sera from immunized dog. Positive serum from immunized dog and pooled negative serum from non-immunized dogs was tested for anti-MLV IgG antibodies using the MLV Capture Antigen. The X-axis represents the dilution series and absorbance units (OD) is represented on the Y-axis. 4-parameter fit analysis is performed in the Spectramax software. The parameters for the equation [Y=(A-D)/(1+(x/C)^B) + D] are listed in the figure. Note that parameter "C" is a direct machine output notation and the designation "e" is equivalent to designations of "E" within the document. R^2 is the efficiency value of the data fit.

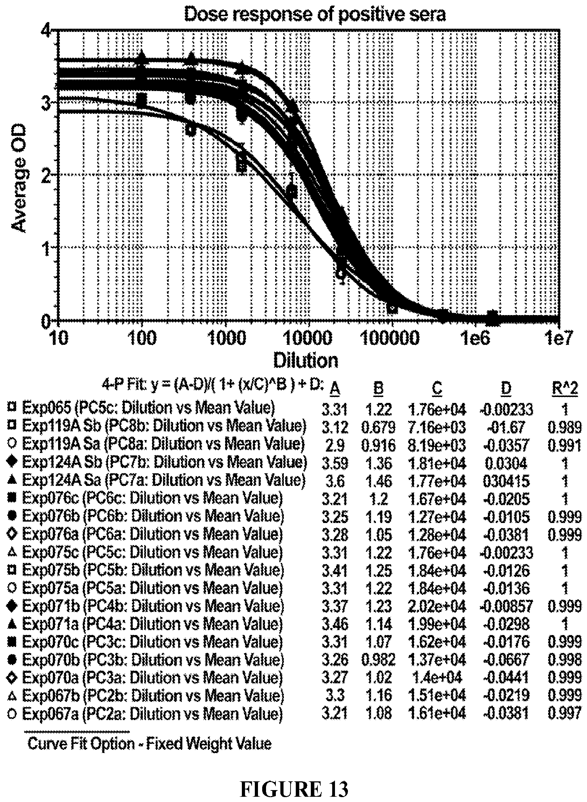

**Figure 13** shows a dose response of Positive Serum Control across several experiments. 4-parameter fit analysis is performed in the Spectramax software. The parameters for the equation [Y=(A-D)/(1+(x/C)^B) + D] are listed in the figure. Note that parameter "C" is a direct machine output notation and the designation "e" is equivalent to designations of "E" within the document. R^2 is the efficiency value of the data fit.

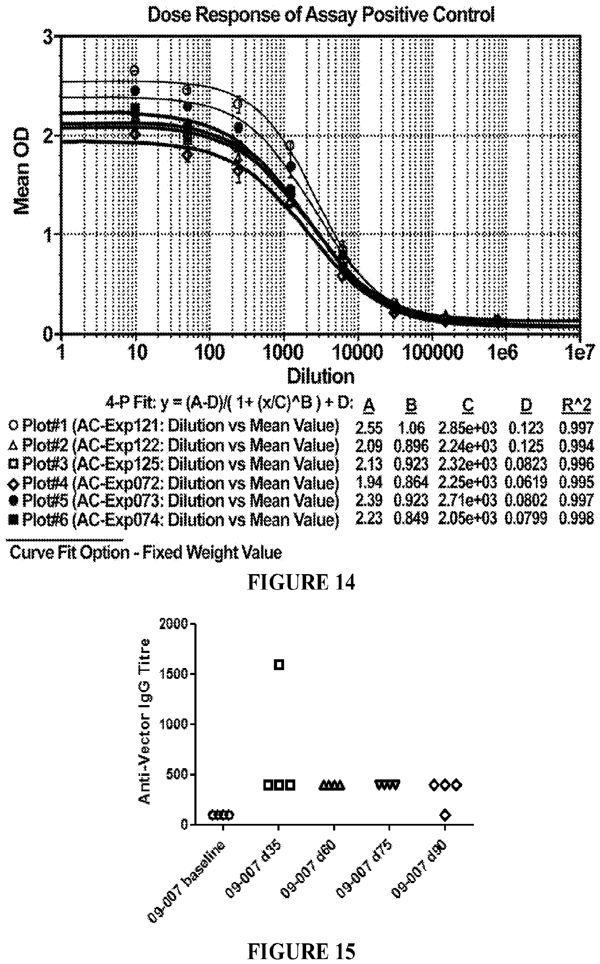

**Figure 14** shows a dose response of Assay Positive Serum Control across several experiments 4-parameter fit analysis is performed in the Spectramax software. The parameters for the equation [Y=(A-D)/(1+(x/C)^B) + D] are listed in the figure. Note that parameter "C" is a direct machine output notation and the designation "e" is equivalent to designations of "E" within the document. R^2 is the efficiency value of the data fit.
**Figure 15** depicts an anti-vector IgG titre for canines administered IC with Toca 511 E7 dose.

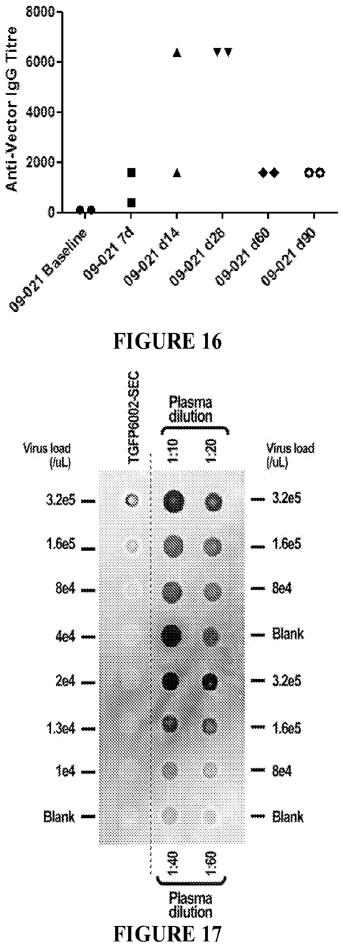

**Figure 16** depicts an anti-vector IgG titre for canines administered IV with Toca 511 E9 dose. Baseline, day 7, 14, 28, 60, and 90 are presented in the figure.
**Figure 17** shows a dot blot showing viral dilutions and spiked plasma blots.

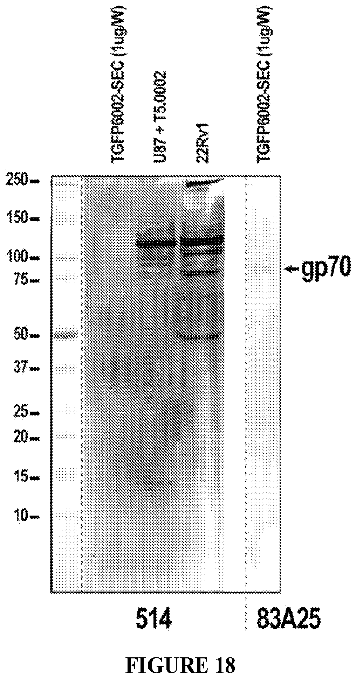

**Figure 18** shows detection of viral gp70 expression in MLV and XMRV samples using the mouse monoclonal anti-gp70 antibody (clone 514) by Western blotting.

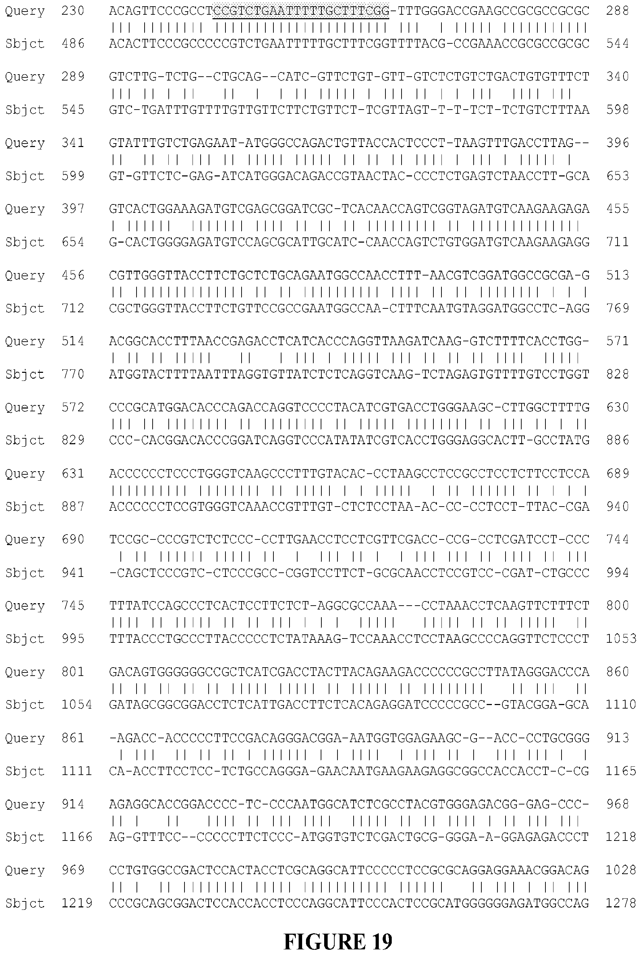

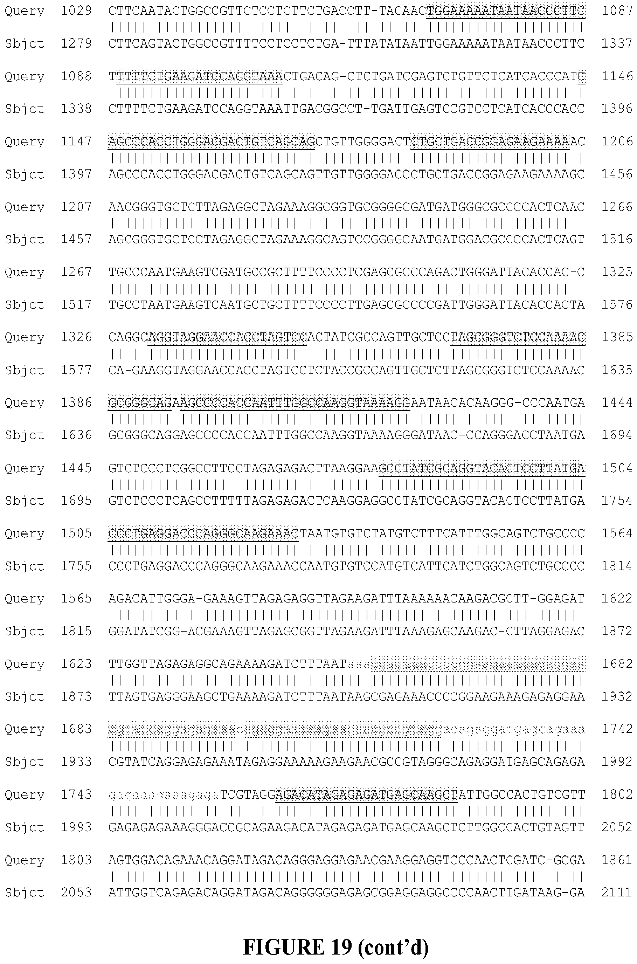

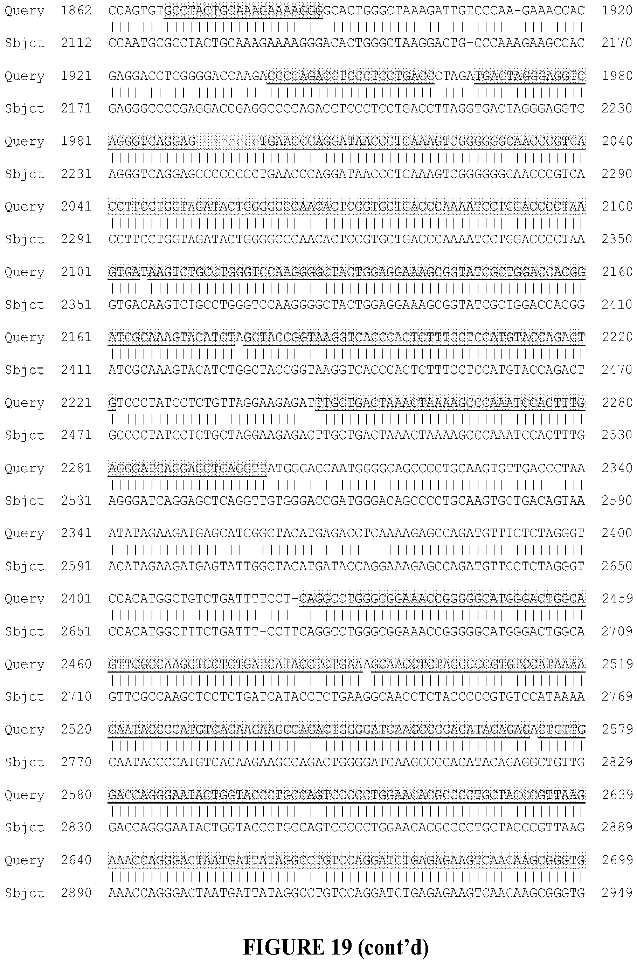

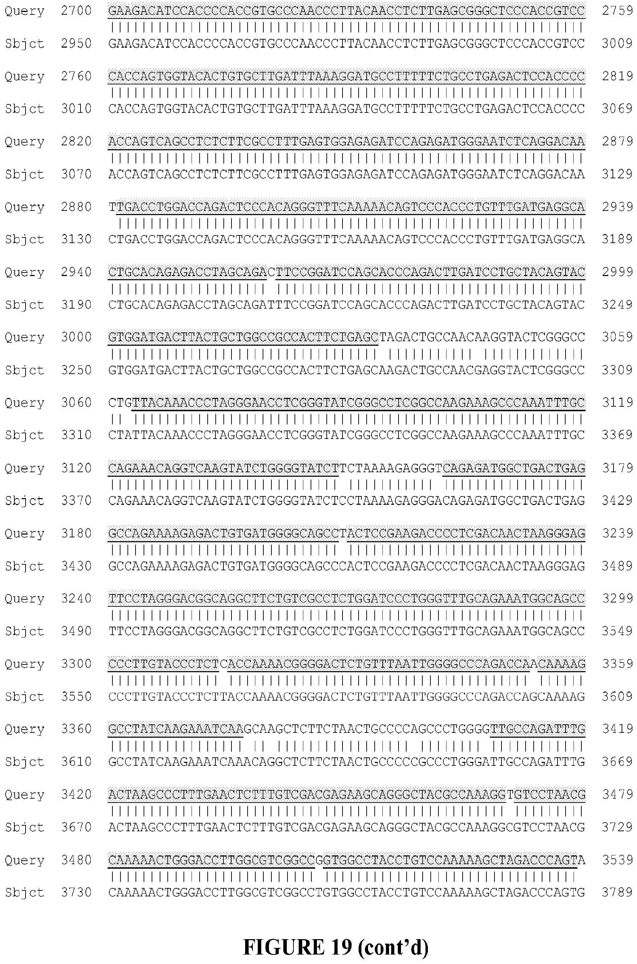

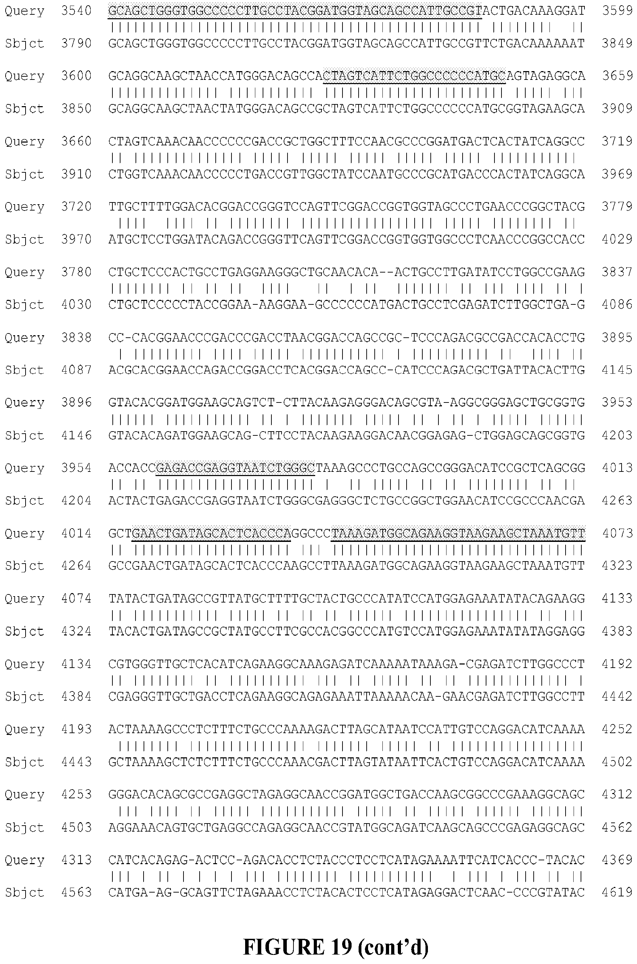

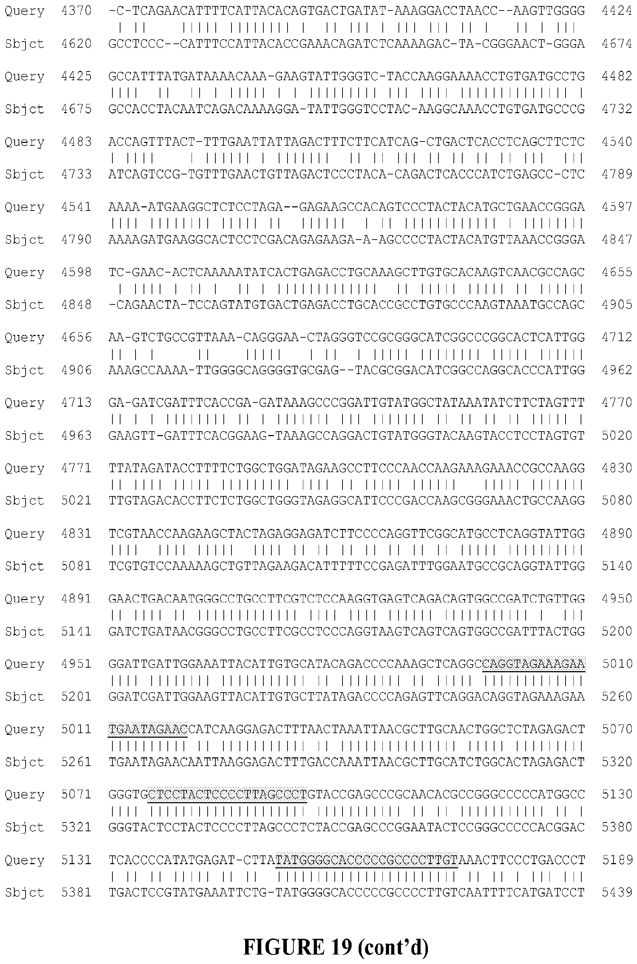

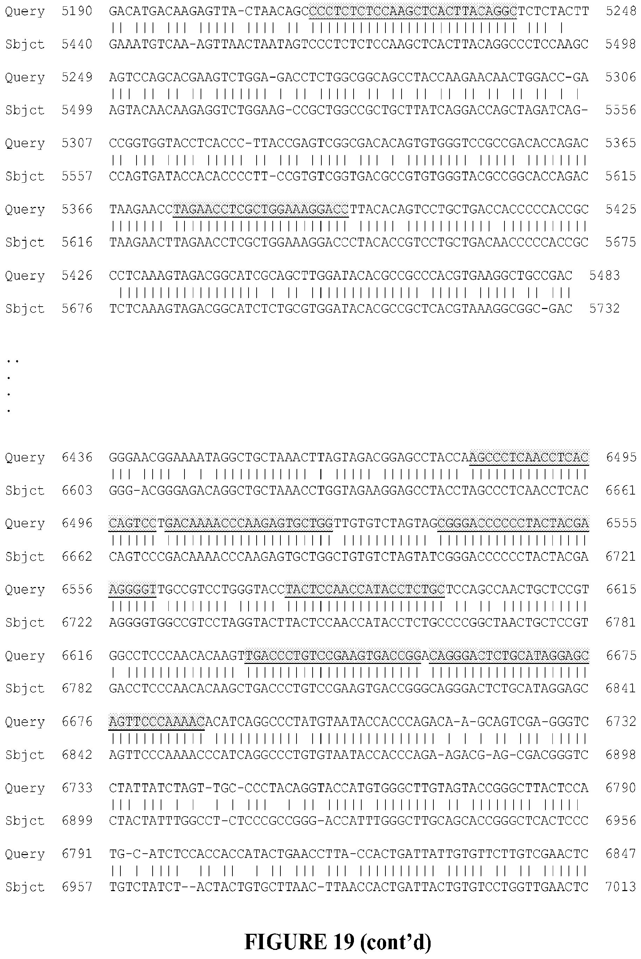

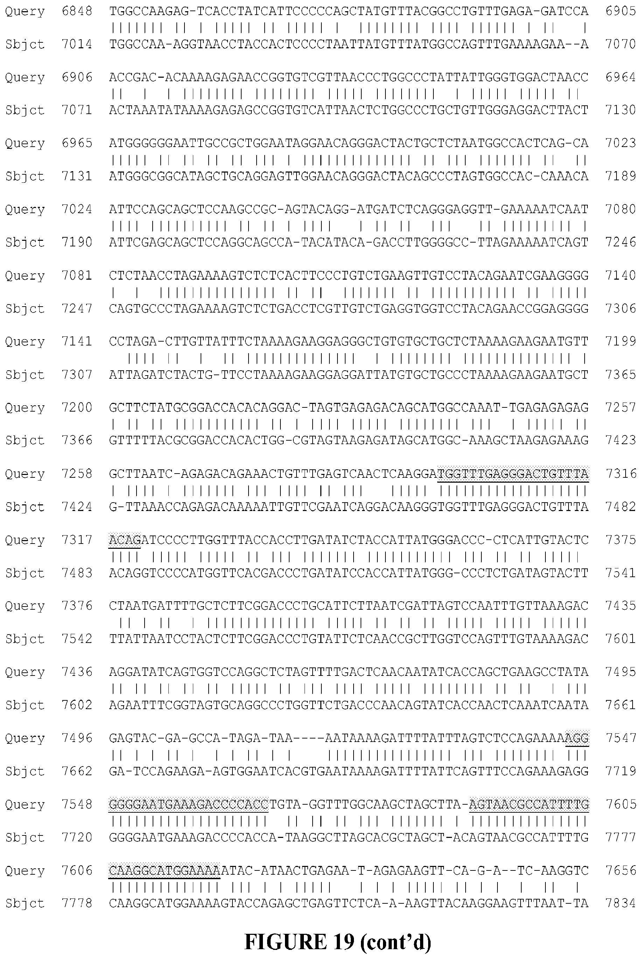

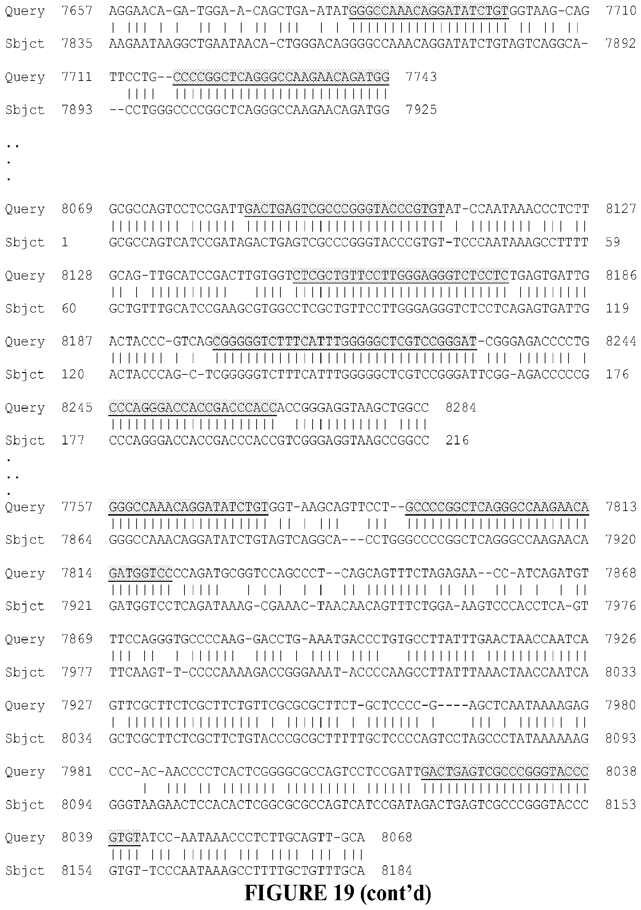

**Figure 19** shows sequences and line up from an MLV "Query" (SEQ ID NO:10) and XMRV "sbjct"(SEQ ID NO:11) useful in the methods and composition of the disclosure.

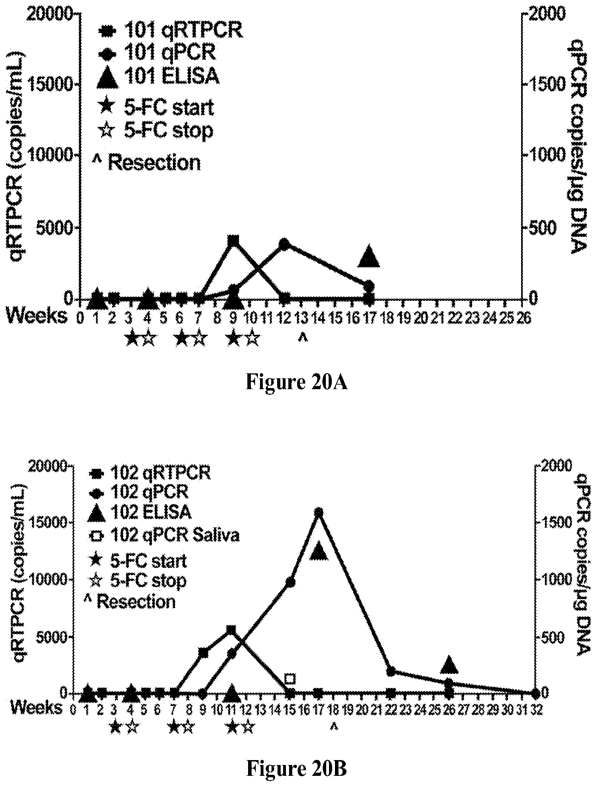

**Figure 20A-B** shows the course of appearance and disappearance of Toca 511 DNA and RNA sequences, plus the attendant ELISA measurements using the assay of this invention, in the blood of three high grade glioma patients dosed intracranially in their tumor at a dose of 2.6 x10³ TU/gram brain. A: patient 101; B patient 102; C patient 103. Pt101 and 102 show detectable nucleic acid sequences (sensitivity 5000 copies/ml for RNA in plasma, 250 copies/microgm for DNA from whole blood), whereas Pt 103 did not show detectable sequences. All patients showed ELISA positivity using compositions and methods of the disclosure (triangles).

### DETAILED DESCRIPTION

As used herein and in the appended claims, the singular forms "a," "and," and "the" include plural referents unless the context clearly dictates otherwise. Thus, for example, reference to "a cell" includes a plurality of such cells and reference to "the vector" includes reference to one or more vectors, and so forth.

Unless defined otherwise, all technical and scientific terms used herein have the same meaning as commonly understood to one of ordinary skill in the art to which this disclosure belongs.

Also, the use of "or" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of."

Retroviruses have been classified in various ways but the nomenclature has been standardized in the last decade (see ICTVdB - The Universal Virus Database, v 4 on the World Wide Web (www) at ncbi.nlm.nih.gov/ICTVdb/ICTVdB/ and the text book "Retroviruses," Eds. Coffin, Hughs and Varmus, Cold Spring Harbor Press 1997. A replication competent retrovirus is derived from the Retroviridae family of viruses and can comprise a member of the Orthoretrovirinae sub-family, or more typically comprises a retrovirus from the gammaretrovirus genus. In one aspect of the disclosure, the virus comprises a replication competent retrovirus which is a retroviral replicating vector (RRV), which, in turn, comprises an internal ribosomal entry site (IRES) 5' to a polynucleotide encoding a cytosine deaminase. In one aspect of the disclosure, the polynucleotide encoding a cytosine deaminase is 3' to an env polynucleotide of a retroviral vector.

The disclosure provides retroviruses of many kinds as immune reactivity reagents. The retrovirus used in the disclosure can be wild-type or modified retroviruses derived from various members of family. The classification of the retroviridae family has changed several times over the last ten to fifteen years. Currently the Retroviridae family consists of two sub-families: the Spumaretrovirinae-which has a single genus, the spumavirus (or foamy viruses) such as the human and simian foamy virus (HFV) and the Orthoretroviriniae sub-family which has 6 genus - betaretrovirus (e.g., MMTV), gammaretrovirus (e.g., MLV), alpharetrovirus (e.g., ALV) deltaretrovirus (e.g., BLV and HTLV-1) lentivirus(e.g., HIV 1) and epsilon retrovirus (e.g., wall eye dermal sarcoma virus). These classifications are made on the basis of common molecular features such as the relative reading frames for *gag, pol* and env, the processing of the polyproteins, the individual tRNAS used for priming reverse transcription, and the nature of the LTR structures. The original method of classification of retroviruses was into groups A, B, C and D on the basis of particle morphology, as seen under the electron microscope during viral maturation. A-type particles represent the immature particles of the B- and D-type viruses seen in the cytoplasm of infected cells. These particles are not infectious. B-type particles bud as mature virion from the plasma membrane by the enveloping of intracytoplasmic A-type particles. At the membrane they possess a toroidal core of 75 nm, from which long glycoprotein spikes project. After budding, B-type particles contain an eccentrically located, electron-dense core. The betaretrovirus, Mouse mammary tumor virus (MMTV) has a B-type morphology, but betaretroviruses can also have a D-type structure. D-type particles resemble B-type particles in that they show as ring-like structures in the infected cell cytoplasm, which bud from the cell surface, but the virion incorporate short surface glycoprotein spikes. The electron-dense cores are also eccentrically located within the particles. Mason Pfizer monkey virus (MPMV), also a betaretrovirus, is the prototype D-type virus. No intracytoplasmic particles can be observed in cells infected by C-type viruses. Instead, mature particles bud directly from the cell surface via a crescent 'C'-shaped condensation which then closes on itself and is enclosed by the plasma membrane. Envelope glycoprotein spikes may be visible, along with a uniformly electron-dense core. Budding may occur from the surface plasma membrane or directly into intracellular vacuoles. Alpharetroviruses, gammaretroviruses, deltaretroviruses and epsilonretroviruses all have the C-type structural appearance.

Retroviruses are defined by the way in which they replicate their genetic material. During replication the RNA is converted into DNA. Following infection of the cell a double-stranded molecule of DNA is generated from the two molecules of RNA which are carried in the viral particle by the molecular process known as reverse transcription. The DNA form becomes covalently integrated in the host cell genome as a provirus, from which viral RNAs are expressed with the aid of cellular and/or viral factors. The expressed viral RNAs are packaged into particles and released as infectious virion.

The retrovirus particle is composed of two identical RNA molecules. Each wild-type genome has a positive sense, single-stranded RNA molecule, which is capped at the 5' end and polyadenylated at the 3' tail. The diploid virus particle contains the two RNA strands complexed with gag proteins, viral enzymes (pol gene products) and host tRNA molecules within a 'core' structure of gag proteins. Surrounding and protecting this capsid is a lipid bilayer, derived from host cell membranes and containing viral envelope (env) proteins. The env proteins bind to a cellular receptor for the virus and the particle typically enters the host cell via receptor-mediated endocytosis and/or membrane fusion.

After the outer envelope is shed, the viral RNA is copied into DNA by reverse transcription. This is catalyzed by the reverse transcriptase enzyme encoded by the pol region and uses the host cell tRNA packaged into the virion as a primer for DNA synthesis. In this way the RNA genome is converted into the more complex DNA genome.

The double-stranded linear DNA produced by reverse transcription may, or may not, have to be circularized in the nucleus. The provirus now has two identical repeats at either end, known as the long terminal repeats (LTR). The termini of the two LTR sequences produces the site recognized by a pol product--the integrase protein--which catalyzes integration, such that the provirus is always joined to host DNA two base pairs (bp) from the ends of the LTRs. A duplication of cellular sequences is seen at the ends of both LTRs, reminiscent of the integration pattern of transposable genetic elements. Integration occurs at a large number of sites within the target cell genome, with biases that depend on the type of retrovirus and the type of cell. However, by modifying the integrase component (int) of the pol gene (see below)it is possible to control or partially direct the integration of a retroviral genome.

Transcription, RNA splicing and translation of the integrated viral DNA is mediated by host cell proteins. Variously spliced transcripts are generated. In the case of the human retroviruses HIV-1/2 and HTLV-I/II viral proteins are also used to regulate gene expression. The interplay between cellular and viral factors is a factor in the control of virus latency and the temporal sequence in which viral genes are expressed.

Retroviruses can be transmitted horizontally and vertically. Efficient infectious transmission of retroviruses requires the expression on the target cell of receptors which specifically recognize the viral envelope proteins, although viruses may use receptor-independent, nonspecific routes of entry at low efficiency. In addition, the target cell type must be able to support all stages of the replication cycle after virus has bound and penetrated. Vertical transmission occurs when the viral genome becomes integrated in the germ line of the host. The provirus will then be passed from generation to generation as though it were a cellular gene. Hence endogenous proviruses become established which frequently lie latent, but which can become activated when the host is exposed to appropriate agents.

As mentioned above, the integrated DNA intermediate is referred to as a provirus. As described below, a helper virus is not required for the production of the recombinant retrovirus of the disclosure, since the sequences for encapsidation are provided in the genome thus providing a replication competent retroviral vector for gene delivery or therapy.

The retroviral genome and the proviral DNA of the disclosure have at least three genes: the gag, the pol, and the env, these genes may be flanked by one or two long terminal (LTR) repeat, or in the provirus are flanked by two long terminal repeat (LTR) and sequences containing cis-acting sequences such as psi. The gag gene encodes the internal structural (matrix, capsid, and nucleocapsid) proteins; the pol gene encodes the RNA-directed DNA polymerase (reverse transcriptase), protease and integrase; and the env gene encodes viral envelope glycoproteins. The 5' and/or 3' LTRs serve to promote transcription and polyadenylation of the virion RNAs. The LTR contains all other cis-acting sequences necessary for viral replication. All of these components are present in the viral particles of the current disclosure plus a tRNA molecule and viral membrane derived from the host cell that produced the virus. The env gene product is embedded in the viral envelope membrane, along with some cell surface molecules from the producer cell. The envelope gene is quite heavily glycosylated in a characteristic pattern that is fairly standardized but will depend on the producer cell. For these reasons viral particles produced to detect antibodies in humans are usually best made in human cell lines, but it can be advantageous to make the virus in cells of another species such as dog (e.g. D17 or Cf2 cells) to rule out detection of such self proteins, for example in human subjectrs with cross-reactive auto-immunity. Lentiviruses have additional genes including vif, vpr, tat, rev, vpu, nef, and vpx (in HIV-1, HIV-2 and/or SIV), and some of these can be incorporated into the viral particles (e.g vif and nef).

Adjacent to the 5' LTR are sequences necessary for reverse transcription of the genome (the tRNA primer binding site) and for efficient encapsidation of viral RNA into particles (the Psi site). If the sequences necessary for encapsidation (or packaging of retroviral RNA into infectious virion) are missing from the viral genome, the result is a cis defect which prevents encapsidation of genomic viral RNA. The disclosure provides a recombinant retrovirus capable of infecting a non-dividing cell, a dividing cell, or a cell having a cell proliferative disorder. The recombinant replication competent retrovirus of the disclosure comprises a polynucleotide sequence encoding a viral GAG, a viral POL, a viral ENV, and optionally a heterologous polynucleotide that is expressed after the viral vector infects a target cell, encapsulated within a virion.

Depending upon the intended use of the retroviral vector of the disclosure any number of heterologous polynucleotide or nucleic acid sequences may be inserted into the retroviral vector. Some examples are given in International Application Publication No. WO2010/036986.

As mentioned elsewhere herein, XMRV itself is thought to be an artifact caused by contamination of samples by mouse DNA and recombination of laboratory Xenotropic isolates (T.Paprotka, et al. "Recombinant Origin of the Retrovirus XMRV" Science 333, 97 (2011); J.Cohen & M.Enserink "False Postive" Science 333: 1694-1701 (2011); G. Simmons et al. "Failure to Confirm XMRV/MLVs in the Blood of Patients with Chronic Fatigue Syndrome: A Multi-Laboratory Study," (www).sciencexpress.org / 10.1126/science.1213841). However, as demonstrated here (see, e.g., Figures 9 and 10, Examples 15 and 16, and Table 7), there are individuals with high naturally occurring anti-MLV titers, indicating that MLV infection of humans with MLV related viruses can and does occur. Therefore in this application, XMRV is used as an example of how such a virus can be detected and followed. As noted above one method of monitoring retroviral infections is to look at the antibodies to viral proteins in the blood/serum/plasma of individuals suspected of being infected or being tested for one reason or another (Blood donation, viral load progression, disease monitoring, therapeutic monitoring of retroviral therapy etc.) Usually the antigen used in such assays is manufactured in bacteria, baculovirus or mammalian cells. These antigens are then used in ELISA or other antibody binding assays, with the protein as the target and serum or plasma from subjects tested for reactivity with the specific antigens. It is desirable to be able to track the presence or absence of serum reactivity as this is normally well correlated with the increasing or decreasing presence of the virus in infected individuals. However, the use of peptides or proteins as assay targets may be of limited use in detection a heterogeneous set of related viruses such as represented by MLV related viruses (MLVRV), as it undesirably narrows the set of viruses that the assay will detect.

In addition, antigens synthesized alone, even in mammalian cells, will not incorporate the three dimensional structures of the virus produced in vivo, and can be inappropriately modified or lack appropriate modification. This can lead to false negatives. Recently specific peptide assays have been designed for XMRV (Qiu et al., Retrovirology,7:68, 2010) and are unlikely to detect all MLV-like variants. Also assays using cell lysates of retrovirally infected cells have also been designed but these use crude unpurified preparations and, unlike the present disclosure, positive samples were not detected (Switzer et al. Retrovirology, 7:57, 2010) probably due to interference and high background.

The life cycle of retroviruses involves a stage when the virus' genetic material is inserted into the genome of a host cell. This step is essential because the inserted viral nucleic acid, the provirus, is replicated through the host cell machinery. As part of this process the RNA genome of the retrovirus is replicated through a double-stranded DNA intermediate prior to insertion into the genome of the host cell. The initial conversion of the viral RNA molecule into a double-stranded DNA (dsDNA) molecule is performed by a reverse-transcriptase. The dsDNA is then integrated into the hose cell genome by an integrase to further be replicated by the host cellular machinery. The reverse transcriptase and the integrase required for the conversion of the RNA into dsDNA and for the integration into the host genome are carried within the viral particle during infection. The proviral DNA is finally transcribed using the host machinery into multiple RNA copies. These RNA molecules are then translated into viral peptides or proteins or integrated into viral particles which are released from the cell into the medium or extracellular milieu.

A retroviral RNA genome usually comprises 6 typical regions leading to the expression of multiple proteins. These region include the *gag*, *pol* and *env* gene sequences associated with a packaging signal, a psi (ψ) signal and flanked by 5' and/or 3' long terminal repeats (LTR) regions. The gag gene leads to the expression of the protein components of the nucleoprotein core of the virus, while the *pol* gene products are involved in the synthesis polynucleotides and recombination. The env gene codes for the envelope components of the retrovirus particle. 5' and 3' LTR regions include promoters and assist in the integration of the viral genome into the chromosomal DNA of the host cell. The psi signal refers to the retroviral packaging signal that controls the efficient packaging of the RNA into the viral particle. Because of their ability to form proviruses, retroviruses are also useful to modify the genome of a target or host cell and various modifications have been made to retroviruses for use in gene therapy. Gene therapy using retroviral vectors is generally performed by adding a heterologous polynucleotide to the viral genome which encodes or produces a polypeptide or transcript of interest, packaging the recombinant genome into a viral particle and infecting a target host cell. The target cell will then incorporate the exogenous gene as being a part of a provirus.

Previously defective retroviral vectors have been used as target antigens to look for immune response after administration of non replicative retroviral vectors (Martineau et al. Human Gene Ther. 8:1231-1241, 1997). The use of individual antigens as described above can be useful, but given the variation in types of MLV with which individuals may be infected, the single antigen target approach risks being overly specific, and not detecting a true positive. However, little is reported about the development of replication competent retroviral vector systems. The methods described here allow immune monitoring of replication competent viral vectors and their persistence.

The term "RCR" or "RRV" as used herein is intended to mean a replication-competent retrovirus (RCR) or replicating retrovirus (RRV). A replication-competent virus is a viral particle that has the capacity to replicate by itself in a host cell. Some RRV/RCR can carry a heterologous gene.

As used herein, the term "RCR plasmid vector" means a plasmid which includes all or part of a retroviral genome including 5' and 3' retroviral long-term repeat (LTR) sequences, a packaging signal (ψ), and may include one or more polynucleotides encoding a protein(s) or polypeptide(s) of interest, such as a therapeutic agent or a selectable marker. The term "therapeutic" is used in a generic sense and includes treating agents, prophylactic agents, and replacement agents.

The terms "vector", "vector construct" and "expression vector" mean the vehicle by which a DNA or RNA sequence (*e.g*., a foreign gene) can be introduced into a host cell, so as to transform the host and promote expression (*e.g*., transcription and translation) of the introduced sequence. Vectors typically comprise the DNA of a transmissible agent, into which foreign DNA encoding a protein is inserted by restriction enzyme technology. A common type of vector is a "plasmid", which generally is a self-contained molecule of double-stranded DNA that can readily accept additional (foreign) DNA and which can readily introduced into a suitable host cell. A large number of vectors, including plasmid and fungal vectors, have been described for replication and/or expression in a variety of eukaryotic and prokaryotic hosts. Non-limiting examples include pKK plasmids (Clonetech), pUC plasmids, pET plasmids (Novagen, Inc., Madison, Wis.), pRSET or pREP plasmids (Invitrogen, San Diego, Calif.), or pMAL plasmids (New England Biolabs, Beverly, Mass.), and many appropriate host cells, using methods disclosed or cited herein or otherwise known to those skilled in the relevant art. Recombinant cloning vectors will often include one or more replication systems for cloning or expression, one or more markers for selection in the host, e.g., antibiotic resistance, and one or more expression cassettes.

The terms "transfecting" or "transfection" as used herein are intended to mean the transfer of at least one exogenous nucleic acid into a cell. The nucleic acid may be RNA, DNA or a combination of both. The exogenous nucleic acid refers to nucleic that is not found as a result of host cell division or host cell multiplication.

The term "virus" as used herein is intended to mean the physical virus or retrovirus particle.

The term "cell line" as used herein refers to cultured cells that can be passed (divided) more than once. The disclosure relates to cell lines that can be passed more than 2 times, up to 200 times, or more and includes any integer therebetween.

The terms "express" and "expression" mean allowing or causing the information in a gene or DNA sequence to become manifest, for example producing a protein by activating the cellular functions involved in transcription and translation of a corresponding gene or DNA sequence. A DNA sequence is expressed in or by a cell to form an "expression product" such as a protein. The expression product itself, e.g. the resulting protein, may also be said to be "expressed" by the cell. A polynucleotide or polypeptide is expressed recombinantly, for example, when it is expressed or produced in a foreign host cell under the control of a foreign or native promoter, or in a native host cell under the control of a foreign promoter.

The terms "stable expression" and "stably expressing" as used herein are intended to mean that the genetic material that is being stably expressed and/or is integrated permanently and stably in the genome of the host cell, and thus has the same expression potential over time as the native genetic material of the host cell.

The terms "transient expression" and "transiently expressing" as used herein are intended to mean that the genetic material temporal expression period and/or is not integrated permanently and stably in the genome of the host cell, and thus does not have the same expression potential over time as the native genetic material of the host cell.

As used herein, the term "heterologous" nucleic acid sequence or transgene refers to (i) a sequence that does not normally exist in a wild-type retrovirus, (ii) a sequence that originates from a foreign species, or (iii) if from the same species, it may be substantially modified from its original form. Alternatively, an unchanged nucleic acid sequence that is not normally expressed in a cell is a heterologous nucleic acid sequence.

The term "therapeutic" as used herein refers to an action that prevents, reverses, or slows the natural course of a disease or disorder, or its symptoms. A therapeutic action can be preventive, curative or merely palliative, and does not mean that the affected human or animal patient will not die from the disease.

The term MLV related virus (MLVRV) refers to a retrovirus that infects either by natural transmission or by intentional administration a species other than rodents and has at least 60 % homology to at least one of the LTR, Gag, pol, integrase, envelope or non-coding regions of canonical MLV's (NCBI ((http://www).ncbi.nlm.nih.gov/) listed MLV's such as ecotropic MLV NC_001501.1, amphotropic MLV (AF010170.1), XMRV (FN692043.1), xenotropic MLV,or polytropic MLV).

An antigenic viral particle or antibody-binding viral particle is produced by a producer cell of the disclosure.In one aspect of the disclosure, the producer cell lines of the disclosure are capable of growth as an adherent culture, in suspension or in a serum-free medium. The producer cell lines can also be grown both in serum-free medium and suspension simultaneously, or as an adherent line in serum free media. Although serum-free medium and the capacity to grow in suspension are the typical conditions, cells of the disclosure (e.g., 293, 293T, HT1080 cells, PC3 cells, LNCaP cells, SupT1 cells, U87 cells, D17 cells or CF2 cells) can be cultured in an adherent manner with regular serum-containing medium to achieve particular purposes. Such purposes can be, for example, to facilitate transfection of cells or to select cell clones.

The type of producer cells used to generate the retrovirus (described more fully below) is useful for the production of replication competent viral particles for use as targets to detect antibody responses to the vector particle or components thereof. Such methods are useful for monitoring infection, tissue sample and gene delivery and gene therapy using retroviral particles.

The disclosure provides a method of generating a producer cell line comprising transforming or transfecting a first mammalian cell type with an RCR plasmid vector of the disclosure, culturing the first cell type to produce retroviral particles, obtaining a cell free media from the first cell type producing the retroviral particles, wherein the cell free media comprises retroviral particles, contacting a second mammalian cell type with the media to infect the second cell type and culturing the second cell type to produce a producer cell line that produces a replication competent retroviral vector for use in transforming mammalian cells. The first cell type can be almost any mammalian cell type that is capable of producing virus after transfection and may include HeLa, COS, Chinese Hamster Ovary (CHO), and HT1080 cells, and the transfection can be with calcium phosphate or other agents such as lipid formulations known to those skilled in the art as useful for transfection.

In one aspect of the disclosure, the first cell type is a human embryonic kidney cell. In another aspect of the disclosure, the human embryonic kidney cell is a 293 cell (also often referred to as HEK 293 cells, 293 cells, or less precisely as HEK cells), which are a cell line originally derived from human embryonic kidney cells grown in tissue culture. HEK 293 cells were generated by transformation of cultures of normal human embryonic kidney cells with sheared adenovirus 5 DNA. HEK 293 cells are easy to grow and transfect very readily and have been widely-used in cell biology research for many years. They are also used by the biotechnology industry to produce therapeutic proteins and viruses for gene therapy.

In another aspect of the disclosure, the first cell type is a mammalian cell transformed with an SV40 Large T-antigen. In a particular aspect of the disclosure, 293T HEK cells are used. An important variant of this cell line is the 293T cell line which contains the SV40 Large T-antigen allowing for episomal replication of transfected plasmids containing the SV40 origin of replication. This allows for amplification of transfected plasmids and extended temporal expression of the desired gene products.

The term "human 293 cell" as used herein includes the HEK 293T cell line, the human 293 cell line (ATCC No. CRL 1573) (Graham, et al., J. Gen. Virol., Vol. 36, pgs. 59-72 (1977)), or a cell line formed by transfecting 293 cells with one or more expression vehicles (e.g., plasmid vectors) including polynucleotides encoding various *gag, pol,* and env proteins. The envelope may be an amphotropic envelope, an ecotropic envelope, a xenotropic envelope,an XMRV envelope, a polytropic envelope, a GALV envelope, an RD114 envelope, an FeLV envelope or other retroviral envelope. Such cells also may include other polynucleotides such as, for example, polynucleotides encoding selectable markers.

The first cell type (*e.g*., HEK 293T cells) may be transformed with an RCR plasmid vector of the disclosure in any number of means including calcium phosphate and the like. Typical culture conditions for mammalian cells, particularly human 293 cells are known in the art.

Once transformed the first cell type is cultured under conditions for production of viral particles. Such conditions typically include refeeding cells in appropriate media, CO₂, and humidity. The culture conditions may also include the addition of antibiotics, anti-fungals, growth factors and the like. Typically the refed medium is harvested after 24, 48, 72, or 96 hours, and such a procedure is known as a transient expression transfection procedure.

The media from the cultured cells above may be used directly in further culturing. Alternatively, the viral particles in the media of cultured cells may be isolated using any number of techniques known in the art including centrifugation, size exclusion techniques, anion exchange chromatography and the like.

Where the media is used directly, the media can be added to media used in the culture of the second cell type. Where the viral particles are first substantially purified, the particles may be washed or resuspended in an appropriate buffer or media or at particular concentration for infectivity before addition to the second cell type, leading to the generation of a stable expression producer cell line.

In one aspect of the disclosure, the second cell type is a human fibroscarcoma cell line. In a specific aspect of the disclosure, the cell line is an HT1080 cell line or a derivative thereof. HT1080 human fibrosarcoma cell line (ATCC, Catalog# CCL-121) can be obtained directly from the American Type Culture Collection (P.O. Box 1549, Manassas, VA). The method includes infecting the HT1080 cells with an RCR of the disclosure to provide a stably transfected host cell. The stably transfected host cell may be cultured to produce viral particles for use in gene therapy or gene delivery or may be "banked" for later use, and may be a pool of transfected cells, or a cloned cell line. The banked cells may be frozen and stored using techniques known in the art.

In a further aspect of the disclosure the cells infected with the virus generated by transient transfection or by other means, can be any mammalian cell line known to be infectable with the corresponding virus. Infectability by a virus can be tested by methods described in Example 1 and shown in Figures 1 and 2. Virus growth can be monitored by PCR of cellular DNA, RT-PCR for viral genomes in the supernatant or Reverse transcriptase assays of reverse transcriptase activities in the viral supernatant such as the PERT assay (e.g., L. Shastry et al., Hum Gen Ther., 16:1227-1236, 2005). Specific examples of cell lines that can be used are HT1080 cells, PC3 cells, LNCaP cells, SupT1 cells, U87 cells, Sup T1 cells, D17 cells, CF2 cells, Hela cells, 293, CV1 and CHO cells. Cell lines derived form these cell lines can also be used. In some aspects of the disclosure cell lines that were isolated and already made an MLV related retrovirus (e.g 22Rvl ATCC CRL-2505) can also be used as virus producer lines. In other aspects of the disclosure cells such as the types listed above can be infected by cocultivation with PBMC from infected individuals. Clones from cells infected in any of these ways can be isolated and cell clones with desirable properties (e.g., that make high titers of virus) can be further purified and isolated.

In one aspect of the disclosure, the cells are cultured in the presence of fetal calf serum or other non-defined additives. In another aspect of the disclosure, the cells will be cultured in serum-free media. In one aspect of the disclosure, the cells are culture in an animal free media or a defined media. RCR viral particles (e.g., MLV related virus) can be substantially purified from the media of the producer cells. The purified virus can be washed, diluted and resuspended in an appropriate pharmaceutically acceptable carrier. Alternatively, the purified vector may be stored either by freezing of lypholization.

In one aspect of the disclosure, the virus will be supplied as an aqueous sterile solution containing the following formulation excipients (in mg/mL): sucrose 10.0, mannitol 10.0, and NaCl 5.3. The solution may also contain Human Serum Albumin (HSA, Baxter) 1.0 and ascorbic acid 0.10.

As described further herein, any number of retroviruses of the disclosure may be used with the producer cell lines and process described herein.

In specific examples provided herein, Toca 511 an amphotropic MLV that carries a heterologous polynucleotide encoding a polypeptide with cytosine deaminase (see, e.g., WO2010036986 A2) is used to demonstrate the methods and compositions of the disclosure. In another specific example, amphotropic MLV (ATCC VR-1450) is used to demonstrate the methods and compositions of the disclosure. As described herein, Toca 511 refers to a replication competent retroviral vector encoded in a plasmid designated as pAC3-yCD2 and Ampho MLV refers to a retrovirus encoded in a plasmid designated pAMS, ATCC # 45167. The virus is comprised of a replication-competent retrovirus derived from a murine leukemia virus (MLV) encoding all retroviral components (gag, pol and env) required for viral replication, with the original ecotropic envelope replaced with the amphotropic envelope from the 4070A virus. Alternatively, other virus encoding plasmids or viral preparations can be used, e.g., XMRV, or MV related virus.

The methods and compositions of the disclosure are applicable to other viruses, viral vectors and recombinant retroviral vectors. The disclosure describes various modification and recombinant vectors that can be produced by the cell lines and methods of the disclosure.

The retrovirus and methods of the disclosure provide a replication competent retrovirus that does not require helper virus or additional nucleic acid sequence or proteins in order to propagate and produce virion. For example, the nucleic acid sequences of the retrovirus of the disclosure encode, for example, a group specific antigen and reverse transcriptase, (and integrase and protease-enzymes necessary for maturation and reverse transcription), respectively, as discussed above. The viral gag and pol can be derived from a lentivirus, such as HIV or a gammaretrovirus such as MoMLV. In addition, the nucleic acid genome of the retrovirus of the disclosure includes a sequence encoding a viral envelope (ENV) protein. The env gene can be derived from any retroviruses. The env may be an amphotropic envelope protein which allows transduction of cells of human and other species, murine leukemia virus (MLV), Moloney murine leukemia virus (MoMLV), amphotropic MoMLV, Xenotropic MLV related retrovirus (XMRV), MLV related virus found in humans, xenotropic MLV, Polytropic MLV, amphotropic MLV, Gibbon ape leukemia virus (GALV), murine mammary tumor virus (MuMTV), Rous Sarcoma Virus (RSV), Gibbon ape leukemia virus (GALV), baboon endogenous virus (BEV), feline virus RD114, other gamma retrovirus, or hybrids of the above.

In addition, the disclosure provides polynucleotide sequence encoding a recombinant retroviral vector of the disclosure. The polynucleotide sequence can be incorporated into various viral particles. For example, various viral vectors which can be utilized for gene delivery include adenovirus, herpes virus, vaccinia, and baculovirus. Retroviruses can be prepared using such vectors (replicative or non-replicative) to deliver the retroviral genes to cells to make producer cells for the retrovirus. The retrovirus can then be harvested and purified according to the methods described in this specification. In yet another aspect, the disclosure provides plasmids comprising a recombinant retroviral derived construct. The plasmid can be directly introduced into a target cell or a cell culture such as HT1080 or other tissue culture cells. The resulting cells release the retroviral vector into the culture medium.

The disclosure provides a polynucleotide construct comprising from 5' to 3': a promoter or regulatory region useful for initiating transcription; a psi packaging signal; a gag encoding nucleic acid sequence, a *pol* encoding nucleic acid sequence; an env encoding nucleic acid sequence. For example, the promoter can comprise a CMV promoter having a sequence as set forth in SEQ ID NO: 7, 8 or 9 from nucleotide 1 to about nucleotide 582 and may include modification to one or more (*e.g*., 2-5, 5-10, 10-20, 20-30, 30-50, 50-100 or more nucleic acid bases) so long as the modified promoter is capable of directing and initiating transcription. In one aspect of the disclosure, the promoter or regulatory region comprises a CMV-R-U5 domain polynucleotide. The CMV-R-U5 domain comprises the immediately early promoter from human cytomegalovirus to the MLV R-U5 region. In one aspect of the disclosure, the CMV-R-U5 domain polynucleotide comprises a sequence as set forth in SEQ ID NO: 7, 8 or 9 from about nucleotide 1 to about nucleotide 1202 or sequences that are at least 95% identical to a sequence as set forth in SEQ ID NO: 7, 8 or 9 wherein the polynucleotide promotes transcription of a nucleic acid molecule operably linked thereto. The gag domain of the polynucleotide may be derived from any number of retroviruses, but will typically be derived from murine leukemia virus (MLV), Moloney murine leukemia virus (MoMLV), amphotropic MoMLV, Xenotropic MLV related retrovirus (XMRV), MLV related virus found in humans, xenotropic MLV, Polytropic MLV, amphotropic MLV, Gibbon ape leukemia virus (GALV), murine mammary tumor virus (MuMTV), Rous Sarcoma Virus (RSV), Gibbon ape leukemia virus (GALV), baboon endogenous virus (BEV), feline virus RD114, other gamma retrovirus, or hybrids of the above.

In one aspect of the disclosure the gag domain comprises a sequence from about nucleotide number 1203 to about nucleotide 2819 or a sequence having at least 60%, 70% 80%, 90%, 95%, 98%, 99% or 99.8% (rounded to the nearest 10^{th}) identity thereto. The pol domain of the polynucleotide may be derived from any number of retroviruses, but will typically be derived from murine leukemia virus (MLV), Moloney murine leukemia virus (MoMLV), amphotropic MoMLV, Xenotropic MLV related retrovirus (XMRV), MLV related virus found in humans, xenotropic MLV, Polytropic MLV, amphotropic MLV, Gibbon ape leukemia virus (GALV), murine mammary tumor virus (MuMTV), Rous Sarcoma Virus (RSV), Gibbon ape leukemia virus (GALV), baboon endogenous virus (BEV), feline virus RD114, other gamma retrovirus, or hybrids of the above.

In one aspect of the disclosure the *pol* domain comprises a sequence from about nucleotide number 2820 to about nucleotide 6358 or a sequence having at least 60%, 70% 80%, 90%, 95%, 98%, 99% or 99.9% (roundest to the nearest 10^{th}) identity thereto. The env domain of the polynucleotide may be derived from any number of retroviruses, but will typically be derived from murine leukemia virus (MLV), Moloney murine leukemia virus (MoMLV), amphotropic MoMLV, Xenotropic MLV related retrovirus (XMRV), MLV related viruses found in humans, xenotropic MLV, Polytropic MLV, amphotropic MLV, Gibbon ape leukemia virus (GALV), murine mammary tumor virus (MuMTV), Rous Sarcoma Virus (RSV), Gibbon ape leukemia virus (GALV), baboon endogenous virus (BEV), feline virus RD114, other gamma retrovirus, or hybrids of the above.

In one aspect of the disclosure, the env domain comprises a sequence from about nucleotide number 6359 to about nucleotide 8323 or a sequence having at least 60%, 70% 80%, 90%, 95%, 98%, 99% or 99.8% (roundest to the nearest 10^{th}) identity thereto. The 3' LTR can be derived from any number of retroviruses, typically murine leukemia virus (MLV), Moloney murine leukemia virus (MoMLV), amphotropic MoMLV, Xenotropic MLV related retrovirus (XMRV), MLV related virus found in humans, xenotropic MLV, Polytropic MLV, amphotropic MLV, Gibbon ape leukemia virus (GALV), murine mammary tumor virus
(MuMTV), Rous Sarcoma Virus (RSV), Gibbon ape leukemia virus (GALV), baboon endogenous virus (BEV), feline virus RD114, other gamma retrovirus, or hybrids of the above.

In one aspect of the disclosure, the 3' LTR comprises a U3-R-U5 domain. In yet another aspect of the disclosure, the LTR comprises a sequence as set forth in SEQ ID NO:7 or 9 from about nucleotide 9405 to about 9998 or a sequence that is at least 60%, 70% 80%, 90%, 95%, 98% or 99.5% (rounded to the nearest 10^{th}) identical thereto.

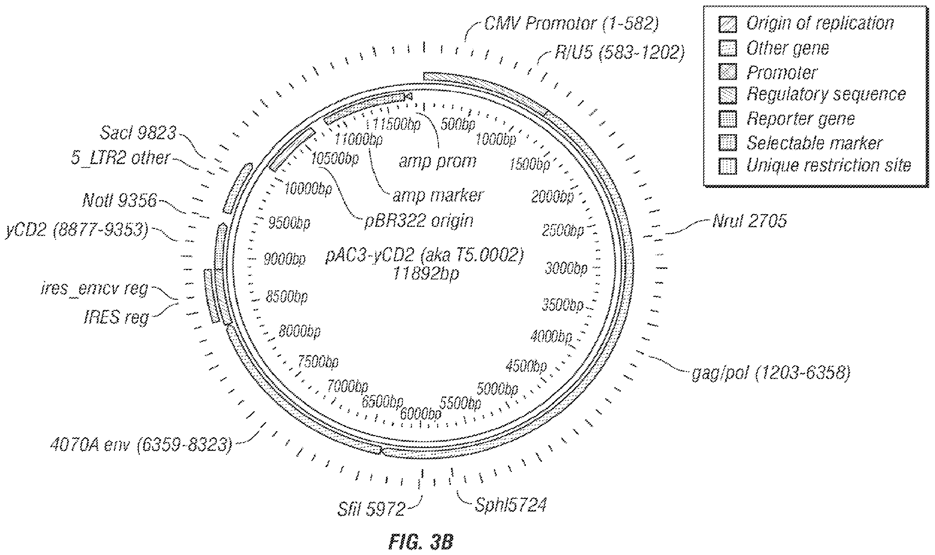

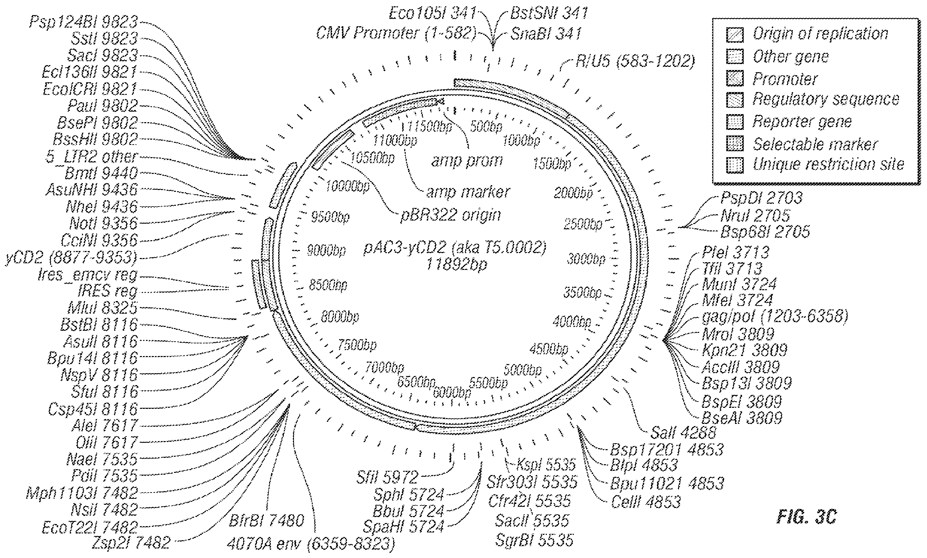

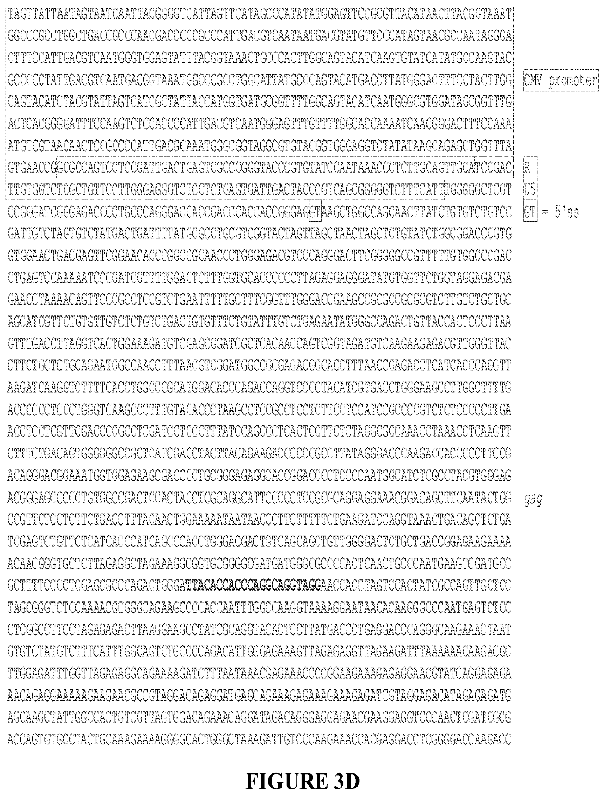

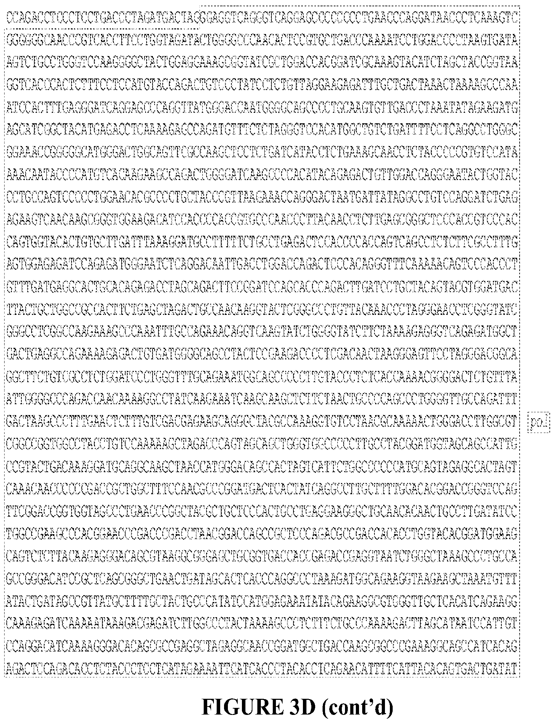

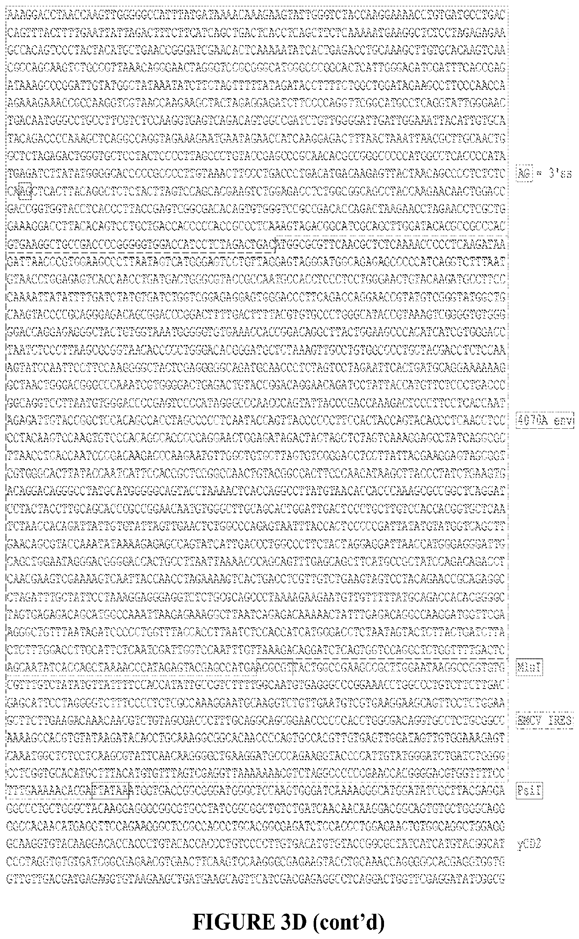

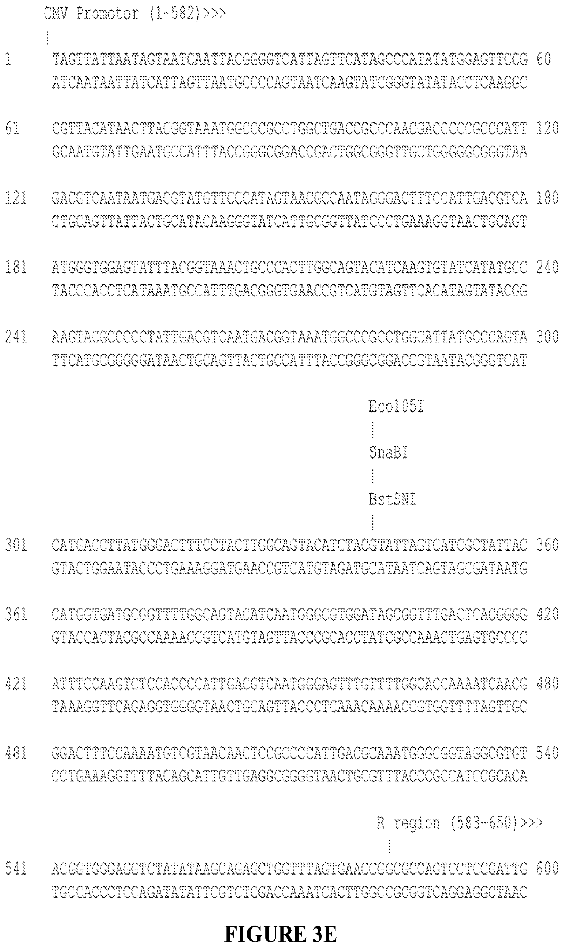

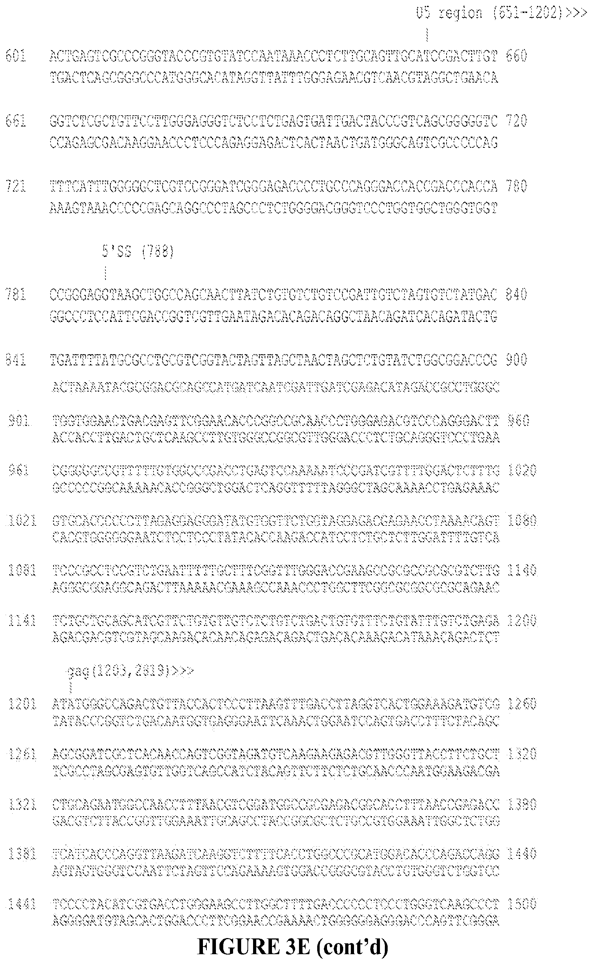

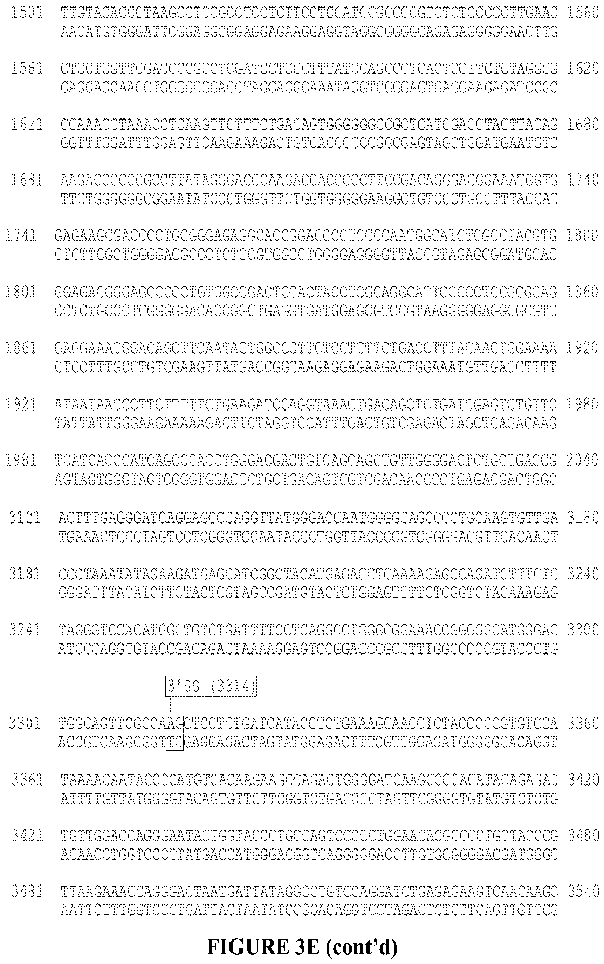

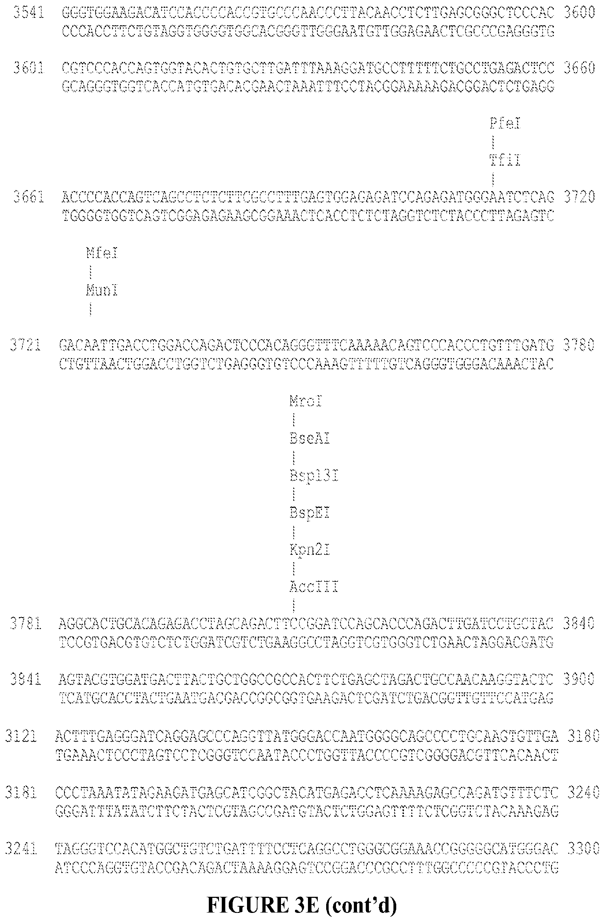

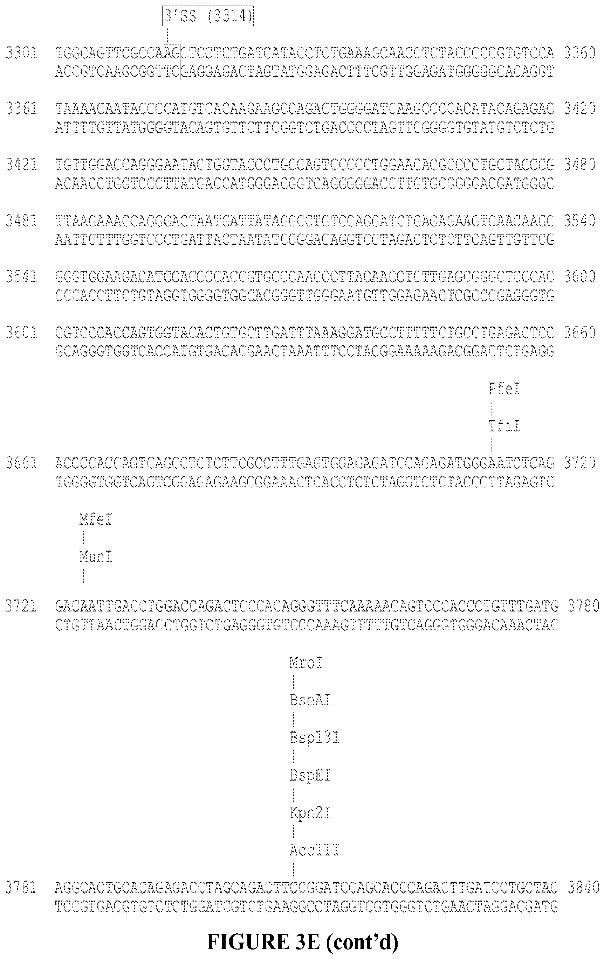

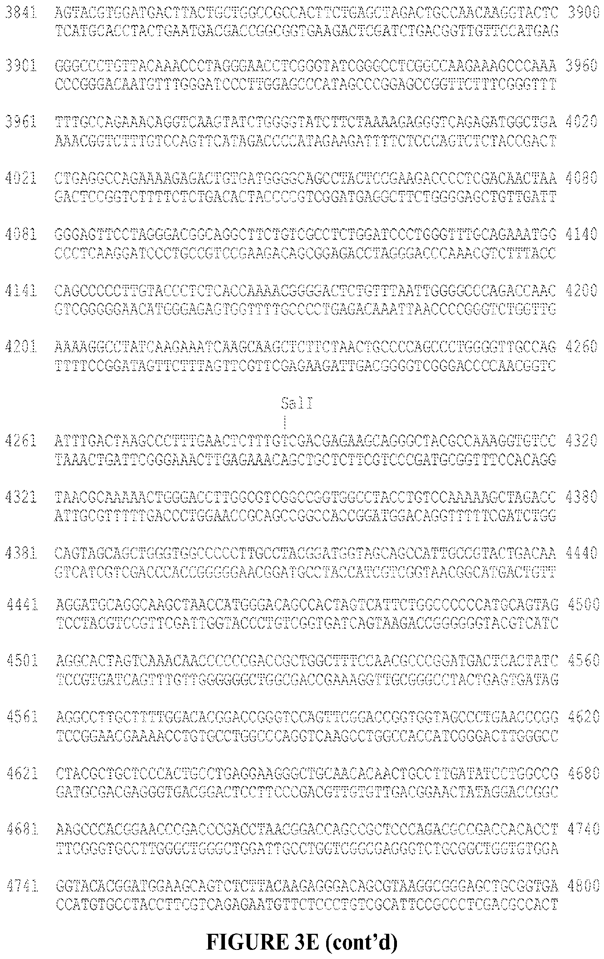

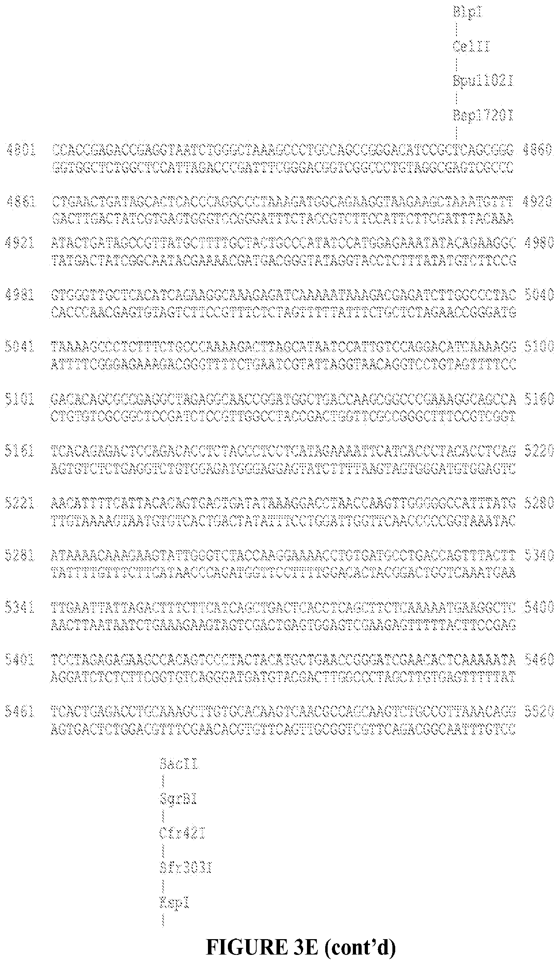

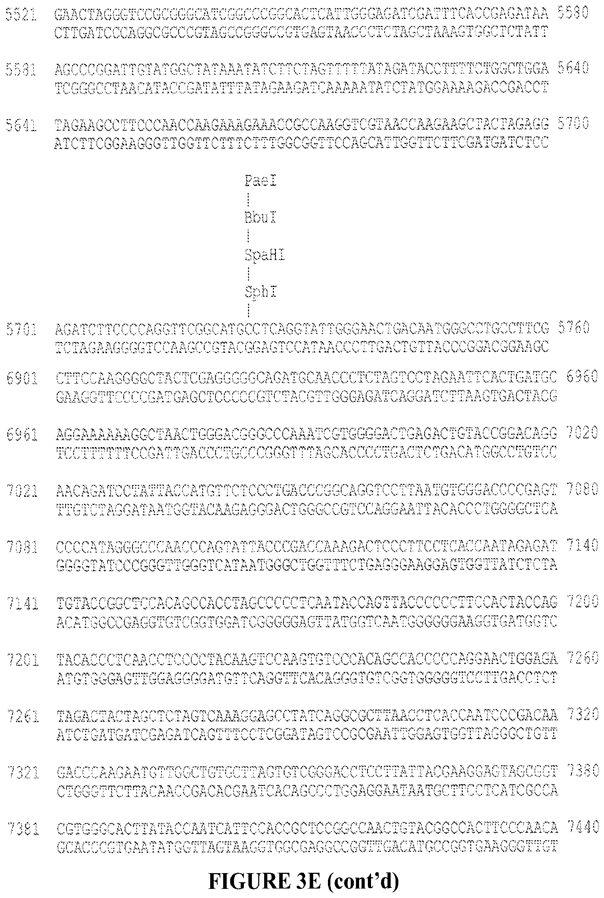

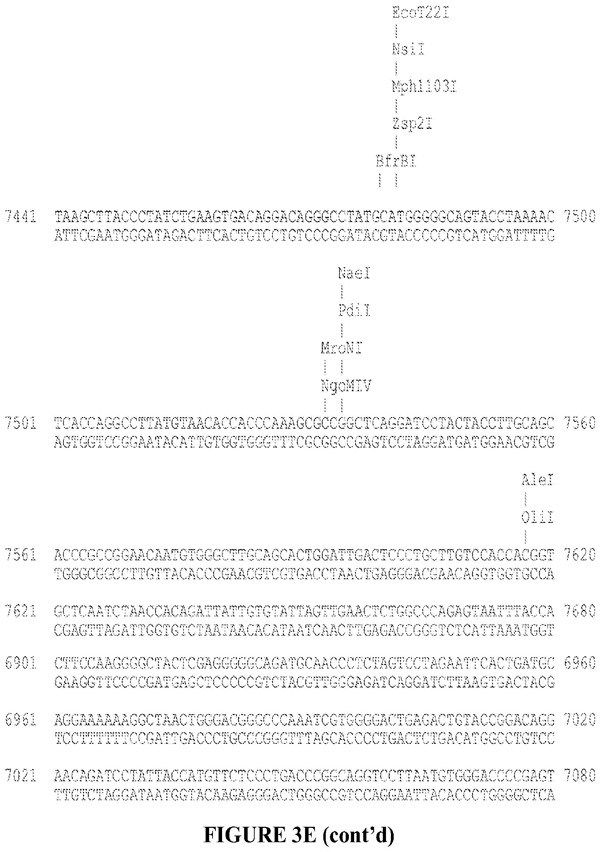

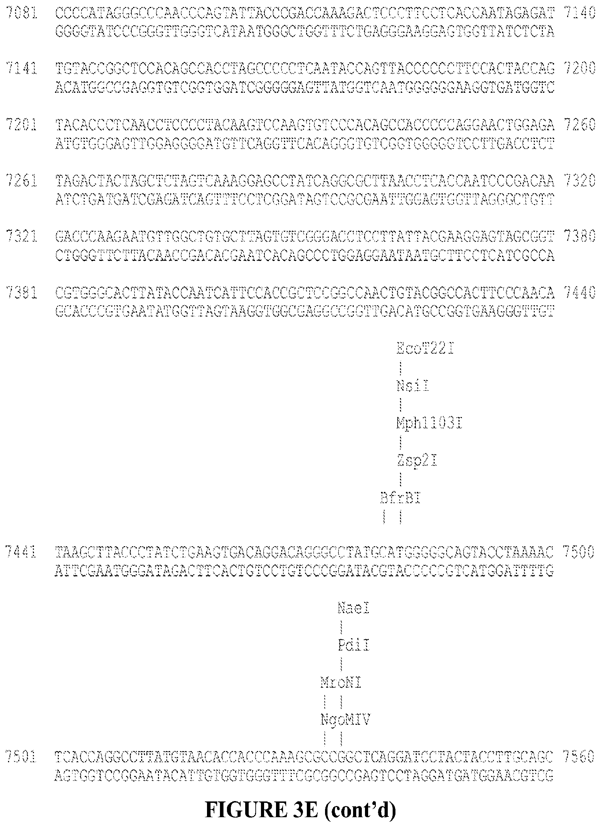

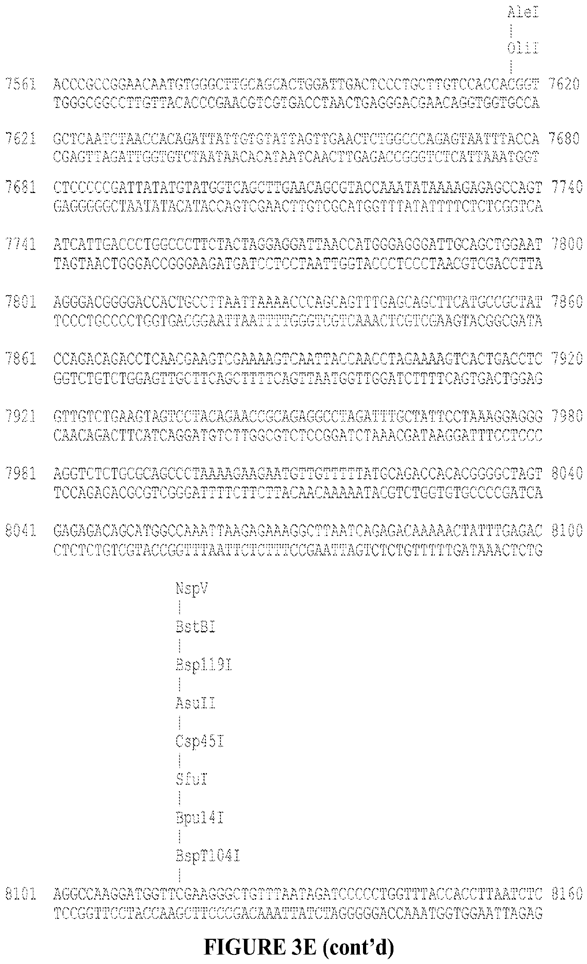

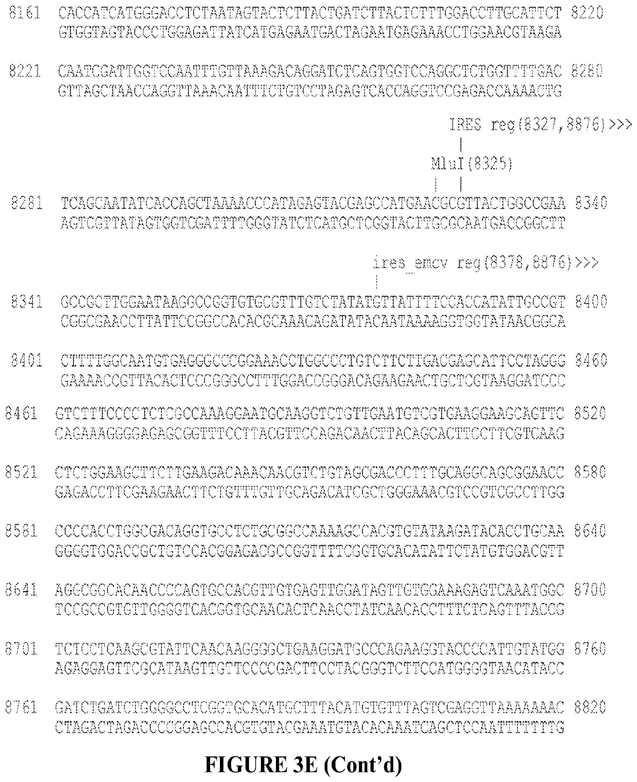

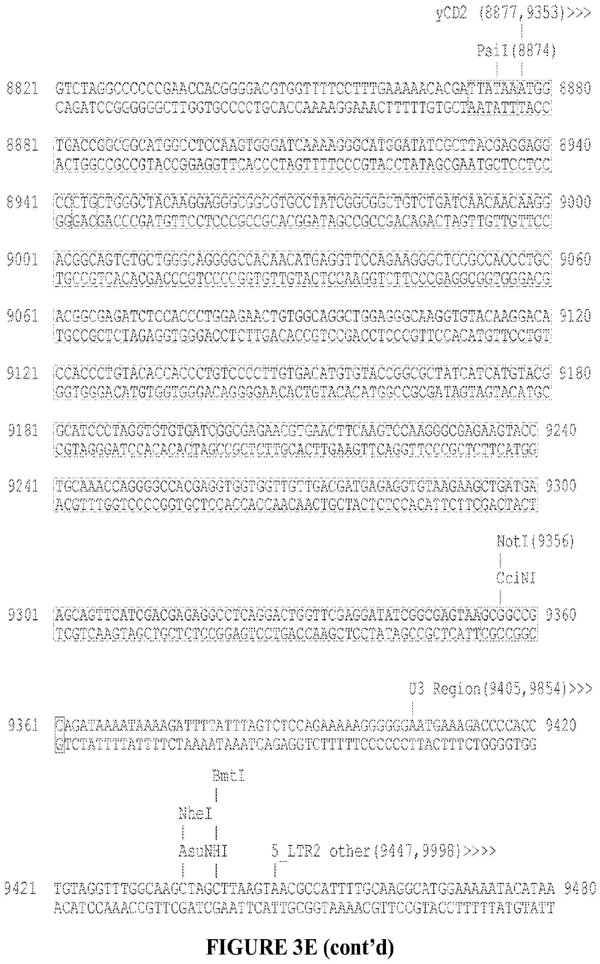

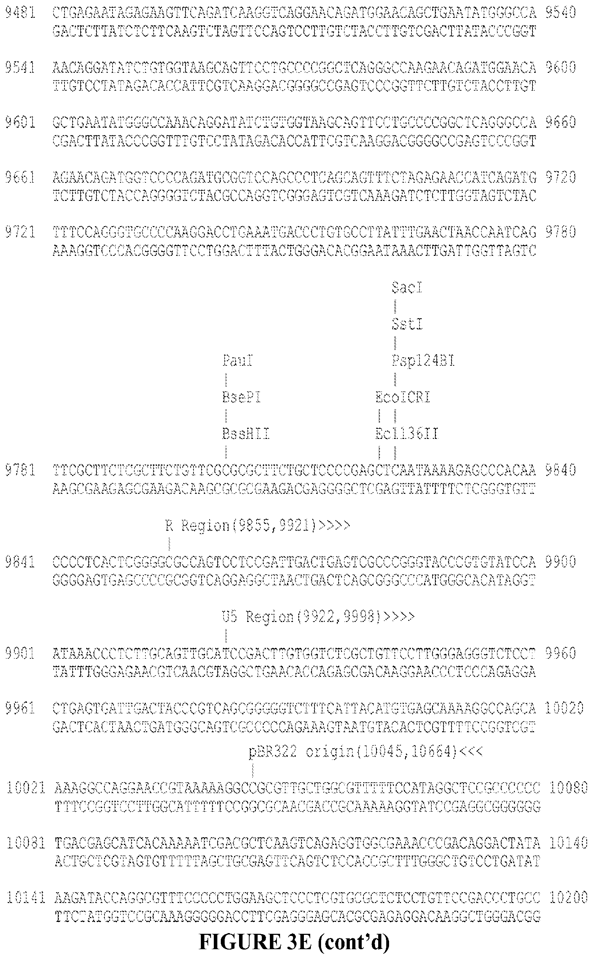

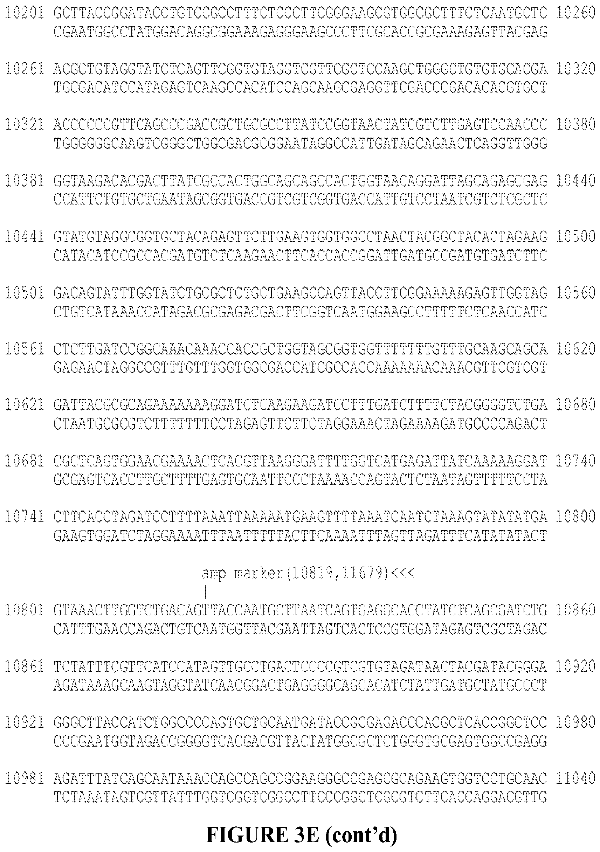

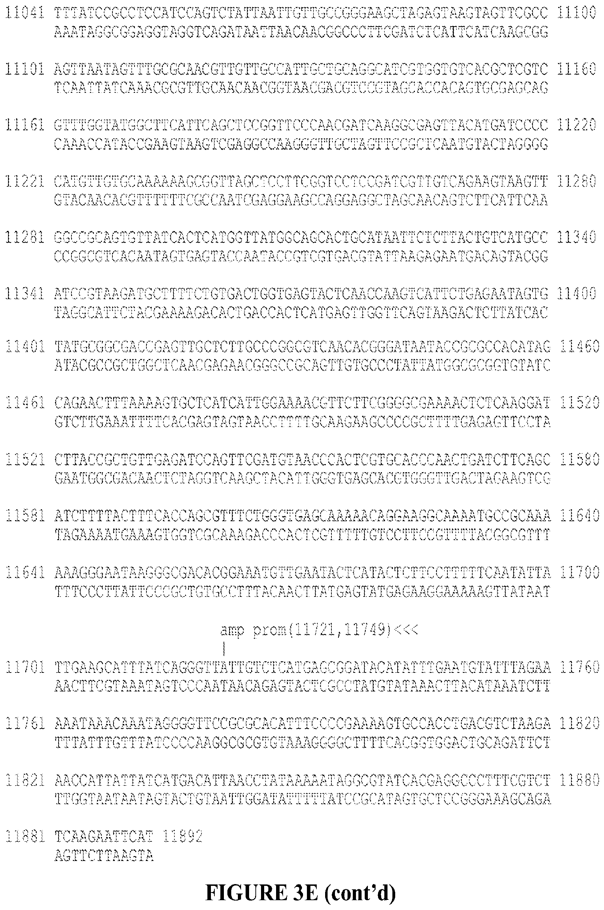

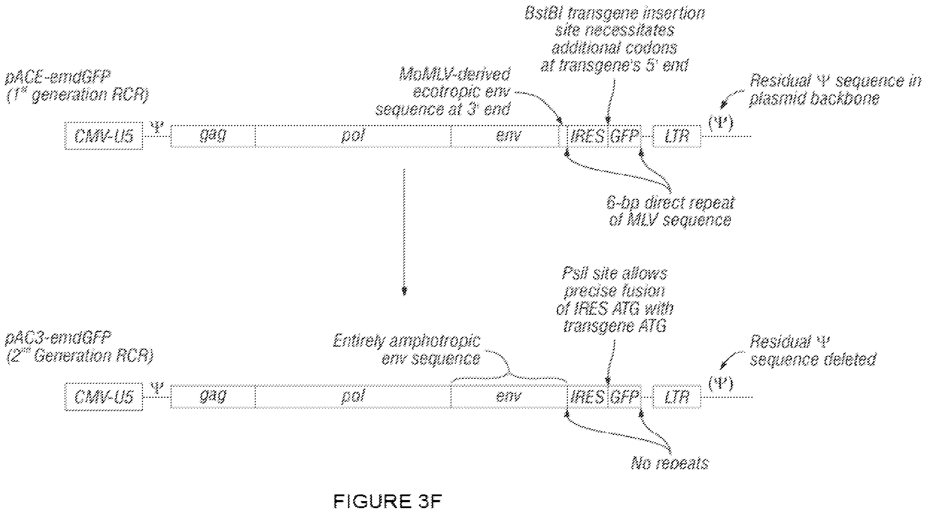

The disclosure also provides a recombinant retroviral vector genome comprising from 5' to 3' a CMV-R-U5, fusion of the immediate early promoter from human cytomegalovirus to the MLV R-U5 region; a PBS, primer binding site for reverse transcriptase; a 5' splice site; a ψ packaging signal; a gag, ORF for MLV group specific antigen; a pol, ORF for MLV polymerase polyprotein; a 3' splice site; a 4070A env, ORF for envelope protein of MLV strain 4070A; a PPT, polypurine tract; and a U3-R-U5, MLV long terminal repeat. This structure is further depicted in Figure 3.

As described above, the disclosure provides a host cells (e.g., 293, 293T, HT1080 cells, PC3 cells, LNCaP cells, SupT1 cells, U87 cells, D17 cells, CF2 cells, HeLa, COS cell, Chinese Hamster Ovary (CHO)) that are transduced (transformed or transfected) with a virus or viral vector provided herein. The vector may be, for example, a plasmid (e.g., as used with 293T cells), a viral particle (as used with HT1080 cells), a phage, etc. The host cells can be cultured in conventional nutrient media modified as appropriate for activating promoters, selecting transformants, or amplifying a coding polynucleotide. Culture conditions, such as temperature, pH and the like, are those previously used with the host cell selected for expression, and will be apparent to those skilled in the art and in the references cited herein, including, *e.g*., Sambrook, Ausubel and Berger, as well as e.g., Freshney (1994) Culture of Animal Cells: A Manual of Basic Technique, 3rd ed. (Wiley-Liss, New York) and the references cited therein.

In one aspect of the disclosure, a producer cell produces replication competent retroviral vectors having increased stability relative to retroviral vectors made by conventional transient transfection techniques. Such increased stability during production, infection and replication is important for the treatment of cell proliferative disorders. The combination of transduction efficiency, transgene stability and target selectivity is provided by the replication competent retrovirus. The compositions and methods provide insert stability and maintains transcription activity of the transgene and the translational viability of the encoded polypeptide.

Viral preparations of the current disclosure can be purified in several ways by centrifugation, ultrafitration, coprecipitation with other agents such as Polyethylene glycol (PEG) but optimally are highly purified by harvesting supernatants, filtering, treating with benzonase, and chromotography. Chromotograohy can be over various types of columns such sulphonated cellulose, OH-apatite or others, but is preferably on anion exchange columns (AEX) followed by size exclusion chromatography (SEC). These procedures allow concentration of the virus, followed by transition into the buffer in the SECF column. Preparations can then be ultrafiltered to sterilize and clarify the preparations before aliquotting for storage and use.

These viral preparations can be used to detect antibodies to viruses with some common epitopes, but viruses that are not necessarily identical to those used for detection. This is because of the related sequences and conformational epitopes in retroviruses in general (e.g., GB Mortuza et al. Nature 431:481-485 2004) and MLV related retroviruses in particular. The methods of the disclosure can use various protein and viral detection methods. In one embodiment, the methods comprise ELISA of whole virus bound on a plate, competitive binding assays, Western blots, and other methods known to those skilled in the art. These can be used on specialized commercial machines such as the Artitect System (Abbott) or in 96 well or 384 well microtiter formats for large sample number screening.

As used herein, a "biological sample" refers to a sample of tissue or fluid isolated from a subject, that commonly includes antibodies produced by the subject. Typical samples that include such antibodies are known in the art and include, but are not limited to, blood, plasma, serum, fecal matter, urine, bone marrow, bile, spinal fluid, lymph fluid, samples of the skin, secretions of the skin, respiratory, intestinal, and genitourinary tracts, tears, saliva, milk, blood cells, organs, biopsies and also samples of in vitro cell culture constituents including but not limited to conditioned media resulting from the growth of cells and tissues in culture medium, e.g., recombinant cells, and cell components. The fluids screened can be blood, plasma, serum, urine, saliva, bucchal swab, nasal swab, fine needle aspirate, semen, vaginal secretion, milk or other bodily fluid or extract, including feces.

A common solid support can be used in the assays and compositions of the disclosure. "Common solid support" intends a single solid matrix to which the retroviral particle used in the subject immunoassays are bound covalently or by noncovalent means such as hydrophobic adsorption.

"Immunologically reactive" means that an antigen will react specifically with anti-retroviral (e.g., anti-MLV) antibodies present in a biological sample from a subject or sample being tested.

"Immune complex" intends the combination formed when an antibody binds to an epitope on an antigen. Antibodies detected can be IgM, IgG, IgA, IgE or IgD, depending on the type of secondary antibody or other secondary binding reagent used in the assay. Detection methods for the secondary reagent can be horseradish peroxidase, alkaline phosphatase or any secondary detection system known to those skilled in the art.

As used herein, the terms "label" and "detectable label" refer to a molecule capable of detection, including, but not limited to, radioactive isotopes, fluorescers, chemiluminescers, chromophores, enzymes, enzyme substrates, enzyme cofactors, enzyme inhibitors, chromophores, dyes, metal ions, metal sols, ligands (e.g., biotin, avidin, strepavidin or haptens) and the like. The term "fluorescer" refers to a substance or a portion thereof which is capable of exhibiting fluorescence in the detectable range. Particular examples of labels which may be used under the invention include, but are not limited to, horse radish peroxidase (HRP), fluorescein, FITC, rhodamine, dansyl, umbelliferone, dimethyl acridinium ester (DMAE), Texas red, luminol, NADPH and α- or β-galactosidase.

In one aspect of the disclosure, a viral particle produced by the methods of the disclosure is bound to a solid support. A sample from a subject is then contacted with the solid support under conditions wherein any antibodies to an epitope of the viral particle can for an immune complex with the viral particle. The solid support may be washed and then contacted with a secondary antibody (e.g., an anti-human antibody) labeled with a detectable label. The solid support can then be developed (as appropriate for the detectable label) and quantitated.

### EXAMPLES

### Reference Example 1. Determination of Growth of XMRV or MLVRV in Different cell lines

Replication of XMRV isolated from prostate cancer cells and CFS PBMCs has been reported and shows that the virus is capable of replicating in human cells. As noted above, XMRV has been suggested to be an artifact caused by contamination of samples by mouse DNA and recombination of laboratory Xenotropic isolates (T.Paprotka, et al., "Recombinant Origin of the Retrovirus XMRV" Science 333:97, 2011); J.Cohen & M.Enserink, "False Postive" Science 333:1694 - 1701, 2011; and G. Simmons et al., "Failure to Confirm XMRV/MLVs in the Blood of Patients with Chronic Fatigue Syndrome: A Multi-Laboratory Study" (www).sciencexpress.org /10.1126/science.1213841). However, as demonstrated here (see, e.g., Figures 9 and 10, Examples 15 and 16, and Table 7), there are individuals with high naturally occurring anti-MLV titers, indicating that MLV infection of humans with MLV related viruses can occur. Therefore in this and following examples, XMRV is used as an example of how such a virus can be detected and followed. XMRV was detected by standard PCR methods in which two primer sets, one in the *gag* region and the other in env region, are used to detect the presence of XMRV proviral DNA and such detection further confirmed by nested PCR (Lombardi et al.). In addition to identification of proviral DNA, the presence of virus has been further demonstrated by detection of viral protein expression in PBMC from CFS patients and by viral transmission in culture cells.

22Rv1 (ATCC CRL-2505) is a human prostate cancer cell line derived from a primary prostatic carcinoma. Multiple integrated copies and high level production of XMRV was described by Knouf et al., J.virol 2009 op.cit). Quantitative PCR as described above revealed approximately 140 copy of XMRV in 22Rv1 genome with a titer of 1.5e7 TU/mL. This virus was used to determine whether a cell line will support XMRV infection. Similar methods can be used for other MLV related viruses.

22Rv1 cell culture supernatant containing XMRV titer of 1.5e7 TU/mL is used to infect a panel of cultured human cell lines: HT1080 (fibrosarcoma), U87-MG (glioma), LNCaP (prostate), and Sup-T1 (T-lymphoblasts). Each cell line is infected with XMRV at a MOI of 0.1. Viral replication kinetic in these cell lines are determined by qPCR using normalized gDNA from each passages and XMRV specific primer set lies in the env region (XMRV 6252F: 5'-TTT GAT TCC TCA GTG GGC TC-3' (SEQ ID NO:1); XMRV6391R: 5'-CGA TAC AGT CTT AGT CCC CAT G-3' (SEQ ID NO:2); XMRV env probe: 5'-HEX-CCC TTT TAC CCG CGT CAG TGA ATT CT-3'-BHQ (SEQ ID NO:3)), (Figures 1 and 2). At maximal infectivity, viral supernatant from each infected cell lines is collected and XMRV titer is determined by qPCR using primer set lies in the pol region (pol-F: 5'-AAC AAG CGG GTG GAA GAC ATC-3' (SEQ ID NO:4); pol-R: 5'-CAA AGG CGA AGA GAG GCT GAC-3' (SEQ ID NO:5); pol probe: 5'-HEX-CCC ACC GTG CCC AAC CCT TAC AAC C-3'-TAMRA (SEQ ID NO:6)). The results reveal that a variety of human cell lines can be infected by XMRV and produce relative high titer (Table 1).

**Table 1**

| Sample | measured titer | average measured titer | standard deviation | CV |
|---|---|---|---|---|
| HT1080 | 0.00E+00 | 0.00E+00 | 0.00E+00 | 0.00E+00 |
| | 0.00E+00 | | | |
| | 0.00E+00 | | | |
| HT1080 lot 15 1:10 | 8.95E+08 | 8.68E+08 | 2.42E+07 | 2.79E-02 |
| | 8.59E+08 | | | |
| | 8.50E+08 | | | |
| HT1080 XMRV | 1.90E+07 | 1.99E+07 | 1.22E+06 | 6.14E-02 |
| | 2.13E+07 | | | |
| | 1.95E+07 | | | |
| LNCaP | 5.88E+03 | 2.02E+03 | 3.34E+03 | 1.65E+00 |
| | 7.70E+01 | | | |
| | 1.19E+02 | | | |
| LNCaP lot 15 1:10 | 2.38E+08 | 2.22E+08 | 1.50E+07 | 6.75E-02 |
| | 2.21E+08 | | | |
| | 2.08E+08 | | | |
| LNCaP XMRV | 3.98E+07 | 3.00E+07 | 1.84E+07 | 6.13E-01 |
| | 4.14E+07 | | | |
| | 8.78E+06 | | | |
| PC3 | 0.00E+00 | 0.00E+00 | 0.00E+00 | 0.00E+00 |
| | 0.00E+00 | | | |
| | 0.00E+00 | | | |
| PC3 lot 15 1:10 | 4.67E+07 | 4.25E+07 | 3.57E+06 | 8.40E-02 |
| | 4.04E+07 | | | |
| | 4.05E+07 | | | |
| PC3 XMRV | 3.93E+06 | 3.44E+06 | 1.18E+06 | 3.43E-01 |
| | 4.30E-06 | | | |
| | 2.10E+06 | | | |
| SUPT1 | 0.00E+00 | 0.00E+00 | 0.00E+00 | 0.00E+00 |
| | 0.00E+00 | | | |
| | 0.00E+00 | | | |
| SUPT1XMRV | 8.31E+06 | 7.90E+06 | 3.57E+05 | 4.52E-02 |
| | 7.71E+06 | | | |
| | 7.67E+06 | | | |
| U87 | 1.92E+03 | 0.00E+00 | 0.00E+00 | 0.00E+00 |
| | 0.00E+60 | | | |
| | 1.77E+03 | | | |
| U87 lot 15 1:10 | 7.16E-08 | 7.89E+08 | 6.38E+07 | 8.08E-02 |
| | 8.28E+08 | | | |
| | 8.24E+08 | | | |
| U87XMRV | 3.00E-t07 | 2.96E+07 | 2.60E+06 | 8.76E-02 |
| | 3.20E+07 | | | |
| | 2.69E+07 | | | |

### Reference Example 2. Stable expression producer cell lines making replication competent virus T5.0002.

The viral vector is encoded by a plasmid (pAC3-yCD2; a.k.a. T5.0002, see, e.g., WO2010045002A2) consisting of 11,893 base pairs of nucleotides. The vector producer cell line "HT1080+T5.0002", was produced by transducing naive HT1080 cells with AC3-yCD2 virus produced transiently in 293T cells by transfection. The transient transfection used to produce AC3-yCD2 (viral particles) was performed using a sequenced "qualified plasmid stock". More specifically, transiently produced AC3-yCD2 vector was harvested after 48 hours post transfection and filtered through a 0.45 µm filter with 0.8 mL of filtered supernatant used to transduce a 75% confluent culture of HT1080 cells containing 15 mL of media. This infection volume converted to an approximate transduction dose of about 0.1 transduction unit (TU) per cell. The transduction was allowed to spread throughout the culture for 9 days with cells refed or passaged every 2-4 days prior to freezing down the initial Pre-Bank stock consisting of 12 vials with each vial containing approximately 5 x10⁶ cells per vial. The freezing media included 10% DMSO, USP (Cryoserv, Bioniche Pharma USA, LLC, Lake Forest, IL) and 90% gamma-irradiated fetal bovine serum (Hyclone Laboratories. Inc, Logan Utah). Cells were frozen in a - 80°C freezer and then transferred to a liquid nitrogen freezer under vapor phase conditions the following day. The media used for growing HT1080+T5.0002 cells to produce the vector comprises a defined DMEM media, GlutaMax (L-glutamine substitute), non-essential amino acids (NEAA), and Defined Fetal Bovine Serum (FBS).

The 293T cell line was developed from HEK (Human Embryonic Kidney) 293 cells and was originally described in 1987 (Dubridge 1987). The cell line was developed by transfecting a temperature sensitive SV40 T-antigen mutant, tsA1609 (Dubridge 1987), into HEK 293 cells (Graham 1977). The 293T cells are more susceptible to transfection than the original HEK 293 cell line. Because of their higher transfectability, 293T cells have commonly been used to produce high titer vectors by transient transfection (Yang et al., Hum. Gene Ther.10:123-132, 1999).

### Reference Example 3. Preparation of virus from HT1080+T5.0002 stable expression producer line as an adherent line with fetal calf serum.

HT1080+T5.0002 cells are grown in disposable multilayered cell culture vessels (Cell Stack, Corning). Production of the crude Toca 511 retroviral vector is performed by harvesting the conditioned media from confluent cultures of HT1080+T5.0002 cells harvested every 10-24 hour period over 2-4 harvest cycles using a manual fed batch process using multiple Cell Stacks containing approximately 1.2 L of conditioned media each. Crude Toca 511 retroviral vector is harvested directly into 10-20L process bags and stored at 2-8°C until approximately 40L of material is collected. Sample of the combined crude pool harvest used for PTC mycloplasma, in vitro viral testing, bioburden, informational PCR titering and retentions.

The crude vector material is clarified by passing through a 0.45 micron filter cartridge and treated with Benzonase to digest host cell genomic DNA. The clarified and DNA digested Toca 511 vector is then captured and concentrated using anion exchange (AEX) chromatography. The eluted concentrated bulk product then undergoes a buffer exchange and purification step using size exclusion (SEC) chromatography. The formulation buffer comprises Tris-based formulation buffer containing sodium chloride, sucrose, mannitol, and human serum albumin. The formulated bulk is 0.2 micron filtered to insure sterility, sampled for testing and then divided in multiple containers as bulk material stored frozen below (<) -65°C.

### Reference Example 4. Quantitative PCR titering assay.

The functional vector concentration, or titer, is determined using a quantitative PCR-based (qPCR) method. In this method, vector is titered by infecting a transducible host cell line (e.g. PC-3 human prostatic carcinoma cells, ATCC Cat# CRL-1435) with a standard volume of vector and measuring the resulting amount of provirus present within the host cells after transduction. The cells and vector are incubated under standard culturing condition (37°C, 5% CO₂) for 24 hr to allow for complete infection prior to the addition of the anti-retroviral AZT to stop vector replication. Next, the cells are harvested from the culture dish and the genomic DNA (gDNA) is purified using an Invitrogen Purelink gDNA purification kit and eluted from the purification column with sterile RNase-/DNase-free water. The A₂₆₀/A₂₈₀ absorbance ratio is measured on a spectrophotometer to determine the concentration and relative purity of the sample. The gDNA concentrations are normalized with additional RNase-/DNase-free water to the lowest concentration of any given set of gDNA preparations such that the input DNA for the qPCR is constant for all samples analyzed. Genomic DNA purity is further assessed by electrophoresis of an aliquot of each sample on an ethidium bromide stained 0.8% agarose gel. If the sample passes an A₂₆₀/A₂₈₀ absorbance range of 1.8-2.0 and shows a single band of gDNA, then the sample is ready for qPCR analysis of provirus copy number of the vector. Using primers that interrogate the LTR region of the provirus (reverse-transcribed vector DNA and vector DNA that is integrated into the host gDNA), qPCR is performed to estimate the total number of transduction events that occurred when the known volume of vector was used to transduce the known number of cells. The number of transduction events per reaction is calculated from a standard curve that utilizes a target-carrying plasmid of known copy-number that is serial diluted from 10⁷ to 10 copies and measured under identical qPCR conditions as the samples. Knowing how many genomic equivalents were used for each qPCR reaction (from the concentration previously determined) and how many transduction events that occurred per reaction, we determine the total number of transduction events that occurred based on the total number of cells that were present at the time of transduction. This value is the titer of the vector after dilution into the medium containing the cells during the initial transduction. To calculate the corrected titer value, the dilution is corrected for by multiplying through by the volume of culture and the volume of titer divided by the volume of titer. These experiments are performed in replicate cultures and analyzed by qPCR using triplicate measurements for each condition to determine an average titer and with its associated standard deviation and coefficient of variance.

### Reference Example 5. Virus Purification and concentration.

Viruses of the disclosure are manufactured by commonly known transient transfection procedures of 293 or HT1080 cells, or from a producer cell non-clonal pool, or from a cloned producer cell line. Depending on cell line adaptation, the medium can be with serum or serum free independent, and the cells can be grown as adherent cells or in suspension, with cell culture supernatant collected under perfusion mode. The culture supernatant is harvested, and stored for up to 2 weeks at 4°C. This bulk harvest is filtered through a 0.45 micron filter cartridge to remove large cellular debris, treated with benzonase to digest cellular DNA (L.Shastry et al. Hum Gene Ther., 15:221, 2004) and subjected further to chromatography purification. (see, e.g., US5792643; T. Rodriguez et al., J Gene Med. 9:233, 2007; P.Sheridan et al., Mol.Ther., 2:262-275, 2000). The benzonase treated viral preparation is loaded on an anion exchange column and the virus is eluted in a stepwise NaCl gradient (see, e.g., Figure 4). The fraction containing the virus can be identified by PCR assay, or by A₂₁₅, A₂₈₀ as well as A₄₀₀. Positive fractions are collected and pooled. The pooled preparation is subsequently loaded on a size exclusion column (SEC) to remove salt as well as other remaining contaminants and to condition the virus into formulation buffer (see, e.g., Figure 5). The SEC is run under isocratic conditions with formulation buffer and the viral fraction from the SEC column is collected from the void volume. Positively identified fractions are pooled, filtered through sterile 0.2 micron filter, aliquoted and frozen at minus 65°C or below. The viral preparation is released based on standard testing such as sterility, mycoplasma and endotoxins, with purity and consistency evaluated by SDS PAGE gel analysis (see, e.g.,Figure 6). Titer is determined as Transducing Units (TU) by PCR quantitation of integrated viral DNA in target cells. The final product is targeted to have a titer of up to 10⁹ TU/ml and is formulated in an isotonic Tris-buffered sucrose solution.

### Reference Example 6. Cloning of a Non-Clonal Pool of Infected HT1080 Cells.

*Dilution Seeding.* Pre-warm media and Multiple 96 well cell culture plates were labelled in order to identify the clone based on the plate and well position and the wells were filled with prewarmed media containing single cell suspension of the HT1080. An early passage of 100% infected HT1080 cells was harvested by trypsinizing and creating a single cell suspension consisting of 1 cell per 600 microL. 200 µL was delivered into each well of a 96 well plate in order to seed approximately 0.3 cells per well. In performing this procedure, a majority of the wells received either 0, 1 or 2 cells per well. Cells were allowed to attach for approximately 4 hours and each well examined to eliminate wells that have received more than 1 cell per well or are empty.

*Clone Propagation.* The wells that initially contained 1 cell per well were cultured by replacing ½ of the media (approximately 100 µL) with fresh 100 µL every 3-4 days for every well. Accidental transfer of cells from one well to another was avoided by replacing the tip used to feed each well during media replacement. Full media replacement was required as cells started to approach confluence in the well. Once the cells reached confluence, each clonal candidate was pasaged to a well of a 48 well plate to continue expansion. Each clone was propagated and passed to a well of a 6 well plate, followed by a T-25 flask, followed by T-75 flask each time the cells reached confluence. Once the clonal cells reached confluence in a T-75 flask, at least 2-3 vials of cryopreserved cells containing 1-2x10⁶ cells per vial were prepared.

*Clone Selection Based on Performance.* Once the clone candidates were frozen down, cell culture experiments were performed to identify the best performing clone and back up clones based on titer production performance and ideal cell culture attributes. The best clone was chosen based on (1) the ability of the clone to provide the highest sustained titers over 2-4 subsequent days with daily media replacement (See Figure 12 and Table 2, below); (2) the ability of the virus produced to transfer expression of the desired gene of interest to a naive cell; (3) the ability of the clone to divide reasonably having a doubling time between 18 - 30 hours and ability to reach 100% confluence as a uniform layer of cells with minimal cell detachment upon reaching confluence.

### Reference Example 7. Infection of D-17 and Cf2-Th Cell Lines to Make a Non-Clonal Pool and Subsequent Clonal Virus Producer Cell Lines.

To produce D-17 (canine osteosarcoma; ATCC # CCL-183) and Cf2-Th (canine thymus; ATCC # CRL-1430) cell line vector producer pools and dilution clones that express MLV replication competent retrovirus, the exact same methods described above for HT-1080 cells were used to create D-17 and Cf2-Th cell lines. Results are shown in Table 2, below.

**Table 2: Data to Support Creation of Producer Pools and Subsequent Dilution Clones of HT-1080, D-17 and Cf2-Th Replication Competent Retroviral Vectors**

| **Cell Line Vector Producing Cell Line** | **MLV Replication Competent Vector Expressed** | **Parental Cell Line** | **Titer Sample** | **Titers Observed (TU/mL)*** |
|---|---|---|---|---|
| HT1080+T5.0002 (Non-Clonal Pool) | AC3-yCD2 | HT-1080 | HT+T5.0002, Day 2 | 1.56E+06 |
| | | | HT+T5.0002, Day 3 | 2.23E+06 |
| | | | HT+T5.0002, Day 4 | 1.90E+07 |
| | | | HT+T5.0002, Day 5.5 | 2.57E+07 |
| HT5.yCD2.128A (Dilution Clone) | AC3-yCD2 | HT-1080 | Clone 12-8, Day 0 | 5.26E+06 |
| | | | Clone 12-8, Day 1 | 7.94E+06 |
| | | | Clone 12-8, Day 2 | 1.00E+07 |
| | | | Clone 12-8, Day 3 | 1.02E+07 |
| D17+T5.0002 (Non-Clonal Pool) | AC3-yCD2 | D-17 | D17+T5.0002, Day 2 | 4.20E+06 |
| | | | D17+T5.0002, Day 3 | 3.83E+06 |
| | | | D17+T5.0002, Day 4 | 4.87E+06 |
| | | | D17+T5.0002, Day 5.5 | 1.39E+06 |
| D5.yCD2.1G7A (Dilution Clone) | AC3-yCD2 | D-17 | D5.yCD2.1G7A, Day 1 | 1.78E+06 |
| | | | D5.yCD2.1G7A, Day 2 | 2.54E+06 |
| | | | D5.yCD2.1G7A, Day 3 | 4.24E+06 |
| CF2+T5.0002 (Non-Clonal Pool) | AC3-yCD2 | Cf2-Th | CF2+T5.0002, Day 2 | 4.17E+04 |
| | | | CF2+T5.0002, Day 3 | 6.97E+03 |
| | | | CF2+T5.0002, Day 4 | 4.97E+06 |
| | | | CF2+T5.0002, Day 5.5 | 3.14E+06 |
| CF5.yCD2.3A12A (Dilution Clone) | AC3-yCD2 | Cf2-Th | CF5.yCD2.3A12A, Day 1 | 1.81E+07 |
| | | | CF5.yCD2.3A12A, Day 2 | 2.68E+07 |
| | | | CF5.yCD2.3A12A, Day 3 | 3.78E+06 |

| | | | | |
|---|---|---|---|---|
| *TU/mL indicates transduction units per mL as determined by quantitative qPCR methods to determine copy number of integrated proviral MLV genomes post transduction into a titering naïve U-87 cell line. | | | | |

### Reference Example 8. Adaptation of HT-1080 Replication Competent MLV Virus Producer Cell Line from Serum and Adherence Dependence to a Serum Free Suspension Culture.

The serum free adaptation process was performed after screening and identification of suitable dilution clone of HT-1080 replication competent virus producing cell line. The serum free adaptation process can also be performed with a non-clonal vector producing HT1080 cell line. The adaptation process was initiated by seeding approximately 2x10⁷ cells into a 125 mL shaker flask containing 10 mL of 5% serum containing conditioned media and 10 mL of a selected serum free media of choice, resulting in a reduced serum concentration of 2.5%. In this case the serum free media was FreeStyle 293 Expression Media distributed through Invitrogen Corp, Carlsbad, CA. The culture was placed on a shaking platform located in a tissue culture incubator with both temperature and CO₂ gas control. The shaking platform was set to about 80 RPM and the incubator is set to about 37°C and 5% CO₂ conditions. Every 3-7 days, the culture was re-fed by collecting cells that are in suspension and reseeded into a new shaker flask containing 10 mL of the same initial conditioned media and 10 mL of fresh serum free media maintaining a level of serum of approximately 2.5%. The culture was examined at each re-feeding event with viable cell counts performed as needed to check for cell propagation. When the cells showed evidence of growth based on cell doubling or glucose consumption, a serum concentration of 1.67% was then targeted by adjusting the volume amount of condition media and fresh serum free media. The culture again was examined and refed every 3-7 days. When the cells show evidence of growth, a serum concentration of 1.25% was targeted by again adjusting the volume of conditioned media and fresh serum free media. This process was continued targeting subsequent serum conditions of 1.0%, 0.9%, 0.83% serum conditions until the cells were in 100% serum free conditions. During this adaptation process the cell culture was expanded to approximately 200 mL volume in a 1,000 mL shaking flask targeting a minimal viable culture of approximately 0.5 to 1.0 x10⁶ cell/mL. Once the cells reached 100% serum free conditions, the cells were continuously passaged under serum free conditions isolating single suspended cells by allowing heavier clumping cells to settle for short periods of time without agitation. Once the culture consists of approximately 95% population of the single cell suspension consistently, the culture could be frozen in cryopreservation media consisting of 10% DMSO and 90% serum free media using standard mammalian cell freezing conditions.

### Reference Example 9. Viral production technique for retroviruses

The example describes a cell culture technique for growing adherent cells producing murine leukemia virus (MLV) related viruses (MLVRV) for viral production and subsequent viral purification. Production of an amphotropic MLV virus (ATCC# VR-1450) was used as an example. However this process can also be used for all viruses described in this disclosure.

Wild type MuLV was obtained from ATCC but can also be generated from a plasmid construct (pAMS, ATCC # 45167) encoding the entire MuLV genomic sequence with a genetically engineered amphotropic envelope gene replacing the original ecotropic envelope gene, and transiently transfected into cells using standard transfection techniques. Preferably, HT1080 cells (ATCC CCL-121) are infected with the virus and propagated under the preferred conditions of 37°C under 5% CO₂ conditions. A newly infected T-75 flask containing MuLV infected HT1080 cells is expanded to two T-175's and then subsequently cultured to ten T-175 flasks with the following growth medium:

| Complete DMEM Medium components: | Ratio |
|---|---|
| 1. DMEM High Glucose, w/o phenol red & w/o glutamine (Biowhittaker) | 500 mL |
| 2. FBS Defined (HyClone) | 25 mL |
| 3. GlutaMax (Gibco) | 5 mL |
| 4. Non essential amino acids (NEAA, Biowhittaker) | 5 mL |

After reaching confluence, cells are harvested with TrpZean (Sigma) and neutralized with the same growth medium using standard cell culture methods. The cells are seeded into three 10-layer Cell Stacks (Corning) at a preferred seeding density of 3.1 x 10^4 viable cells/cm² in the same medium previously described above, to produce the virus. Each Cell Stack contained 1.1L of the growth medium. The Cell Stacks were incubated at 37°C and 5% CO₂.

Two days after seeding, the Cell Stack cultures will reach confluence. The medium in each culture is replaced with fresh medium. Two days later, the medium, containing produced virus, is harvested (Harvest #1), and the cultures re-fed with the same volume (1.1L) of fresh medium. Ten hours later, a second harvest (Harvest #2) is conducted and the cell cultures re-fed with same volume (1.1 L) of fresh growth medium. Sixteen hours following 2^{nd} harvest, a 3^{rd} harvest (Harvest #3) is performed. The 3 harvests are then pooled for purification. Viral titers for the 3 harvests and the pool are listed in the following table.

| **Harvest** | **Viral titer (# TU/mL)** |
|---|---|
| Harvest #1 | 1.8 x 10⁶ |
| Harvest #2 | 2.4 x 10⁶ |
| Harvest #3 | 2.4 x 10⁶ |
| Pool of 3 harvests | 2.1 x 10⁶ |

### Reference Example 10. Viral production by another cell line with the same technique

The same culturing technique described above are used to produce any of the disclosed viruses, in any of HT1080 cells, PC3 cells, LNCaPcells, SupT1 cells, U87 cells, D17 cells, CF2 cells, 293 cells, Hela cells, CV1 cells, CHO cells or cell lines derived from any of these. Three harvests, as described in Example 9, can be collected and pooled. The viral titer in the harvest pool using these cells is 10⁵ TU/mL or greater, usually about 5x10⁶TU /mL.

### Reference Example 11. Adaption of producer cell line to suspension culture and producing virus in a bioreactor for large scale production

A clonal or non clonal HT1080 viral producer cell line producing MLV related virus is adapted to zero-serum and suspension growth using a slow weaning process transitioning from 5% serum conditioned media to a defined serum free media. The adaptation process is initiated by seeding approximately 2x10⁷ cells into a 125 mL shaker flask containing 10 mL of 5% serum containing conditioned media and 10 mL of a selected serum free media of choice, resulting in a reduced serum concentration of 2.5%. In this case the serum free media is FreeStyle 293 Expression Media distributed through Invitrogen Corp, Carlsbad, CA, supplemented with 0.1% human serum albumin (with Baxter 25% human serum solution). The culture is placed on a shaking platform located in a tissue culture incubator with both temperature and CO₂ gas control. The shaking platform is set to a about 80 RPM and the incubator is set to about 37°C and 5% CO₂ conditions. Every 3-7 days, the culture is re-fed by collecting cells that are in suspension and reseeded into a new shaker flask containing 10 mL of the same initial conditioned media and 10 mL of fresh serum free media maintaining a level of serum of approximately 2.5%. The culture is examined at each re-feeding event with cell counts taken as needed to check for cell propagation. When the cells show evidence of growth based on cell doubling or glucose consumption, a serum concentration of 1.67% is then targeted by adjusting the volume amount of condition media and fresh serum free media. The culture again is examined and refed every 3- 7 days as before. When the cells show evidence of growth, a serum concentration of 1.25% is targeted by again adjusting the volume of conditioned media and fresh serum free media. This process is continued targeting subsequent serum conditions of 1.0%, 0.9%, 0.83% serum conditions until the cells are in 100% serum free conditions. During this adaptation process, the cell culture is expanded to approximately 200 mL volume in a 1,000 mL shaking flask targeting a minimal viable cell culture density of approximately 0.5 to 1.0 x10⁶ cell/mL. Once the cells reach 100% serum free conditions, the cells are continuously passaged under serum free conditions by isolating single suspended cells from the top half of the culture by allowing heavier clumping cells to settle for short periods of time without agitation. Once the culture consists of approximately 95% population of the single cell suspension consistently, the culture can be frozen in cryopreservation media consisting of 10% DMSO and 90% serum free media using standard mammalian cell freezing conditions.

The suspension cells are expanded in shake flasks - 125-mL (20 mL culture), 250-mL (40 mL), 500-mL (100 mL), and 1-L (200 mL; all from Corning), in the fully defined serum-free medium (Gibco Cat# 12338), supplemented with 0.1% human serum albumin (HSA, from Baxter). The cultures are incubated at 37°C and 5% CO₂ with shaker speed of 80 rpm (orbit ¾" (1.905 cm)). Five 1-L shake flask cultures are used to inoculate a WAVE bioreactor (WAVE 20/50 EHT, GE Healthcare) containing a 20-L Cellbag with 10L working volume. The initial cell density in the bioreactor is a preferred 4 x 10⁵ viable cells/mL (viability 91%). The temperature is a preferred 37°C. The initial operating conditions are: 5% CO₂, rocker speed 15 rpm, angle 6°, air flow rate 0.2 L/min. The pH control is set at 7.2, and DO control at 40%. Both pH and DO controls are implemented by a WAVE POD console system (GE Healthcare).

After the cell density reaches about∼1 x 10⁶ viable cells/mL, a cell perfusion process is started using a hollow fiber cartridge (GE Healthcare, Part# CFP-6D-6A). The feed (and permeate) rate is initially set at 0.25 volume/day, and progressively increased with cell density for up to 3.8 volumes/day. A total of 180 L of permeate containing the virus is harvested within a 15 day period. The viral titer in the 180L harvests is 1x10⁵ TU/mL to 1x10⁷ TU/mL.

### Example 12. This example illustrates detection of human antibody against MLV in plasma samples from donors.

A human anti-MLV IgG ELISA assay was developed for the detection of anti-MLV specific antibodies. The assay currently discriminates positive and negative samples and provides a titer for the antibody response. A monoclonal antibody, 83A25, specific for the gp70 protein of Moloney MLV was used as an assay positive control. 83A25 recognizes an epitope of gp70 env protein found universally in a variety of murine leukemia viruses including amphotropic leukemia virus (Evans et al., 1977, J. Virol., 24:865-874; Evans et al., 1990, J. Virol., 64(2):6176-6183). 83A25 is a rat IgG2a antibody. 83A25 mAb is derived from hybridoma cells (ATCC cat# HB-10392, Lot# 100005) using standard antibody purification procedures. The assay is designed to detect the presence of anti-MLV antibodies in human samples. The assay positive control (83A25) is a rat antibody that is detected with an anti-rat Ig secondary. An anti-human IgG HRP conjugated antibodies is used for detection of human IgG.

The specific anti-MLV antibody capture ELISA was developed for antibody detection with species specific positive and negative controls. Briefly, 100 µL/well of capture antigen (1.56 µg/mL) was incubated overnight at 2-8°C in ELISA 96-well microtiter plates (Costar). Plates were blocked with blocking buffer (1X PBS, 1% BSA, 0.05% TW20, 0.05% sodium azide). Human or canine sera collected at different time points across the studies was diluted 1:100 and was titred at 1:4 series in PBS added to the capture antigen coated plates and incubated for 60-90 min at room temperature with constant shaking at 150 RPM. Any bound antibody was detected with an HRP-conjugated goat anti-human IgG antibody or anti-canine IgG antibody (Southern Biotech). ELISAs were washed 5X with PBS and developed using a TMB substrate (Southern Biotech) for 10 min incubation and read on a SpectraMax 190 spectraphotometer at 450nm. Positive controls were obtained from immunized animals, negative controls were obtained from healthy animals. Human donor panels were screened to identify suitable human positive and negative serum.

**Table 3: Titer of human serum samples using anti-human IgG-HRP conjugated antibody alone or in combination with anti-rat Ig HRP conjugated antibody. Sera from healthy individuals were assessed for the presence of anti-MLV antibody using human anti-MLV ELISA protocol.**

| **Titer of anti-MLV antibody in human samples** | | |
|---|---|---|
| | **Anti-human IgG only** | **Anti-human IgG + anti-rat Ig** |
| Subject #1 | 1:100 | 1:100 |
| Subject #5 | 1:500 | 1:500 |
| Subject #9 | 1:100 | 1:100 |
| Subject #14 | 1:100 | 1:100 |
| Subject #20 | 1:100 | 1:100 |
| Subject #24 | 1:100 | 1:100 |
| Subject #26 | 1:100 | 1:100 |

Human serum from 10 different healthy donors, tested negative for HBsAg, HIV 1/2 Ab, HIV-1 RNA, HCV Ab, HCV RNA, and STS and verbally screened to not have been in contact with any rodents (mice, rats, guinea pigs) including pets, as well as primates and to never have worked with any viruses, was purchased from BioReclamation Incorporation. All sera were screened using human anti-MLV ELISA protocol and serum tested negative for the presence of anti-amphotropic MLV antibodies were pooled and used as Negative Control Serum. Absorbance values (ODs) at 450 nm were generally recorded at below 0.400.

Reactivity of 83A25 is detected by an anti-rat Ig HRP conjugated antibody. As seen in Figure 7 and Table 3, a conjugate cocktail (anti-rat and anti-human IgG HRP) can be added to the either assay place and not alter the generated titer value. As observed in Figure 2, the reactivity of AC (83A25 monoclonal antibody) when developed with either anti-rat Ig or the conjugate cocktail produced a proper dilution curve (Figure 8A). Although the cocktail generated higher OD values in comparison to an anti-rat antibody alone, the resulting titer from each dose curve was 1:312500.

### Example 13. This example illustrates the detection of anti-MLV specific antibodies in human samples.

To evaluate the detection of a specific anti-MLV antibody within a serum matrix, the 83A25 antibody was spiked into human serum diluted 1:100 in dilution buffer or dilution buffer alone. The spikes were serial diluted and evaluated per the anti-MLV ELISA procedure. Figures 8A and 8B show the generated dose response curves (solid square) for each spike preparation.

### Example 14. This example illustrates the assay specificity and sensitivity.

Using plate bound MLV viral vector, detection of anti-MLV IgG antibodies in human sera or plasma is achived using a direct assay. A competitive assay can be performed using the 83A25 antibody that recognizes gp70-env protein from amphotropic MLV virus. The titer of test samples is established by serially diluting 1:5 from a starting 1:100 dilution and compared to baseline samples; positive signals are defined as those above the cutoff established for that assay (greater than the mean negative control value + 2 SD). Since 83A25 is a rat antibody, an assay threshold values based on the mean of the OD values + 2SD of the dilution buffer is used.
Table 4 and Table 5 present inter-assay performance with replicate analysis of assay positive control and negative serum control.

**Table 4. Inter-Assay reproducibility of Assay Positive Control. Serial dilution curves of assay positive control were analyzed across six experiments. Mean, standard deviation (SD) and percent CV (CV) are displayed.**

| **Mean OD of Assay Positive Control and Inter Assay Analysis** | | | | | | | | | | | | | | | |
|---|---|---|---|---|---|---|---|---|---|---|---|---|---|---|---|
| | **Exp121-Ana** | | **Exp122-Ana** | | **Exp125-Ana** | | **Exp072-DTV** | | **Exp073-DTV** | | **Exp074-DTV** | | **Inter Assay** | | |
| **Dilution Factor** | **Plate** 1 | **Plate 2** | **Plate 1** | **Plate 2** | **Plate 1** | **Plate 2** | **Plate 1** | **Plate 2** | **Plate 1** | **Plate 2** | **Plate 1** | **Plate 2** | **Mean** | **SD** | **%CV** |
| 10 | 2.648 | 2.647 | 2.175 | 2.179 | 2.175 | 2.265 | 1.978 | 2.037 | 2.434 | 2.478 | 2.253 | 2.305 | **2.298** | **0.216** | **9.40** |
| 50 | 2.427 | 2.460 | 2.009 | 1.892 | 1.987 | 1.958 | 1.748 | 1.864 | 2.272 | 2.305 | 2.012 | 2.138 | **2.089** | **0.230** | **10.99** |
| 250 | 2.372 | 2.250 | 1.859 | 1.696 | 1.855 | 1.879 | 1.703 | 1.559 | 2.047 | 2.118 | 1.918 | 1.885 | **1.928** | **0.235** | **12.17** |
| 1250 | 1.928 | 1.844 | 1.481 | 1.400 | 1.440 | 1.438 | 1.298 | 1.307 | 1.782 | 1.606 | 1.450 | 1.390 | **1.530** | **0.211** | **13.82** |
| 6250 | 0.903 | 0.758 | 0.640 | 0.643 | 0.653 | 0.635 | 0.558 | 0.612 | 0.803 | 0.773 | 0.636 | 0.699 | **0.693** | **0.098** | **14.11** |
| 31250 | 0.322 | 0.276 | 0.289 | 0.271 | 0.235 | 0.239 | 0.202 | 0.210 | 0.282 | 0.276 | 0.253 | 0.250 | **0.259** | **0.034** | **13.22** |
| 156250 | 0.163 | 0.159 | 0.180 | 0.174 | 0.136 | 0.124 | 0.116 | 0.119 | 0.140 | 0.139 | 0.145 | 0.131 | **0.144** | **0.021** | **14.62** |
| 781250 | 0.137 | 0.131 | 0.162 | 0.141 | 0.105 | 0.105 | 0.087 | 0.104 | 0.107 | 0.110 | 0.114 | 0.107 | **0.118** | **0.021** | **17.79** |

**Table 5. Inter-Assay reproducibility of Negative Control serum. Serial dilution curves of negative control serum (NC) were analyzed across six experiments (1:100 dilution only). Mean, standard deviation (SD) and %CV (CV) are shown.**

| **Mean OD of Negative Control Serum and Inter Assay Analysis** | | | | | | | | | | | | | | |
|---|---|---|---|---|---|---|---|---|---|---|---|---|---|---|
| **Exp121-Ana** | | **Exp122-Ana** | | **Exp125-Ana** | | **Exp072-DTV** | | **Exp073-DTV** | | **Exp074-DTV** | | **Inter Assay** | | |
| **Plate 1** | **Plate 2** | **Plate 1** | **Plate 2** | **Plate 1** | **Plate 2** | **Plate 1** | **Plate 2** | **Plate 1** | **Plate 2** | **Plate 1** | **Plate 2** | **Mean** | **SD** | **%CV** |
| 0.316 | 0.301 | 0.326 | 0.319 | 0.238 | 0.243 | 0.250 | 0.252 | 0.248 | 0.256 | 0.252 | 0.239 | **0.270** | **0.034** | **12.76** |

### Example 15. This example illustrates the detection of naturally occurring antibodies against MLV in human plasma sample.

Human plasma samples from individual donors were screened using the human anti-MLV ELISA assay. Samples that gave mean OD₄₅₀ nm values below 0.200 were pooled and tested to generate a Negative Control (NC) plasma sample for use in future assays. By contrast, samples that gave high means OD₄₅₀ nm values were selected as potential candidates for Positive Control (PC) plasma. These candidates were tested by Western blotting under non-reducing conditions to confirm the presence of anti-MLV specific antibodies. The sample that gave the best anti-MLV reactivity, and a high OD₄₅₀ nm value as tested in ELISA, was selected as the Positive Control plasma for use in future assays.

Plasmas were obtained from Bioreclamation Inc. (Westbury, NY) and collected in tubes containing K-2 EDTA as an anticoagulant. All samples tested negative for HBsAg, HIV 1 or 2 Ab, HIV-1 RNA, HCV Ab, hCV RNA and STS as reported by the supplier. Donors were classified as Black, Caucasian or Hispanic and varied from 20 to 78 years of age. Upon receipt the plasmas were stored at -80°C until use. Plasmas from donors 866, 867, 871, 872 and 863 with mean OD₄₅₀ nm values <0.200 (Table 6) were pooled to generate a Human Negative Control plasma (Lot# OT02DV-123-19Feb10). In the assays, the NC plasma is diluted 1:100 in Blocking/Dilution buffer with 8 well replicates per plate.

| Donor ID | Sample ID | Sex | Donor Age | Race | Mean OD value in ELISA |
|---|---|---|---|---|---|
| BRH338866 | 866 | F | 50 | B | 0.167 ± 0.010 |
| BRH338867 | 867 | F | 52 | B | 0.127 ± 0.020 |
| BRH338871 | 871 | F | 33 | B | 0.116 ± 0.017 |
| BRH338872 | 872 | F | 55 | C | 0.174 ± 0.015 |
| BRH338863 | 863 | M | 34 | B | 0.138 ± 0.007 |

**Table 6** - **Plasmas selected for the generation of the Human Negative Control plasma (NC).** Human plasmas from 4 female (F) and 1 male (M) donors that gave the lowest mean OD₄₅₀ₙₘ values when screened were chosen for the generation of the Negative Control Plasma. These samples were pooled in equal volumes with aliquots prepared and stored for qualification and use. B - Black; C - Caucasian.

Plasma samples from donors that showed either high or intermediate mean OD₄₅₀ₙₘ values in the anti-MLV ELISA (Table 7) were selected to be analyzed further by Western blotting. This procedure would confirm anti-MLV specificity in the samples. Additionally, plasmas with high mean OD₄₅₀ₙₘ would serve as potential candidates for the Human Positive Control plasma for use in future assays. Further screening shows rare individuals (around 1/200) with ELISA readings in this assay of >3.0 OD units.

**Table 7 - Plasmas selected for Western blot analysis and as potential candidates for the Human Positive Control plasma (PC).**

| Donor ID | Sample ID | Sex | Donor Age | Race | Mean OD value in ELISA | OD value relative to all plasmas |
|---|---|---|---|---|---|---|
| BRH338856 | 856A | M | 58 | C | 0.861 ± 0.077 | Intermediate |
| BRH353131 | 131 | M | 42 | B | 1.766 ± 0.153 | High |
| BRH353183 | 183 | M | 40 | B | 1.059 ± 0.110 | Intermediate |
| BRH353194 | 194 | M | 50 | B | 1.906 ± 0.251 | High |

Details of the human plasmas that showed high or intermediate mean OD₄₅₀ₙₘ values as screened using the anti-MLV ELISA assay and that were to be analyzed for anti-MLV specificity using Western blotting. B - Black; C - Caucasian.

### Example 16

The example illustrates MLV viral antigen specificity of detected positive ELISA human samples. Plasma samples that showed high mean OD₄₅₀ₙₘ values in the anti-MLV ELISA were tested for antigen specificity in Western blotting. Selected plasmas were incubated with a PVDF membrane that contained MLV antigens that had been separated by electrophoresis under denaturing, non-reducing conditions. Lanes with HT1080 cell lysates and bovine serum albumin (BSA) were included as additional antigen samples. Antibodies in the plasma that recognized and bound to the different MLV antigens were then visualized using a horseradish peroxidase (HRP)-conjugated anti-human IgG antibody. Negative Control plasma (Lot# OT02DV-123-19Feb10) was used as a control in the assay.

Figure 9 identifies those plasma samples that were selected for either Positive or Negative Control evaluation as compared to all plasmas screened using the anti-MLV ELISA. Samples 131 and 194 were identified from the human plasma screen that gave the highest mean OD₄₅₀ nm values as tested by ELISA. To confirm the presence of antibodies to MLV in samples 131 and 194, Western blotting was performed using the same MLV antigen lot preparation used in the ELISA assay. Samples 856A and 183 that showed intermediate mean OD₄₅₀ nm values were also included in the assay to compare the level of anti-MLV reactivity. Plasma sample 856A originated from the same donor but was collected on a different day from sample designated 856 (Fig. 10). The Negative Control plasma was used as a control.

Results of the Western blotting analysis are shown in Figure 10. Samples giving both high and intermediate mean OD₄₅₀ nm values in ELISA showed good reactivity to a number of MLV antigens of 68kD, 50kD, 30kD and 17kD in size. The greatest reactivity was observed in samples 131 and 183 against antigens 17kD in size. As noted in similar tests conducted using mouse and dog samples, little correlation between OD₄₅₀ nm values and antigen recognition is observed. No reactivity against MLV antigens or BSA (used to coat the ELISA plates) are seen with the NC plasma, although some reactivity is seen against antigens from HT1080 cell lysates preparations. These lysates were included in the assay since this cell line was originally used to generate the MLV lot. However, no cell proteins are expected to be present in the MLV sample as these are removed during standard viral purification procedures.

Two candidates were identified from the human plasma screen that gave the highest mean OD₄₅₀ nm values as tested by ELISA (samples 131 and 194). To confirm the presence of antibodies to MLV in samples 131 and 194, Western blotting was performed using the same MLV antigen lot preparation used in the ELISA assay. Additional samples 856A and 183 that showed intermediate mean OD₄₅₀ nm values were also included in the assay to compare the level of anti-MLV reactivity (Figure 10). Plasma sample 856A originated from the same donor but was collected on a different day from sample designated 856 (Figure 10). The Negative Control plasma was used as a control. Samples giving both high and intermediate mean OD₄₅₀ nm values in ELISA showed good reactivity to a number of MLV antigens of 68kD, 50kD, 30kD and 17kD in size. The greatest reactivity was observed in samples 131 and 183 against antigens 17kD in size. As noted in similar tests conducted using mouse and dog samples, little correlation between OD₄₅₀ nm values and antigen recognition is observed. No reactivity against MLV antigens or BSA (used to coat the ELISA plates) is seen with the NC plasma, although some reactivity is seen against antigens from HT1080 cell lysates preparations. These lysates were included in the assay since this cell line was originally used to generate the MLV lot. However, no cell proteins are expected to be present in the MLV sample as these are removed during standard viral purification procedures.

A human Positive Control plasma (PC) was generated from donor 131. The newly generated NC and PC plasma samples were with an example of PC titration curved presented in Figure 11. PC titer was calculated at 1:250 for two PC replicates on the first ELISA plate and 1:1250 for the second plate. Mean OD₄₅₀ nm values for the NC were 0.156 ± 0.009 from 8 replicates. Accordingly, the assay acceptance criteria were set at PC ≥ 1:250 titer and NC mean OD₄₅₀ nm values < 0.300. A human matched species control is used as the positive control for the anti-MLV ELISA when testing human samples.

### Example 17

This example illustrates direct detection of antibody against MLV in plasma from canines. Production of positive control serum for the anti-MLV IgG ELISA was achieved by challenging an experimental dog with the virus and collecting sera at specified time intervals. Ampho MLV (T5.0002) was used to immunize a beagle with adjuvant (1:1 dilution in complete freund's adjuvant), primed subcutaneously and at day 30, boosted w/vector (40 µg/dog) diluted 1:1 w/ Incomplete Freund's Adjuvant. Five subcutaneous injections (8 µg each) were given at day 15, 22 and 28 after priming; days 6 and 14 after boost (days 36 and 44 after priming).

A series of experiments were conducted to evaluate the anti-MLV response curves of sera from the immunized dog that had been collected at different timepoints following innoculation. The serum had been separated and stored at -80°C until use. Sera collected on days 15, 22 and 28 gave weak, but progressively higher antibody response curves, culminating with a strong dose response on days 36 and 44. Similarity in the dose response curves of sera collected at these last two timepoints, and a comparison with d36+d44 pooled sera at a 1:5 dilution factor is shown in Figure 12. To improve the linear portion of the dose response curve, different dilution factors were tested (1:3, 1:4 and 1:5) and compared (Figure 13). Based on this information, a 1:4 dilution factor starting at a 1:100 dilution was selected as optimum.

### Example 18

The example illustrates the selectivity, sensitivity and reproducibility determined for the detection of MLV antibodies in canine samples.

Results generated with positive control serum (from dog immunized with viral vector) and negative control serum (from naive dogs) suggests that signal in this assay is indeed a reflection of IgG binding to plate-bound viral vector. The titer of test samples will be established by serially diluting 1:4 from a starting 1:100 dilution and compared to pre-bleed samples; positive signals are defined as those above the cutoff established for that assay (greater than the mean negative control value + 2 SD).

To assess inter-assay performance, data from several studies were analyzed, with replicate analysis of positive control and negative controls (Tables 8 and 9).

**Table 8. Inter-Assay reproducibility of Positive Control serum.**

| | **Exp067** | | **Exp071** | | **Exp119** | | **Exp124** | |
|---|---|---|---|---|---|---|---|---|
| Dilution | PC1 | PC2 | PC1 | PC2 | PC1 | PC2 | PC1 | PC2 |
| 100 | 3.325 | 3.319 | 3.464 | 3.406 | 2.989 | 3.062 | 3.578 | 3.584 |
| 400 | 3.049 | 3.287 | 3.415 | 3.352 | 2.619 | 2.618 | 3.572 | 3.553 |
| 1600 | 2.889 | 2.967 | 3.274 | 3.145 | 2.234 | 2.113 | 3.441 | 3.374 |
| 6400 | 2.384 | 2.451 | 2.687 | 2.739 | 1.781 | 1.753 | 2.925 | 2.893 |
| 25600 | 1.184 | 1.142 | 1.495 | 1.433 | 0.639 | 0.788 | 1.318 | 1.358 |
| 102400 | 0.321 | 0.275 | 0.398 | 0.382 | 0.172 | 0.153 | 0.268 | 0.312 |
| 409600 | 0.061 | 0.057 | 0.077 | 0.078 | 0.062 | 0.037 | 0.053 | 0.049 |
| 1639400 | 0.005 | 0.007 | 0.010 | 0.011 | 0.044 | 0.000 | 0.010 | 0.007 |
| | | | | | | | | |

| | | | **Inter Assay** | | | | | |
|---|---|---|---|---|---|---|---|---|
| | | | Mean | StdDev | CV% | | | |
| | | | 3.341 | 0.220 | 6.6 | | | |
| | | | 3.183 | 0.385 | 12.1 | | | |
| | | | 2.930 | 0.504 | 17.2 | | | |
| | | | 2.452 | 0.463 | 18.9 | | | |
| | | | 1.170 | 0.307 | 26.3 | | | |
| | | | 0.285 | 0.088 | 31.0 | | | |
| | | | 0.059 | 0.014 | 22.9 | | | |
| | | | 0.012 | 0.014 | 116.0 | | | |

Serial dilution curves of positive control serum (PC) were analyzed across four experiments. Mean, standard deviation (SD) and %CV (CV) are shown.

**Table 9. Inter-Assay reproducibility of Negative Control serum.**

| | | **MeanValue** | **Std.Dev.** | **CV%** |
|---|---|---|---|---|
| Exp067 | NC1 | 0.272 | 0.014 | 5.3 |
| | NC2 | 0.288 | 0.016 | 5.5 |
| Exp071 | NC1 | 0.407 | 0.029 | 7.1 |
| | NC2 | 0.412 | 0.025 | 6.0 |
| Exp119 | NC1 | 0.195 | 0.026 | 13.1 |
| | NC2 | 0.202 | 0.026 | 13.1 |
| Exp124 | NC1 | 0.447 | 0.025 | 5.6 |
| | NC2 | 0.432 | 0.019 | 4.3 |
| Inter Assay | Mean | 0.332 | | |
| | Std. Dev. | 0.104 | | |
| | CV% | 31.4 | | |

Negative control serum (NC) were analyzed across four experiments (1:100 dilution only). Mean, standard deviation (SD) and %CV (CV) are shown.

A four-parameter curve analysis for Positive Control sera was performed using data from several studies. The average of duplicates was used to generate the data in figure 14.

### Example 19

The example illustrates direct detection of antibodies against MLV in sera of patients using direct assays.
Two canine studies were conducted for evaluating immune responses to amphotropic MLV by intracranial and intravenous.

An immune response to MLV was observed in all the high dose groups E7 dogs (N=4) and E9 (N=2) (Figure 15-16). Compared with pre-administration sera of the E7 dose, all four beagles' MLV titers increased by 4-fold (a single animals increased by 16-fold). This response was seen at 30 days post administration for all animals and levels maintained for 3 months (sera drawn every other week) for two of the four animals (Fig. 15). The other two animals' signal has returned to pre-MLV exposure titers by 75 days. Beagles that did not receive Toca 511 (N=2) did not show the increase in titers at all time-points tested (control animals). At E9 dose, one animal was observed to have a 16 fold change from baseline as early as 7 days. A 64-fold anti-MLV IgG response over baseline testing was observed in both animals at day 28 (Fig. 16). By day 60 and continuing to day 90 both animals' anti-MLV IgG response had decreased to a 16-fold increase over baseline (Fig. 16).

### Example 20

The example illustrates the direct detection of amphotropic ENV viral protein in human samples by direct immunoblot approach.

An amphotropic MLV viral particle was serially diluted to give corresponding viral loads from 3.2e5 down to le4 and blotted onto a nitrocellulose membrane. Human plasma samples were diluted to 1:10, 1:20, 1:40 and 1:60 (final) and spiked with virus at 3.2e5, 1.6e5 and 8e4 (final). All samples were denatured/reduced in LDS buffer containing 2-ME and heated to 100°C. After blocking, the membrane was incubated with anti-gp70 antibody (clone: 83A25) at 1ug/mL for 1h at RT. The membranes were developed with a streptavidin:HRP system and visualized with a BioRad Imager (Fig. 17) .

### Example 21

The example illustrates the direct detection of XMRV proteins by immunoblotting using antibody clone 514.

Detection of viral gp70 expression in MLV and XMRV samples using the mouse monoclonal anti-gp70 antibody (clone 514) was tested by Western blotting. Purified virus TGFP6002-SEC and cell lysates from T5.0002-infected U87 cells were compared to cell lysates obtained from the 22Rv1 cell line. Samples were loaded on a 4-12% Bis-Tris gel (BioRad) at 1 pg/well total protein (TGFP6002-SEC) and 40 pg/well total proteins for the cell lysates. The gel was run at 200V for 35min. The separated proteins were transferred to a PVDF membrane which was blocked overnight in 5% non-fat milk at 4°C. The membrane was then incubated with clone 514 (1µg/mL) for 2h at RT on a shaking plate. After washing with TBS-Tween, bound anti-gp70 was detected using a secondary HRP-labeled anti-mouse IgM antibody (1:2000, 1h 45min, RT) and a chemiluminescent substrate. A second anti-gp70 antibody (clone 83A25) was run as a control (Figure 18).

### Example 22

The example shows the detection of antibodies with the ELISA in high grade glioma (HGG) patients treated with Toca 511 by intracranial injection into the tumor followed by treatment with 5-fluorocytosine (5-FC). The trial is a multicenter, open-label, ascending-dose trial of the safety and tolerability of increasing doses of Toca 511 administered to subjects with recurrent HGG who have undergone surgery followed by adjuvant radiation and chemotherapy. The primary goal of the trial is to identify the highest, safe and well-tolerated dose of Toca 511 administered intratumorally via stereotactic, transcranial injection. Secondary goals are: to evaluate the safety and tolerability of treatment with 5-fluorocytosine (5-FC) at approximately 130 mg/kg/day for 6 days beginning approximately 3 weeks after administration of Toca 511 and repeated approximately every 4 weeks; to evaluate the objective response rate of the Toca 511/5-FC combination as assessed using the Macdonald criteria;to assess the percentage of subjects who have not progressed or died at 6 months (PFS-6); to assess the safety and tolerability of Toca 511 administered into residual tumor at the time of craniotomy and partial resection for recurrent high grade glioma (HGG)and followed by cyclic treatment with 5-FC.

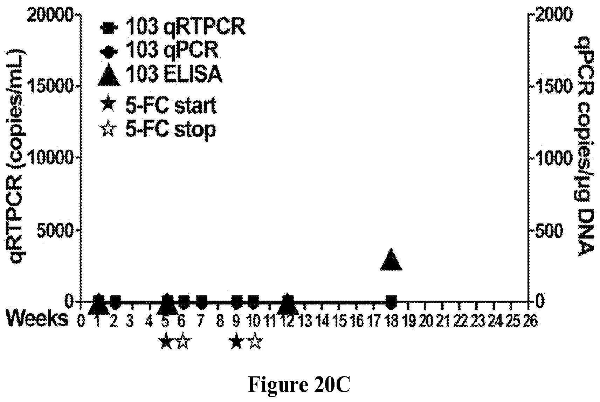

Trial subjects are adults with recurrent HGG that has recurred following surgery, radiation and chemotherapy. Approximately three weeks after vector administration subjects underwent a baseline gadolinium-enhanced MRI (Gd-MRI) scan and then began treatment with oral 5-FC at approximately 130 mg/kg/day for 6 days. 6-day courses of 5-FC are repeated approximately every 4 weeks (± 1 week) until institution of new antineoplastic treatment for tumor progression. Pts 101 and 102 had their tumors resected at the time frames shown in Figure 20. Blood samples were taken at the time points shown in Figure 20 and DNA from whole blood, RNA from plasma and antibodies from plasma were measured by quantitative PCR (DNA), by quantitative RT-PCR (RNA) and by ELISA (anti-virus antibodies).

## Claims

1. A method for detecting anti-viral antibodies for amphotropic murine leukemia virus (MLV) or a murine leukemia virus-related virus (MLVRV) in a biological sample comprising:
a) incubating a biological fluid sample with a capture reagent immobilized on a solid support to bind multiple anti-viral antibodies to amphotropic MLV or MLVRV to the capture reagent, wherein the capture reagent comprises a preparation of serum-free whole viral particles of MLV or MLVRV;
b) detecting bound anti-viral antibodies bound to immobilized capture reagent by contacting the bound anti-viral antibodies with a labeled detecting secondary agent.

2. The method of claim 1, wherein the biological fluid sample is isolated from a mammal and is selected from plasma, serum, urine, saliva, fine needle aspirate, semen, vaginal secretion and milk.

3. The method of claim 2, wherein the mammal is a human.

4. The method of claim 2 or claim 3, wherein the mammal has an autoimmune disorder, a viral infection, a cancer, or is immunosuppressed.

## Patentansprüche

1. Verfahren zum Nachweisen von antiviralen Antikörpern für amphotropes murines Leukämie-Virus (MLV) oder ein mit murinem Leukämie-Virus verwandtes Virus (MLVRV) in einer biologischen Probe, wobei das Verfahren Folgendes umfasst:
a) Inkubieren einer biologischen Flüssigkeitsprobe mit einem Capture-Reagenz, das auf einem festen Träger immobilisiert ist, um mehrere antivirale Antikörper zu amphotropem MLV oder MLVRV an dem Capture-Reagenz zu binden, wobei das Capture-Reagenz ein Präparat von serumfreien Vollviruspartikeln von MLV oder MLVRV umfasst;
b) Nachweisen von gebundenen antiviralen Antikörpern, die an immobilisiertes Capture-Reagenz gebunden sind, durch Inkontaktbringen der gebundenen antiviralen Antikörper mit einem markierten sekundären Nachweisagens.

2. Verfahren nach Anspruch 1, wobei die biologische Flüssigkeitsprobe von einem Säugetier isoliert ist und aus Plasma, Serum, Urin, Speichel, Feinnadelaspirat, Sperma, Vaginalsekret und Milch ausgewählt ist.

3. Verfahren nach Anspruch 2, wobei das Säugetier ein Mensch ist.

4. Verfahren nach Anspruch 2 oder 3, wobei das Säugetier eine Autoimmunerkrankung, eine Virusinfektion oder eine Krebserkrankung aufweist oder immunsupprimiert ist.

## Revendications

1. Méthode de détection d'anticorps antiviraux dirigés contre le virus amphotropique de la leucémie murine (MLV) ou un virus apparenté au virus de la leucémie murine (MLVRV) dans un échantillon biologique qui comprend :
a) l'incubation d'un échantillon biologique liquide avec un réactif de capture immobilisé sur un support solide pour lier de multiples anticorps antiviraux dirigés contre le MLV amphotropique ou le MLVRV au réactif de capture, où le réactif de capture comprend une préparation de particules virales entières du MLV ou du MLVRV exemptes de sérum ;
b) la détection des anticorps antiviraux liés au réactif de capture immobilisé par la mise en contact des anticorps antiviraux liés avec un agent de détection secondaire marqué.

2. Méthode de la revendication 1, où l'échantillon biologique liquide est isolé à partir d'un mammifère et sélectionné parmi le plasma, le sérum, l'urine, la salive, un échantillon obtenu par aspiration à l'aiguille fine, le sperme, les sécrétions vaginales et le lait.

3. Méthode de la revendication 2, où le mammifère est un être humain.

4. Méthode de la revendication 2 ou de la revendication 3, où le mammifère souffre d'une maladie auto-immune, d'une infection virale, d'un cancer ou d'immunodépression.
